(19) Europäisches Patentamt
European Patent Office
Office européen des brevets

(11) EP 4 330 262 B1

(12) # EUROPEAN PATENT SPECIFICATION

(45) Date of publication and mention of the grant of the patent:
**29.01.2025 Bulletin 2025/05**

(21) Application number: **22724780.6**

(22) Date of filing: **25.04.2022**

(51) International Patent Classification (IPC):
***C07D 513/20*** (2006.01) ***C07D 513/22*** (2006.01)
***C07D 519/00*** (2006.01) ***A61P 35/00*** (2006.01)
***A61K 31/553*** (2006.01)

(52) Cooperative Patent Classification (CPC):
**C07D 513/20; A61P 35/00; C07D 513/22; C07D 519/00**

(86) International application number:
**PCT/EP2022/060898**

(87) International publication number:
**WO 2022/229102 (03.11.2022 Gazette 2022/44)**

(54) **MACROCYCLIC 2-ALLYLTETRAHYDROFURANS AS INHIBITORS OF MCL-1**

MAKROCYCLISCHE 2-ALLYLTETRAHYDROFURANE ALS INHIBITOREN VON MCL-1

2-ALLYLTETRAHYDROFURANS MACROCYCLIQUES EN TANT QU'INHIBITEURS DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2021 EP 21170425**

(43) Date of publication of application:
**06.03.2024 Bulletin 2024/10**

(73) Proprietor: **JANSSEN Pharmaceutica NV**
**2340 Beerse (BE)**

(72) Inventors:
- **ROMBOUTS, Frederik, Jan, Rita**
  **2340 Beerse (BE)**
- **HSIAO, Meng-Yang**
  **2340 Beerse (BE)**
- **JERHAOUI, Soufyan**
  **2340 Beerse (BE)**
- **ROMANOV MICHAILIDIS, Fedor**
  **2340 Beerse (BE)**
- **SURKYN, Michel**
  **2340 Beerse (BE)**
- **URNER, Lorenz, Michael**
  **2340 Beerse (BE)**
- **DIELS, Gaston, Stanislas, M**
  **2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2017/147410** **WO-A1-2019/036575**
**WO-A1-2019/222112** **WO-A1-2020/097577**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 330 262 B1

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal antiapoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2019046150 discloses macrocyclic compounds that inhibit mcl-1 protein.

WO2019173181 discloses alpha-hydroxy phenylacetic acid pharmacophore or bioisostere mcl-1 protein antagonists.WO2016033486 discloses tetrahydronaphthalene derivatives that inhibit mcl-1 protein.

WO2019036575, WO2017147410, and WO2018183418 disclose compounds that inhibit mcl-1 protein.

WO2019222112 discloses MCL-1 inhibitors for treating cancer.

WO2020097577 discloses spiro-sulfonamide derivatives as inhibitors of myeloid cell leukemia-1 (MCL-1) protein.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia (AML), and acute lymphoblastic leukemia (ALL).

SUMMARY OF THE INVENTION

The present invention concerns compounds of Formula (I):

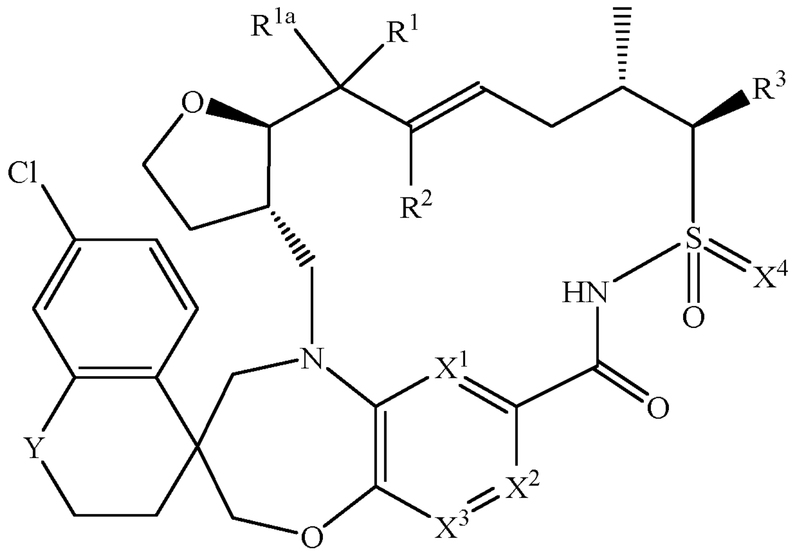

(I)

wherein

$R^1$ represents hydrogen, $-OR^a$, $-CH_2OR^b$, $-CH_2NR^cR^d$, or $-CH_2-C(=O)-NR^cR^d$;
$R^{1a}$ represents hydrogen, $-OR^a$, $-CH_2OR^b$, $-CH_2NR^cR^d$, or $-CH_2-C(=O)-NR^cR^d$;
provided that $R^1$ and $R^{1a}$ cannot both be $-OR^a$; wherein at least one of R1 or R1a is other than hydrogen;
$R^a$ is selected from the group consisting of hydrogen, $C_{1-4}$alkyl, and $C_{1-4}$alkyl substituted with one substituent selected from the group consisting of $Het^1$, $-OR^e$, and $-NR^fR^g$;
$R^b$ is selected from the group consisting of hydrogen, $C_{1-4}$alkyl, and $C_{1-4}$alkyl substituted with one substituent selected from the group consisting of $Het^1$, $-OR^e$, and $-NR^fR^g$;
$R^c$ and $R^d$ are each independently selected from the group consisting of hydrogen; $C_{1-4}$alkyl; $C_{1-4}$alkyl substituted with $-O-C_{1-4}$alkyl; and $C_{2-4}$alkyl substituted with one $Het^1$;
or $R^c$ and $R^d$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$;
wherein said monocyclic or fused bicyclic heterocyclyl is optionally substituted on one nitrogen with one $C_{1-4}$alkyl;
$R^f$ and $R^g$ are each independently selected from the group consisting of hydrogen; $C_{1-4}$alkyl; $C_{1-4}$alkyl substituted with $-O-C_{1-4}$alkyl; and $C_{2-4}$alkyl substituted with one $Het^1$;
or $R^f$ and $R^g$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$;
wherein said monocyclic or fused bicyclic heterocyclyl is optionally substituted on one nitrogen with one $C_{1-4}$alkyl;
$R^e$ represents hydrogen or $C_{1-4}$alkyl; $-C_{2-4}$alkyl-O-$C_{1-4}$alkyl, or $-C_{2-4}$alkyl-O-$C_{2-4}$alkyl-O-$C_{1-4}$alkyl;
$R^2$ represents hydrogen or fluoro;
$R^3$ represents hydrogen or methyl;
$R^4$ represents -(C=O)-phenyl, -(C=O)-$Het^2$ or -(C=O)-$Het^3$; wherein said phenyl, $Het^2$ or $Het^3$ are optionally substituted with one or two substituents selected from methyl or methoxy;
$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;
$Het^2$ represents a C-linked 4- to 7-membered monocyclic fully saturated heterocyclyl containing one or two

heteroatoms each independently selected from O, S, and N;
wherein said S-atom might be substituted to form S(=O) or $S(=O)_2$;
$Het^3$ represents a C-linked 5-or 6-membered monocyclic aromatic ring containing one, two or three heteroatoms each independently selected from O, S, and N;
Y represents $CH_2$;
$X^1$ represents CH or N;
$X^2$ represents CH ;
$X^3$ represents CH or N;
$X^4$ represents O or $NR^4$;
and the pharmaceutically acceptable salts and the solvates thereof.

The present invention also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

The invention also relates to the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

The invention also relates to a product comprising a compound of Formula (I), a pharmaceutically acceptable salt, or a solvate thereof, and an additional pharmaceutical agent, as a combined preparation for simultaneous, separate or sequential use in the treatment or prevention of cancer.

DETAILED DESCRIPTION OF THE INVENTION

The term ‘halo’ or ‘halogen’ as used herein represents fluoro, chloro, bromo and iodo.

The prefix '$C_{x-y}$' (where x and y are integers) as used herein refers to the number of carbon atoms in a given group. Thus, a $C_{1-4}$alkyl group contains from 1 to 4 carbon atoms, and so on.

The term '$C_{1-4}$alkyl' as used herein as a group or part of a group represents a straight or branched chain fully saturated hydrocarbon radical having from 1 to 4 carbon atoms, such as methyl, ethyl, *n*-propyl, isopropyl, *n*-butyl, *s*-butyl, *t*-butyl and the like.

The term '$C_{2-4}$alkyl' as used herein as a group or part of a group represents a straight or branched chain fully saturated hydrocarbon radical having from 2 to 4 carbon atoms, such as ethyl, *n*-propyl, isopropyl, *n*-butyl, *s*-butyl, *t*-butyl and the like.

It will be clear for the skilled person that $S(=O)_2$ or $SO_2$ represents a sulfonyl moiety.

It will be clear for the skilled person that CO or C(=O) represents a carbonyl moiety.

Non-limiting examples of two R groups taken together to form together with the N-atom to which they are attached a 4- to 7-membered monocyclic fully saturated heterocyclyl containing one N-atom and optionally one additional heteroatom selected from O, S, and N, include, but are not limited to N-linked azetidinyl, N-linked pyrrolidinyl, N-linked morpholinyl and N-linked piperidinyl.

Non-limiting examples of 4- to 7-membered monocyclic fully saturated heterocyclyl containing one or two heteroatoms each independently selected from O, S, and N, include, but are not limited to tetrahydropyranyl, morpholinyl and azetidinyl.

Non-limiting examples of C-linked 5- or 6-membered monocyclic aromatic ring containing one, two or three heteroatoms each independently selected from O, S, and N, include, but are not limited to C-linked pyrrolyl, C-linked pyridinyl, C-linked pyrimidinyl, C-linked thiazolyl, and C-linked oxazolyl.

Fused bicyclic groups are two cycles that share two atoms and the bond between these atoms.

Examples of fused bicyclic 6- to 11-membered fully saturated heterocyclyl containing one N-atom and optionally one or two additional heteroatoms each independently selected from O, S, and N, include, but are not limited to

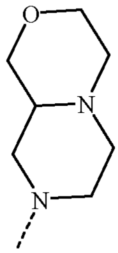
,
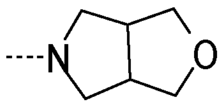
,
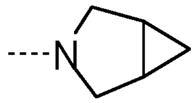
,

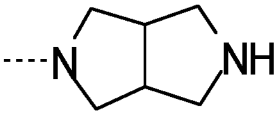
,
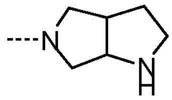
,
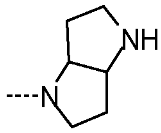
,

and the like.

Unless otherwise specified or clear from the context, heterocyclyl goups (e.g. $Het^1$) can be attached to the remainder of the molecule of Formula (I) through any available ring carbon atom (C-linked) or nitrogen atom (N-linked) if available.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Unless otherwise specified or clear from the context, aromatic rings and heterocyclyl goups, may optionally be substituted, where possible, on carbon and/or nitrogen atoms according to the embodiments.

Combinations of substituents and/or variables are permissible only if such combinations result in chemically stable compounds. 'Stable compound' is meant to indicate a compound that is sufficiently robust to survive isolation to a useful degree of purity from a reaction mixture.

When two or more substituents are present on a moiety they may, where possible and unless otherwise indicated or clear from the context, replace hydrogens on the same atom or they may replace hydrogen atoms on different atoms in the moiety.

When any variable occurs more than one time in any constituent, each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R, S*) around one or more atoms, contemplates each possible stereoisomer, or mixture of two or more stereoisomers. Where the stereochemistry of any particular chiral atom is not specified in the structures shown herein, then all stereoisomers are contemplated and included as the compounds of the invention, either as a pure stereoisomer or as a mixture of two or more stereoisomers.

Hereinbefore and hereinafter, the term "compound of Formula (I)" is meant to include the stereoisomers thereof and the tautomeric forms thereof. However where stereochemistry, as mentioned in the previous paragraph, is specified

by bonds which are shown as solid wedged or hashed wedged bonds, or are otherwise indicated as having a particular configuration (e.g. *R, S*), or when the stereochemistry around a double bond is shown (e.g. in Formula (I)), then that stereoisomer is so specified and defined. It will be clear this also applies to subgroups of Formula (I).

Some of the compounds according to Formula (I) may also exist in their tautomeric form. Such forms in so far as they may exist, although not explicitly indicated in the above Formula (I) are intended to be included within the scope of the present invention. It follows that a single compound may exist in both stereoisomeric and tautomeric form.

For example

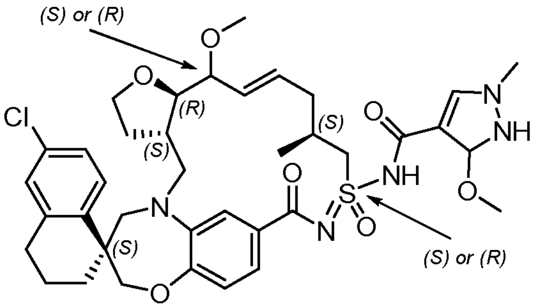

8

also covers the other tautomeric form

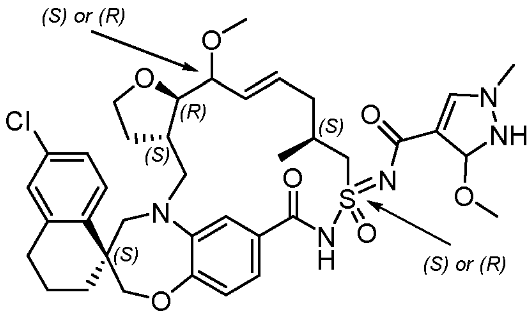

8

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Diastereomers (or diastereoisomers) are stereoisomers that are not enantiomers, i.e. they are not related as mirror images. If a compound contains a double bond, the substituents may be in the E or the Z configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, diastereomers, racemates, *E* isomers, *Z* isomers, cis isomers, trans isomers and mixtures thereof, unless the context indicates otherwise and whenever chemically possible.

The meaning of all those terms, i.e. enantiomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of

Formula (I) is for instance specified as *E*, this means that the compound is substantially free of the *Z* isomer; when a compound of Formula (I) is for instance specified as cis, this means that the compound is substantially free of the trans isomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, N-oxides and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine $_>$ chlorine and iodine, such as $^{2}H$, $^{3}H$, $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the isotope is selected from the group of $^{2}H$, $^{3}H$, $^{11}C$ and $^{18}F$. More preferably, the isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and

$^{18}F$ are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

In an embodiment, the present invention concerns novel compounds of Formula (I), wherein

$R^1$ represents hydrogen, $-OR^a$, $-CH_2OR^b$, $-CH_2NR^cR^d$, or $-CH_2-C(=O)-NR^cR^d$;

$R^{1a}$ represents hydrogen, $-OR^a$, $-CH_2OR^b$, $-CH_2NR^cR^d$, or $-CH_2-C(=O)-NR^cR^d$;

provided that $R^1$ and $R^{1a}$ cannot both be $-OR^a$; wherein at least one of R1 or R1a is other than hydrogen;

$R^a$ is selected from the group consisting of hydrogen, $C_{1-4}$alkyl, and $C_{1-4}$alkyl substituted with one substituent selected from the group consisting of $Het^1$, $-OR^e$, and $-NR^fR^g$;

$R^b$ is selected from the group consisting of hydrogen, $C_{1-4}$alkyl, and $C_{1-4}$alkyl substituted with one substituent selected from the group consisting of $Het^1$, $-OR^e$, and $-NR^fR^g$;

$R^c$ and $R^d$ are each independently selected from the group consisting of hydrogen; $C_{1-4}$alkyl; $C_{1-4}$alkyl substituted with $-O-C_{1-4}$alkyl; and $C_{2-4}$alkyl substituted with one $Het^1$; or $R^c$ and $R^d$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl;

$R^e$ represents hydrogen or $C_{1-4}$alkyl; $-C_{2-4}$alkyl-O-$C_{1-4}$alkyl, or $-C_{2-4}$alkyl-O-$C_{2-4}$alkyl-O-$C_{1-4}$alkyl;

$R^2$ represents hydrogen or fluoro;

$R^3$ represents hydrogen or methyl;

$R^4$ represents -(C=O)-phenyl, -(C=O)-$Het^2$ or -(C=O)-$Het^3$; wherein said phenyl, $Het^2$ or $Het^3$ are optionally substituted with one or two substituents selected from methyl or methoxy;

$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;

$Het^2$ represents a C-linked 4- to 7-membered monocyclic fully saturated heterocyclyl containing one or two heteroatoms each independently selected from O, S, and N;

wherein said S-atom might be substituted to form S(=O) or $S(=O)_2$;

$Het^3$ represents a C-linked 5-or 6-membered monocyclic aromatic ring containing one, two or three heteroatoms each independently selected from O, S, and N;

Y represents $CH_2$;

$X^1$ represents CH or N;

$X^2$ represents CH ;

$X^3$ represents CH or N;

$X^4$ represents O or $NR^4$;

and the pharmaceutically acceptable salts and the solvates thereof.

In an embodiment, the present invention concerns novel compounds of Formula (I), wherein

$R^1$ represents hydrogen, $-OR^a$, $-CH_2OR^b$, $-CH_2NR^cR^d$, or $-CH_2-C(=O)-NR^cR^d$;
$R^{1a}$ represents hydrogen, $-OR^a$, $-CH_2OR^b$, $-CH_2NR^cR^d$, or $-CH_2-C(=O)-NR^cR^d$;
provided that $R^1$ and $R^{1a}$ cannot both be $-OR^a$; wherein at least one of R1 or R1a is other than hydrogen;
$R^a$ is selected from the group consisting of hydrogen, $C_{1-4}$alkyl, and $C_{1-4}$alkyl substituted with one substituent selected from the group consisting of $Het^1$, and $-OR^e$;
$R^b$ is selected from the group consisting of hydrogen, $C_{1-4}$alkyl, and $C_{1-4}$alkyl substituted with one substituent selected from the group consisting of $Het^1$, and $-OR^e$;
$R^c$ and $R^d$ are each independently selected from the group consisting of hydrogen; $C_{1-4}$alkyl; $C_{1-4}$alkyl substituted with $-O-C_{1-4}$alkyl; and $C_{2-4}$alkyl substituted with one $Het^1$; or $R^c$ and $R^d$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl;
$R^e$ represents hydrogen or $C_{1-4}$alkyl; $-C_{2-4}$alkyl-O-$C_{1-4}$alkyl, or $-C_{2-4}$alkyl-O-$C_{2-4}$alkyl-O-$C_{1-4}$alkyl;
$R^2$ represents hydrogen or fluoro;
$R^3$ represents hydrogen or methyl;
$R^4$ represents -(C=O)-phenyl, -(C=O)-$Het^2$ or -(C=O)-$Het^3$; wherein said phenyl, $Het^2$ or $Het^3$ are optionally substituted with one or two substituents selected from methyl or methoxy;
$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;
$Het^2$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;
$Het^3$ represents a C-linked 5-or 6-membered monocyclic aromatic ring containing one, two or three heteroatoms each independently selected from O, S, and N;
Y represents $CH_2$;
$X^1$ represents CH or N;
$X^2$ represents CH ;
$X^3$ represents CH or N;
$X^4$ represents O or $NR^4$;
and the pharmaceutically acceptable salts and the solvates thereof.

In an embodiment, the present invention concerns novel compounds of Formula (I), wherein

$R^1$ represents hydrogen, $-CH_2NR^cR^d$, or $-CH_2-C(=O)-NR^cR^d$;
$R^{1a}$ represents $-OR^a$ or $-CH_2OR^b$;
$R^a$ is selected from the group consisting of hydrogen, $C_{1-4}$alkyl, and $C_{1-4}$alkyl substituted with one $Het^1$ substituent;
$R^b$ is selected from the group consisting of hydrogen and $C_{1-4}$alkyl;
$R^c$ and $R^d$ are each independently selected from $C_{1-4}$alkyl substituted with $-O-C_{1-4}$alkyl; or $R^c$ and $R^d$ are taken together to form together with the N-atom to which they are attached a fused bicyclic 6- to 11-membered fully saturated heterocyclyl containing one N-atom and optionally one or two additional heteroatoms each independently selected from O and N;
$R^2$ represents hydrogen or fluoro;
$R^3$ represents hydrogen or methyl;
$R^4$ represents -(C=O)-$Het^3$; wherein said $Het^3$ is optionally substituted with one or two substituents selected from methyl or methoxy;
$Het^1$ represents a 4- to 7-membered monocyclic fully saturated heterocyclyl containing one or two heteroatoms each independently selected from O and N;
$Het^3$ represents a C-linked 5-or 6-membered monocyclic aromatic ring containing one, two or three heteroatoms each independently selected from O and N;

Y represents $CH_2$;
$X^1$ represents CH or N;
$X^2$ represents CH;
$X^3$ represents CH or N;
$X^4$ represents O or $NR^4$;
and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein

$R^1$ represents hydrogen, $-CH_2NR^cR^d$, or $-CH_2-C(=O)-NR^cR^d$;
$R^{1a}$ represents $-OR^a$, $-CH_2OR^b$, $-CH_2NR^cR^d$, or $-CH_2-C(=O)-NR^cR^d$.

In an embodiment, the present invention relates to those compounds of Formula (I) and the pharmaceutically acceptable salts, and the solvates thereof, or any subgroup thereof as mentioned in any of the other embodiments, wherein

$R^1$ represents hydrogen, $-CH_2NR^cR^d$, or $-CH_2-C(=O)-NR^cR^d$;
$R^{1a}$ represents $-OR^a$ or $-CH_2OR^b$.

In an embodiment, the present invention relates to those compounds of Formula (I) and the pharmaceutically acceptable salts, and the solvates thereof, or any subgroup thereof as mentioned in any of the other embodiments, wherein

$R^a$ is selected from the group consisting of hydrogen, $C_{1-4}$alkyl, and $C_{1-4}$alkyl substituted with one substituent selected from the group consisting of $Het^1$ and $-OR^e$;
$R^b$ is selected from the group consisting of hydrogen, $C_{1-4}$alkyl, and $C_{1-4}$alkyl substituted with one substituent selected from the group consisting of $Het^1$ and $-OR^e$.

In an embodiment, the present invention relates to those compounds of Formula (I) and the pharmaceutically acceptable salts, and the solvates thereof, or any subgroup thereof as mentioned in any of the other embodiments, wherein $X^1$, $X^2$ and $X^3$ represent CH.

In an embodiment, the present invention relates to those compounds of Formula (I) and the pharmaceutically acceptable salts, and the solvates thereof, or any subgroup thereof as mentioned in any of the other embodiments, wherein $X^1$ and $X^3$ represent N, and $X^2$ represents CH.

In an embodiment, the present invention relates to those compounds of Formula (I) and the pharmaceutically acceptable salts, and the solvates thereof, or any subgroup thereof as mentioned in any of the other embodiments, wherein $Het^1$ represents morpholinyl, in particular 1-morpholinyl.

In an embodiment, the present invention relates to those compounds of Formula (I) and the pharmaceutically acceptable salts, and the solvates thereof, or any subgroup thereof as mentioned in any of the other embodiments, wherein $Het^3$ represents 1H-pyrazolyl, in particular 1H-pyrazol-4-yl.

In an embodiment, the present invention relates to those compounds of Formula (I) and the pharmaceutically acceptable salts, and the solvates thereof, or any subgroup thereof as mentioned in any of the other embodiments, wherein $R^c$ and $R^d$ are taken together to form together with the N-atom to which they are attached

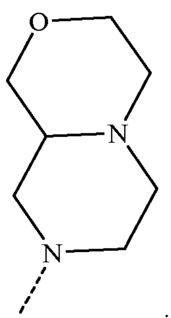

In an embodiment, the present invention relates to those compounds of Formula (I) and the pharmaceutically acceptable salts,

acceptable salts, and the solvates thereof, or any subgroup thereof as mentioned in any of the other embodiments, wherein $Het^1$ represents 1-morpholinyl; and $Het^3$ represents 1H-pyrazol-4-yl.

In an embodiment, the present invention relates to those compounds of Formula (I) and the pharmaceutically acceptable salts, and the solvates thereof, or any subgroup thereof as mentioned in any of the other embodiments, wherein $Het^1$ represents 1-morpholinyl; $Het^3$ represents 1H-pyrazol-4-yl; and wherein $R^c$ and $R^d$ are taken together to form together with the N-atom to which they are attached

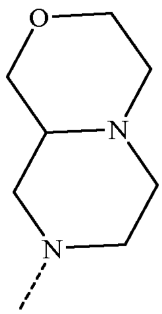

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-a):

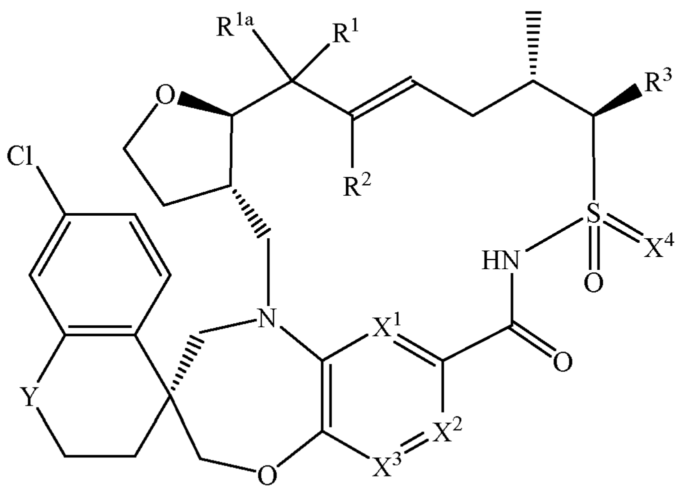

(I-a)

It will be clear that all variables in the structure of Formula (I-a), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-b):

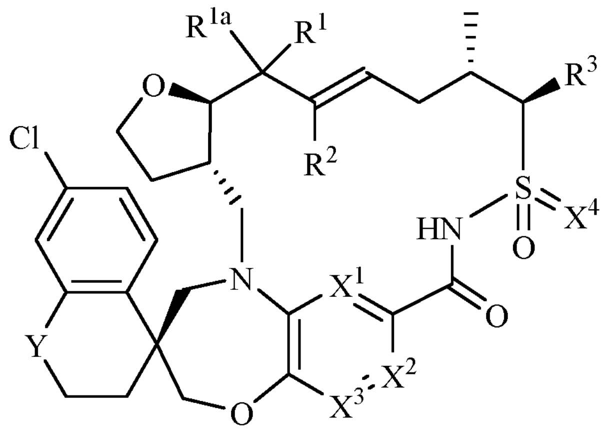

(I-b)

It will be clear that all variables in the structure of Formula (I-b), are defined as defined for the compounds of Formula (I) or any subgroup thereof as mentioned in any of the other embodiments.
In an embodiment, the present invention relates to a subgroup of Formula (I) as defined in the general reaction schemes.
In an embodiment the compound of Formula (I) is selected from the group consisting of any of the exemplified compounds,
and the free bases, the pharmaceutically acceptable salts, and the solvates thereof.
All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.
The general preparation of some typical examples of the compounds of Formula (I) is described hereunder and in the specific examples, and are generally prepared from starting materials which are either commercially available or can be prepared by published methods. The following schemes are only meant to represent examples of the invention and are in no way meant to be a limit of the invention.
Alternatively, compounds of the present invention may also be prepared by analogous reaction protocols as described in the general schemes below, combined with standard synthetic processes commonly used by those skilled in the art including also analogous reaction protocols as described in WO2016033486, WO2017147410 and WO2018183418.
The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.
The skilled person will realize that in the reactions described in the Schemes, it may be advisable or necessary to perform the reaction under an inert atmosphere, such as for example under $N_2$-gas atmosphere.
It will be apparent for the skilled person that it may be necessary to cool the reaction mixture before reaction work-up (refers to the series of manipulations required to isolate and purify the product(s) of a chemical reaction such as for example quenching, column chromatography, extraction).
The skilled person will realize that heating the reaction mixture under stirring may enhance the reaction outcome. In some reactions microwave heating may be used instead of conventional heating to shorten the overall reaction time.
The skilled person will realize that another sequence of the chemical reactions shown in the Schemes below, may also result in the desired compound of Formula (I).
The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.
The meaning of the chemical abbreviations used in the schemes below are as defined in Table 1.
Compounds of Formula (I), wherein $X^1$, $X^2$, $X^3$, $X^4$, Y, $R^1$ and $R^3$ are as defined in Formula (I), and wherein $R^{1a}$ and

$R^2$ are defined as H (hydrogen) and $X^4$ is defined as O; hereby referred to as compounds of Formula (Ia), can be prepared according to Scheme 1,

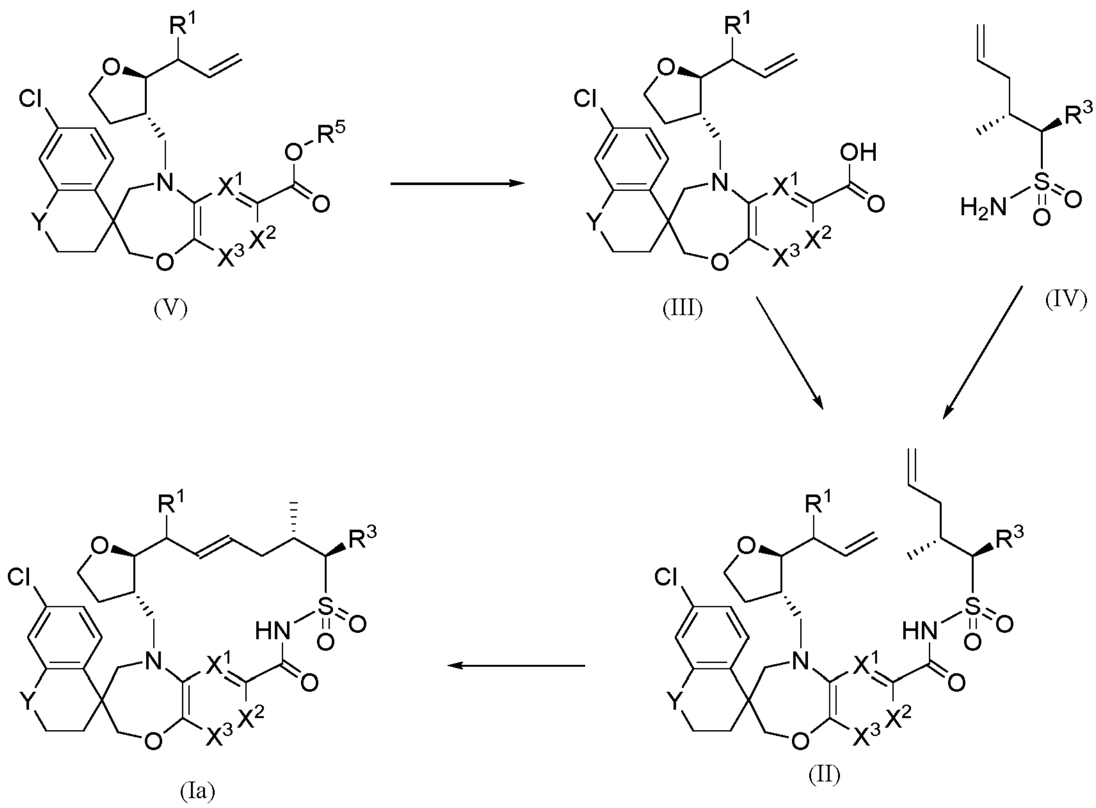

Scheme 1

- by reacting an intermediate of Formula (II), in the presence of a suitable ring closing metathesis catalyst such as, for example, Grubbs-Hoveyda 2nd generation catalyst, in a suitable solvent such as dichloroethane, at a suitable temperature such as room temperature or 60 °C.
- Intermediates of Formula (II) can be prepared by reacting an intermediate of Formula (III), with an intermediate of Formula (IV), in a suitable solvent such as, for example, dichloromethane (DCM), in the presence of a suitable base such as, for example, 4-dimethylaminopyridine (DMAP), and in the presence of a suitable coupling agent such as, for example, 1-ethyl-3-(3-dimethylaminopropyl)carbodiimide hydrochloride (EDC.HCl), at a suitable temperature such as, for example, room temperature.
- Intermediates of Formula (III) can be prepared by reacting an intermediate of Formula (V) with a suitable base such as, for example, LiOH or NaOH, in a suitable solvent such as, for example, water, or a suitable mixture of solvents such as, for example, water and methanol (MeOH), or a mixture of water, MeOH, and tetrahydrofuran (THF), at a suitable temperature such as, for example, room temperature, or 60 °C.

In case $R^1$ in compounds of Formula (Ia) is an alcohol such as, for instance, -OH or - $CH_2OH$, this alcohol might be protected with a suitable protective group such as, for instance, tert-butyldimethylsilyl (TBS). In such case, a deprotection can be done with a suitable reagent such as, tetrabutylammonium fluoride (TBAF), in a suitable solvent such as, for instance, THF, at a suitable temperature such as, for instance, room temperature.

In case $R^1$ in compounds of Formula (Ia) is an alcohol such as, for instance, -OH or - $CH_2OH$, a subsequent alkylation can be done with a suitable alkyl halide such as, for instance, methyl iodide or 4-(2-bromoethyl)morpholine, in presence of a suitable base such as, for instance, sodium hydride, in a suitable solvent such as, for instance, THF, at a suitable temperature such as, for instance, 45 °C.

Compounds of Formula (I), wherein $X^1$, $X^2$, $X^3$, Y, $R^1$ and $R^3$ are as defined in Formula (I), and wherein $R^{1a}$ and $R^2$ are defined as H (hydrogen), and $X^4$ is defined as $NR^4$ in which $R^4$ is as defined in Formula (I); hereby referred to as compounds of Formula (Ib), can be prepared according to Scheme 2,

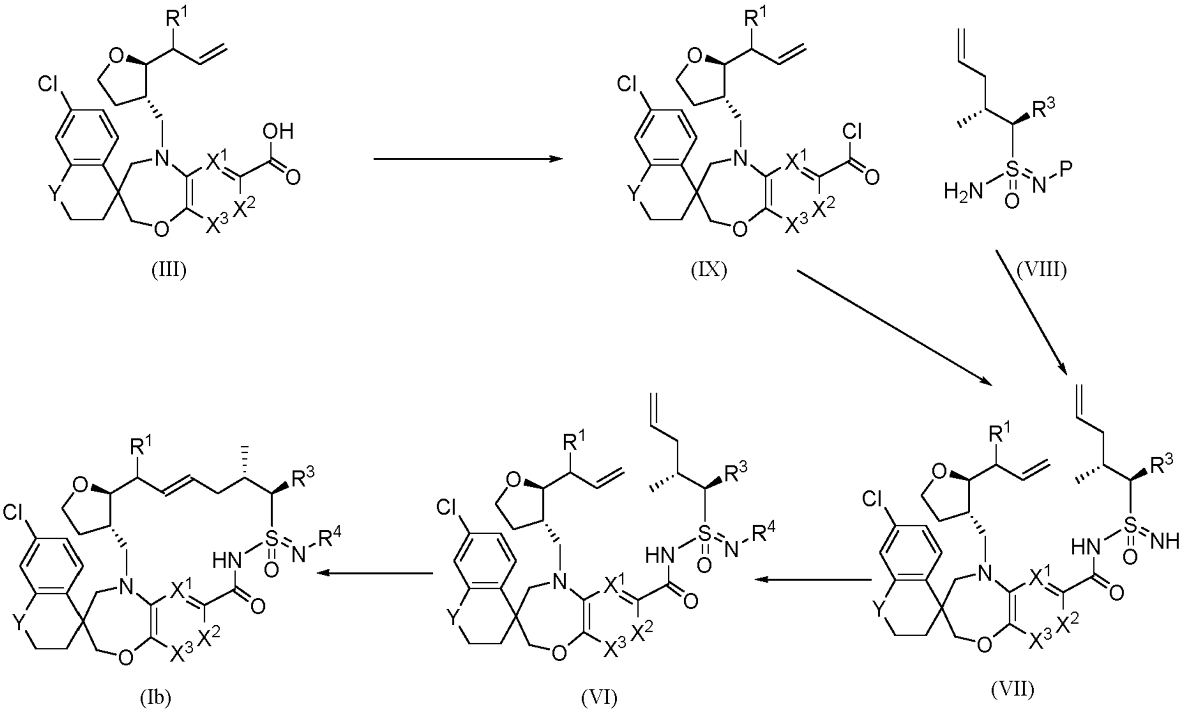

Scheme 2

- by reacting an intermediate of Formula (VI), in the presence of a suitable ring closing metathesis catalyst such as, for example, Grubbs-Hoveyda 2nd generation catalyst ((1,3-bis-(2,4,6-trimethylphenyl)-2-imidazolidinylidene)dichloro(o-isopropoxyphenylmethylene)ruthenium), in a suitable solvent such as dichloroethane, at a suitable temperature such as room temperature or 60 °C.
- Intermediates of Formula (VI) can be prepared by reacting an intermediate of Formula (VII) with a suitable carboxylic acid such as, for instance, 3-methoxy-1-methyl-1H-pyrazole-4-carboxylic acid, with a suitable amide coupling reagent such as, for instance, EDC.HCl, in presence of a suitable base such as, for instance, DMAP, in a suitable solvent such as, for instance, DCM, at a suitable temperature such as, for instance, room temperature.
- Intermediates of Formula (VII) can be prepared by reacting an intermediate of Formula (IX), with an intermediate of Formula (VIII), in a suitable solvent such as, for example, acetonitrile (ACN), in the presence of a suitable base such as, for example, pyridazine, at a suitable temperature such as, for example, room temperature. A skilled person will understand that some protective groups such as, for instance, tert-butyldimethylsilyl (TBS), may be removed during this reaction step, while other protective groups may need an additional deprotection step.
- Intermediates of Formula (IX) can be prepared by reacting an intermediate of Formula (III) with a suitable activating reagent such as, for instance, thionyl chloride, in a suitable solvent such as, for instance, DCM, at a suitable temperature such as, for instance, room temperature.

In case $R^1$ in compounds of Formula (Ib) is an alcohol such as, for instance, -OH or - $CH_2OH$, this alcohol might be protected with a suitable protective group such as, for instance, tert-butyldimethylsilyl (TBS). In such case, a deprotection can be done with a suitable reagent such as, TBAF, in a suitable solvent such as, for instance, THF, at a suitable temperature such as, for instance, room temperature.

In case $R^1$ in compounds of Formula (Ib) is an alcohol such as, for instance, -OH or - $CH_2OH$, a subsequent alkylation can be done with a suitable alkyl halide such as, for instance, methyl iodide or 4-(2-bromoethyl)morpholine, in presence of a suitable base such as, for instance, sodium hydride, in a suitable solvent such as, for instance, THF, at a suitable temperature such as, for instance, 45 °C.

Compounds of Formula (I), wherein $X^1$, $X^2$, $X^3$, Y, $R^1$, $R^a$ are as defined in Formula (I), and wherein $R^{1a}$ is defined as H (hydrogen), $R^2$ is defined as F (fluorine) and $X^4$ is defined as O or $NR^4$; hereby referred to as compounds of Formulas (Ic) and (Id), can be prepared according to Scheme 3,

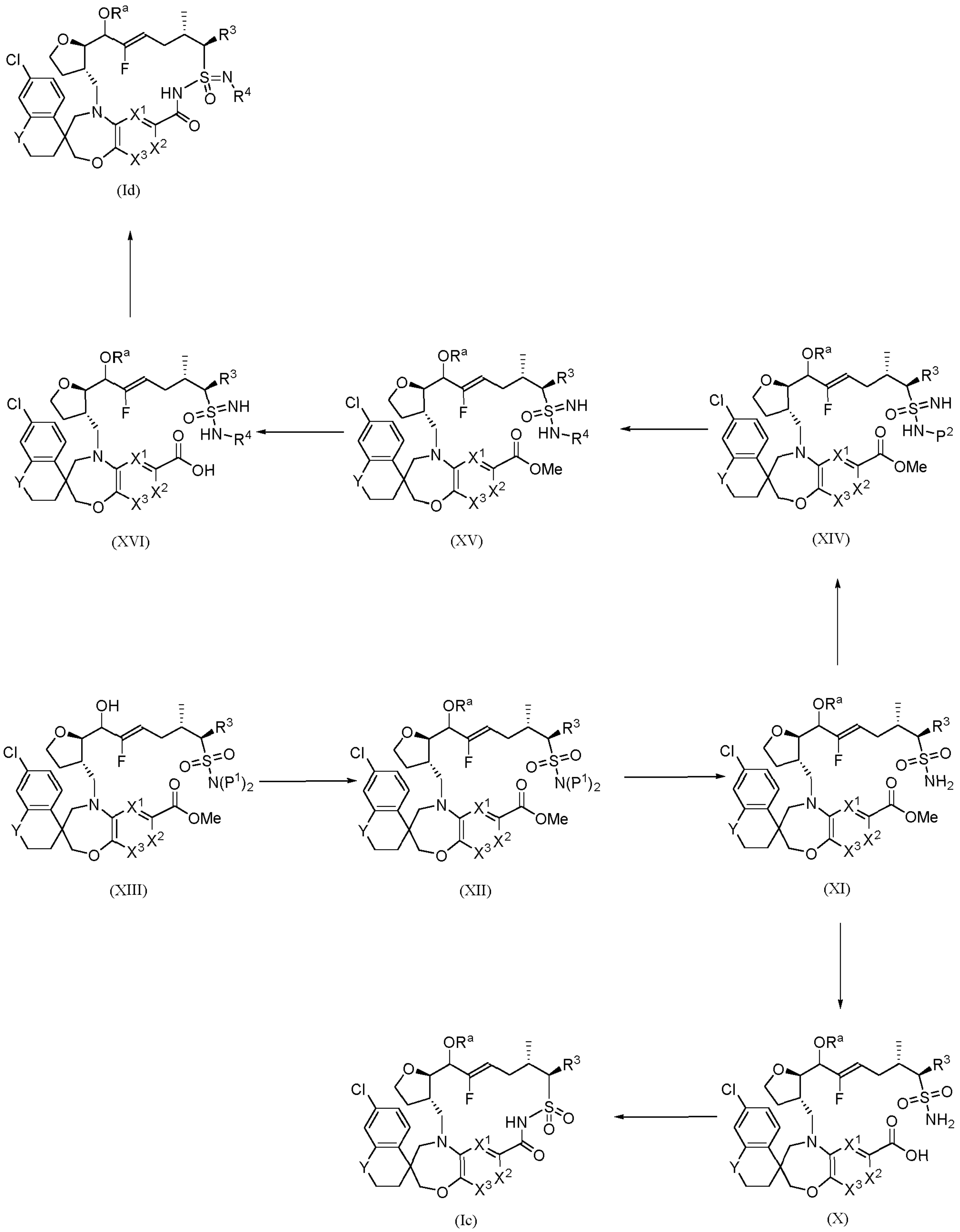

(Id)

(XVI) (XV) (XIV)

(XIII) (XII) (XI)

(Ic) (X)

Scheme 3

- by intramolecularly reacting intermediates of Formulas (X) and (XVI) with a suitable coupling agent such as, for example, diethyl chlorophosphate (DECP) or EDC.HCl, in presence of a suitable base such as, for example, DMAP or triethylamine ($Et_3N$), in a suitable solvent such as, for example, N,N-dimethylformamide (DMF), at a suitable temperature such as, for example, room temperature.

- Intermediates of Formula (X) can be prepared by reacting an intermediate of Formula (XI) with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature or 60 °C.
- Intermediates of Formula (XI) can be prepared by reacting an intermediate of Formula (XII), wherein $P^1$ is a suitable protecting group such as, for example, p-methoxy benzyl (PMB), with a suitable deprotecting agent such as, for example, trifuloroacetic acid (TFA), in a suitable solvent such as, for example, DCM or neat, at a suitable temperature such as, for example, room temperature or 0 °C.
- Intermediates of Formula (XII) can be prepared by reacting an intermediate of Formula (XIII) with a suitable alkylation agent such as, for example, MeI, in presence of a suitable base such as, for example, NaH, in a suitable solvent such as, for example, DMF or tetrahydrofuran (THF), at a suitable temperature such as, for example, room temperature, or 50 °C.
- Intermediates of Formula (XIV) can be prepared by (1) protecting intermediate of Formula (XI) with a suitable protecting group $P^2$ such as, for example, TBS, using a suitable base such as, for example, triethylamine or NaH, in a suitable solvent such as, for example, tetrahydrofuran, at a suitable temperature such as, for example, room temperature or 60 °C; (2) reacting it with a suitable chlorinating reagent such as, for example, $Ph_3PCl_2$, in a suitable solvent such as, for example, DCM, at a suitable temperature such as, for example, 0 °C; and (3) reacting it with a suitable amine such as, for example, gaseous ammonia at a suitable temperature such as, for example, 0 °C.
- Intermediates of Formula (XV) can be prepared by reacting an intermediate of Formula (XIV) with a suitable acylation agent such as, for example, alkyl and (hetero)aryl acyl chloride, in presence of a suitable base such as, for example, pyridazine, in a suitable solvent such as, for example, acetonitrile (MeCN), at a suitable temperature such as, for example, room temperature. A skilled person will understand that some protective groups such as, for instance, TBS, may be removed during this reaction step, while other protective groups may need an additional deprotection step.
- Intermediates of Formula (XVI) can be prepared by reacting an intermediate of Formula (XV) with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.

Compounds of Formula (I), wherein $X^1$, $X^2$, $X^3$, Y, are as defined in Formula (I), and wherein $R^{1a}$ is defined as $-CH_2NR^cR^d$, $R^1$ is defined as $-OR^a$, $R^2$ is defined as F (fluorine) and $X^4$ is defined as O or $NR^4$; hereby referred to as compounds of Formulas (Ie) and (If), can be prepared according to Scheme 4,

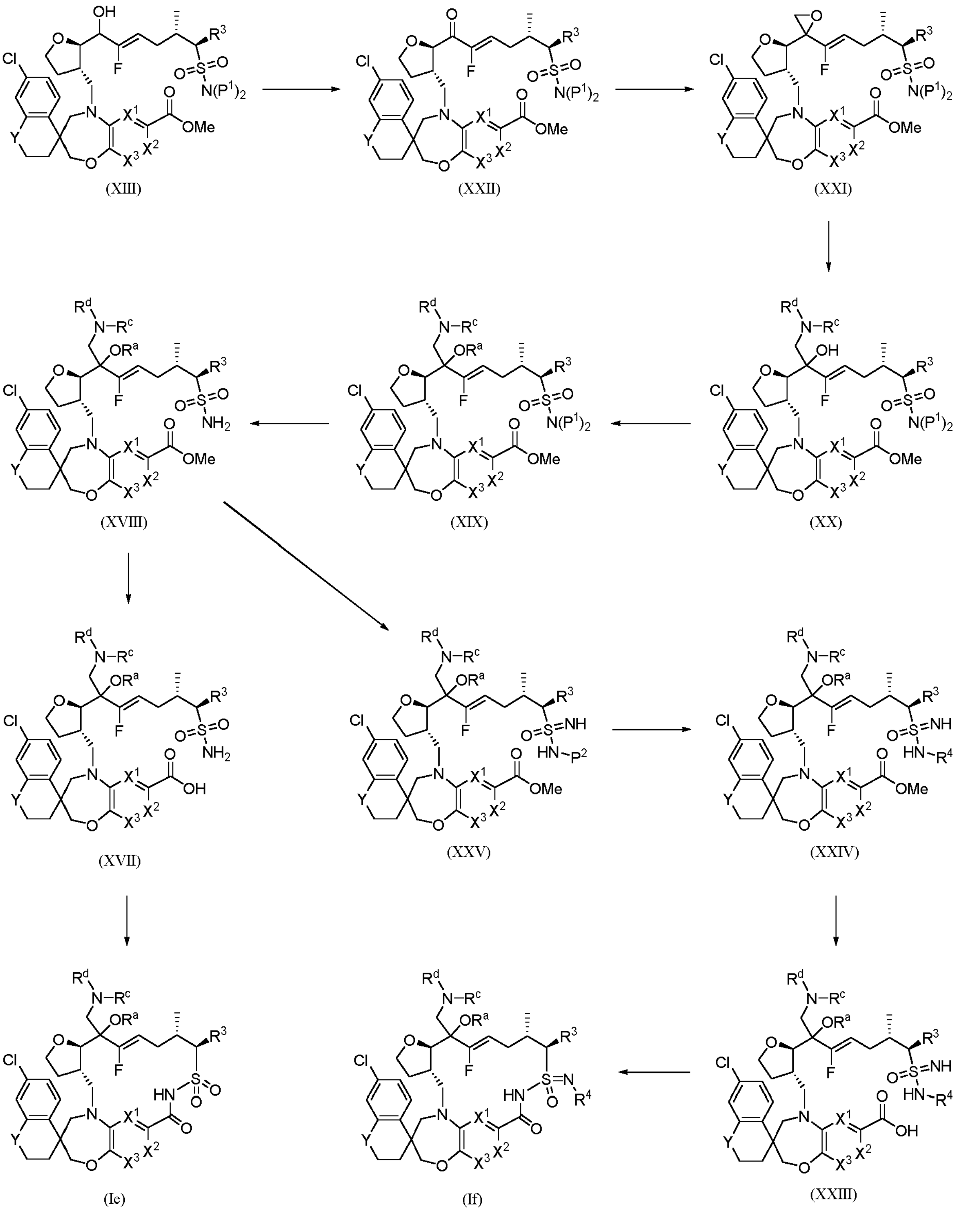

Scheme 4

- By intramolecularly reacting intermediates of Formulas (XVII) or (XXIII), respectively for (Ie) or (If), with a suitable coupling agent such as, for example, DECP or EDC.HCl, in presence of a suitable base such as, for example, DMAP or $Et_3N$, in a suitable solvent such as, for example, DMF, at a suitable temperature such as, for example, room temperature.
- Intermediates of Formula (XVII) can be prepared by reacting an intermediate of Formula (XVIII) with a suitable base such as, for example, LiOH or NaOH, in a suitable solvent such as, for example, water, or a suitable mixture of solvents

such as, for example, water and MeOH, or a mixture of water, MeOH, and THF, at a suitable temperature such as, for example, room temperature, or 60 °C.

- Intermediates of Formula (XVIII) can be prepared by reacting an intermediate of Formula (XIX), wherein $P^1$ is a suitable protecting group such as, for example, PMB, with a suitable deprotecting agent such as, for example, TFA, in a suitable solvent such as, for example, DCM or neat, at a suitable temperature such as, for example, room temperature or 0 °C.
- Intermediates of Formula (XIX) can be prepared by reacting an intermediate of Formula (XX) with an alkylating agent such as, for example, MeI, with a suitable base such as, for example, NaH, in suitable solvent such as, for example, DMF or THF, at a suitable temperature such as, for example, room temperature, or 50 °C.
- Intermediates of Formula (XX) can be prepared by reacting an intermediate of Formula (XXI) with an amine such as, for example, (*S*)-octahydropyrazino[2,1-c][1,4]oxazine dihydrochloride, in presence of a suitable base such as, for example, $Et_3N$, in a suitable solvent such as, for example, MeOH, at a suitable temperature such as, for example, 70 °C.
- Intermediates of Formula (XXI) can be prepared by reacting an intermediate of Formula (XXII) with a Corey-Chaykovsky reagent such as, for example, trimethylsulfoxonium iodide, with a suitable base such as, for example, potassium tert-butoxide (KOtBu), in suitable solvents such as, for example, dimethylsulfoxide (DMSO), at a suitable temperature such as, for example, room temperature, or 15 °C.
- Intermediates of Formula (XXII) can be prepared by reacting an intermediate of Formula (XIII), with a suitable oxidant such as, for example, Dess-Martin periodinane (DMP), and in a suitable solvent such as, for example, DCM, at a suitable temperature such as, for example, room temperature.
- Intermediates of Formula (XXIII) can be prepared by reacting an intermediate of Formula (XXIV) with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.
- Intermediates of Formula (XXIV) can be prepared by reacting an intermediate of Formula (XXV) with a suitable acylation agent such as, for example, alkyl and (hetero)aryl acyl chloride, in presence of a suitable base such as, for example, pyridazine, in a suitable solvent such as, for example, MeCN, at a suitable temperature such as, for example, room temperature. A skilled person will understand that some protective groups such as, for instance, TBS, may be removed during this reaction step, while other protective groups may need an additional deprotection step.
- Intermediates of Formula (XXV) can be prepared by (1) protecting intermediate of Formula (XVIII) with a suitable protecting group $P^2$ such as, for example, tert-butyl dimethylsilyl (TBS), using a suitable base such as, for example, triethylamine orNaH, in a suitable solvent such as, for example, tetrahydrofuran, at a suitable temperature such as, for example, room temperature or 60 °C; (2) reacting it with a suitable chlorinating reagent such as, for example, triphenylphosphine dichloride ($Ph_3PCl_2$), in a suitable solvent such as, for example, DCM, at a suitable temperature such as, for example, 0 °C; and (3) reacting it with a suitable amine such as, for example, gaseous ammonia at a suitable temperature such as, for example, 0 °C.

Compounds of Formula (III) and (XIII), wherein $X^1$, $X^2$, $X^3$, Y, $R^a$ are as defined in Formula (I), $R^1$ is OH or $CH_2OH$, and $R^5$ is a suitable alkyl group such as, for instance, methyl, can be prepared according to Scheme 5,

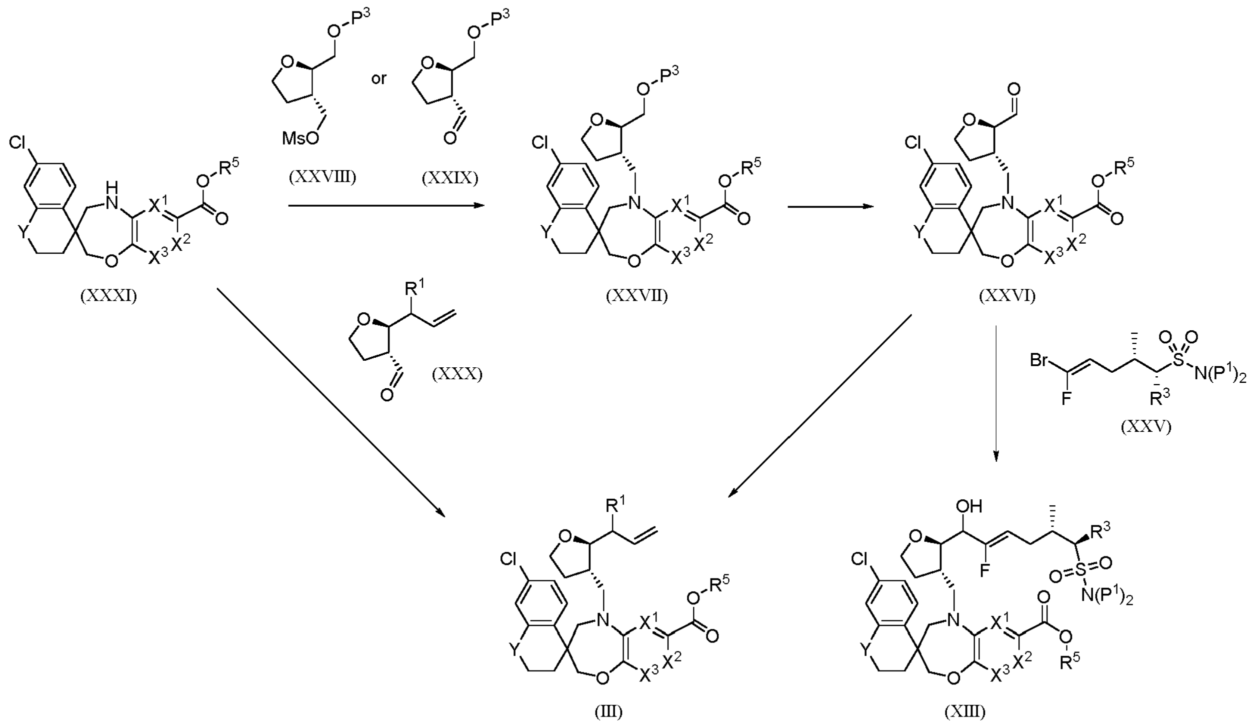

Scheme 5

- By reacting an intermediate of Formula (XXXI) with and intermediate of Formula (XXX) or a benzotriazole adduct thereof, in the presence of a suitable acid such as, for example, acetic acid (AcOH), and in the presence of a suitable reducing agent such as, for example, sodium triacetoxyborohydride ($NaBH(OAc)_3$), in a suitable solvent such as, for example, DCM, at a suitable temperature such as, for example, 0 °C or room temperature.
- Alternatively, by reacting an intermediate of Formula (XXVI) with a suitable alkene nucleophile such as, for instance diethenylzinc, in a suitable solvent such as, for instance, toluene, at a suitable temperature such as, for instance, -15 °C.
- Intermediates of Formula (XXVI) can be prepared by reacting an intermediate of Formula (XXVII) in which $P^3$ is a H (hydrogen) with a suitable oxidizing reagent such as, for instance, DMSO and oxalyl choride, in the presence of a suitable base such as, for instance, triethylamine, in a suitable solvent such as, for instance, DCM, at a suitable temperature such as, for instance, -78 °C or -20 °C. A skilled person will understand that in case $P^3$ is a protective group such as, for instance, benzyl or tert-butyl(chloro)diphenylsilane (TBDPS), a deprotection to the corresponding alcohol is first required under art known conditions.
- Intermediates of Formula (XXVII) can be prepared by reacting an intermediate of Formula (XXXI), wherein one or more $X^1$, $X^2$, $X^3$ are N (nitrogen), with a suitable intermediate of Formula (XXVIII), in presence of a suitable base such as, for instance, NaH, in a suitable solvent such as, for instance, DMF, at a suitable temperature such as, for instance, room temperature or 50 °C.
- Alternatively, intermediates of Formula (XXVII) can be prepared by reacting an intermediate of Formula (XXXI), wherein $X^1$, $X^2$, $X^3$ are CH, with a suitable intermediate of Formula (XXIX), or a benzotriazole adduct thereof, in the presence of a suitable acid such as, for example, AcOH, and in the presence of a suitable reducing agent such as, for example, $NaBH(OAc)_3$, in a suitable solvent such as, for example, DCM, at a suitable temperature such as, for example, 0 °C or room temperature.
- Intermediates of Formula (XIII), wherein $X^1$, $X^2$, $X^3$, Y, are as defined in Formula (I), and wherein $R^{1a}$ is defined as H (hydrogen), $R^1$ is defined as $-OR^5$ or $CH_2OR^5$, $R^2$ is defined as F (fluorine) and $X^4$ is defined as O, can be prepared by reacting an intermediates of Formula (XXVI) with a suitable intermediate of Formula (XXV), wherein $R^3$ is defined as in Formula I, and $P^1$ is a suitable protective group such as, for instance, p-methoxybenzyl (PMB), with a suitable metalation reagent such as, for instance, $CrCl_2$ and $NiBr_2$, in the presence of a suitable ligand such as, for instance, ethylene diamine, in a suitable solvent such as, for instance, DMF, at a suitable temperature such as, for instance, room temperature.

In case $R^1$ in compounds of Formula (III) is an alcohol such as, for instance, -OH or $-CH_2OH$, this alcohol might be protected with a suitable protective group such as, for instance, tert-butyldimethylsilyl (TBS).

Alternatively, in case $R^1$ in compounds of Formula (III) is an alcohol such as, for instance, -OH or $-CH_2OH$, an alkylation can be done with a suitable alkyl halide such as, for instance, methyl iodide, in presence of a suitable base such as, for instance, sodium hydride, in a suitable solvent such as, for instance, THF, at a suitable temperature such as, for instance, 45 °C.

Intermediates of Formula (XXX), wherein $R^1$ is $OP^4$, and wherein $P^4$ is a suitable protective group such as, for instance, tert-butyldimethylsilyl (TBS), hereby named intermediates of Formula (XXX-a) can be prepared according to Scheme 6,

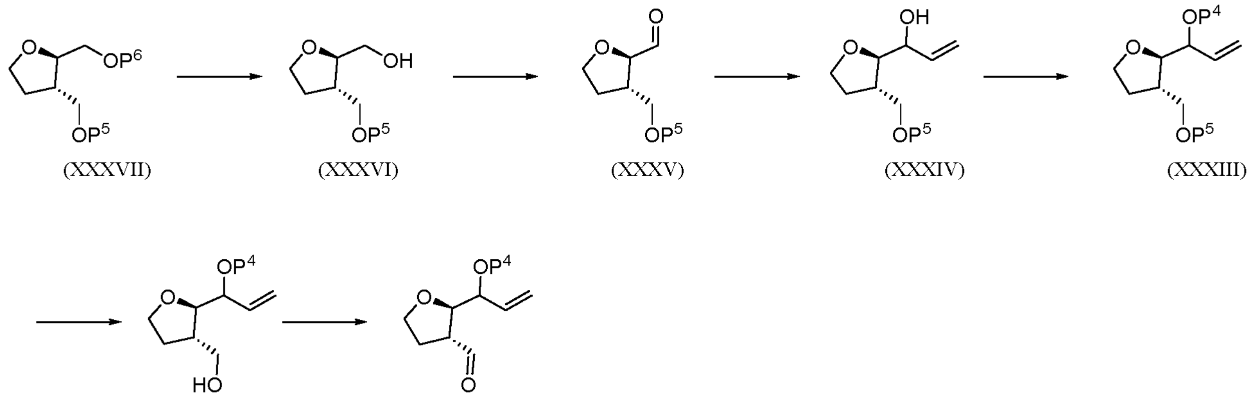

(XXXVII) (XXXVI) (XXXV) (XXXIV) (XXXIII)

(XXXII) (XXX-a)

Scheme 6

- By reacting an intermediate of Formula (XXXII), with a suitable oxidant such as, for example, DMP, and in a suitable solvent such as, for example, DCM, at a suitable temperature such as, for example, room temperature.
- Intermediates of Formula (XXXII) can be prepared by reacting an intermediate of Formula (XXXIII) in which $P^5$ is a suitable protective group such as, for instance, benzoyl (Bz), with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.
- Intermediates of Formula (XXXIII), in which $P^4$ is a suitable protective group such as, for instance, tert-butyldimethylsilyl (TBS), can be prepared by reacting an intermediate of Formula (XXXIV) with a suitable protecting reagent such as, for instance tert-butyl(chloro)dimethylsilane (TBSCl), in the presence of suitable base such as, for instance imidazole, in a suitable solvent such as, for instance, DMF, at a suitable temperature such as, for instance, room temperature.
- Intermediates of Formula (XXXIV) can be prepared by reacting an intermediate of Formula (XXXV) with a suitable alkene nucleophile such as, for instance vinylmagnesium bromide or an in-situ generated vinylzinc reagent, in a suitable solvent such as, for instance, toluene, at a suitable temperature such as, for instance, -78 °C or -15 °C.
- Intermediates of Formula (XXXV) can be prepared by reacting an intermediate of Formula (XXXVI), with a suitable oxidant such as, for example, DMSO/oxalyl chloride/$Et_3N$, in a suitable solvent such as, for example, DCM, at a suitable temperature such as, for example, -78°C.
- Intermediates of Formula (XXXVI) can be prepared by reacting an intermediate of Formula (XXXVII), wherein $P^6$ is a suitable protecting group such as, for example, benzyl, with a suitable deprotecting agent such as, for example, Pd/C under $H_2$ atmosphere, in a suitable solvent such as, for example, ethyl acetate (EtOAc) or MeOH, at a suitable temperature such as, for example, room temperature.

Intermediates of Formula (XXX), wherein $R^1$ is $CH_2OP^7$, and wherein $P^7$ is a suitable protective group such as, for instance, tert-butyldimethylsilyl (TBS), hereby named intermediates of Formula (XXX-b) can be prepared according to Scheme 7,

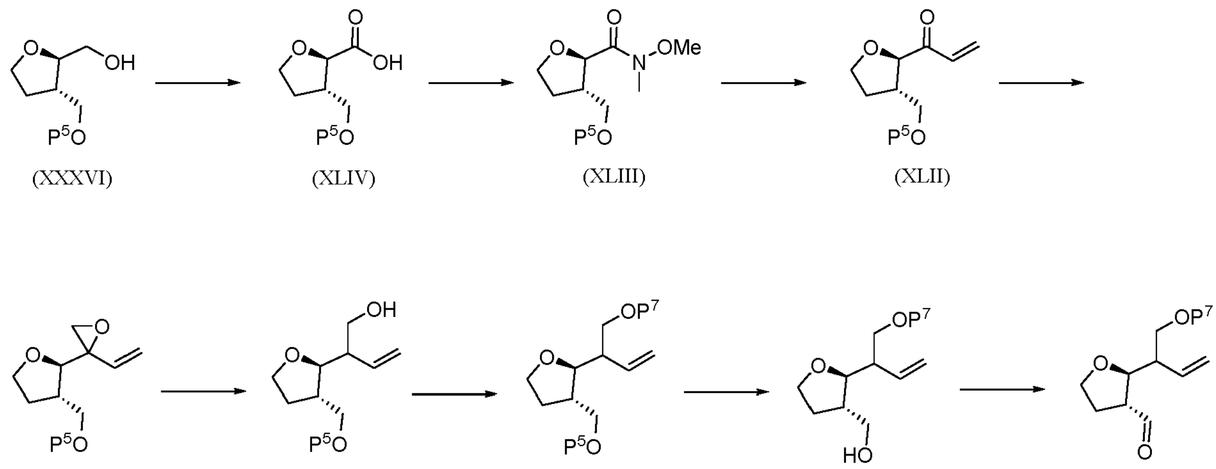

(XXXVI) (XLIV) (XLIII) (XLII)

(XLI) (XL) (XXXIX) (XXXVIII) (XXX-b)

Scheme 7

- By reacting an intermediate of Formula (XXXVIII), with a suitable oxidant such as, for example, DMP, and in a suitable solvent such as, for example, DCM, at a suitable temperature such as, for example, room temperature.
- Intermediates of Formula (XXXVIII) can be prepared by reacting an intermediate of Formula (XXXIX) with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.
- Intermediates of Formula (XXXIX), wherein $P^7$ is a suitable protective group such as, for instance, tert-butyldimethylsilyl (TBS), can be prepared by reacting an intermediate of Formula (XL) with a suitable protecting reagent such as, for instance TBSCl, in the presence of suitable base such as, for instance imidazole, in a suitable solvent such as, for instance, DMF, at a suitable temperature such as, for instance, room temperature.
- Intermediates of Formula (XL) can be prepared by reacting an intermediate of Formula (XLI) with a suitable reductant such as, for example, formic acid, in the presence of a suitable catalyst such as, for instance, tributylphosphine and tris(dibenzylideneacetone)dipalladium-chloroform, with a suitable base such as, for example, $Et_3N$, in suitable solvent such as, for example, THF, at a suitable temperature such as, for example, room temperature.
- Intermediates of Formula (XLI) can be prepared by reacting an intermediate of Formula (XI,II) with a suitable reagent such as, for example, trimethylsulfoxonium iodide, with a suitable base such as, for example, potassium bis(trimethylsilyl)amide (KHMDS), in a suitable solvent such as, for example, THF, at a suitable temperature such as, for example, room temperature or 15 °C.
- Intermediates of Formula (XI,II) can be prepared by reacting an intermediate of Formula (XI,III) with a suitable alkene nucleophile such as, for instance vinylmagnesium bromide, in a suitable solvent such as, for instance, toluene, at a suitable temperature such as, for instance, 0 °C.
- Intermediates of Formula (XI,III) can be prepared by reacting an intermediate of Formula (XLIV) with a suitable primary amine, such as, for example, *N,O*-dimethylhydroxylamine hydrochloride, with a suitable coupling agent such as, for example, EDC.HCl, in presence of a suitable base such as, for example, *N*-methylmorpholine (NMM), in a suitable solvent such as, for example, DCM, at a suitable temperature such as, for example, room temperature.
- Intermediates of Formula (XLIV) can be prepared by reacting an intermediate of Formula (XXXVI), wherein $P^5$ is a suitable protective group such as, for instance, benzoyl (Bz), with a suitable oxidant such as, for example, 2,2,6,6-tetramethylpiperidine-1-oxyl (TEMPO) and (diacetoxyiodo)benzene (PIDA), in a suitable solvent mixture such as, for example,acetonitrile and water, at a suitable temperature such as, for example, room temperature.

Intermediates of Formula (XXVIII) or (XXIX), wherein $R^1$ is $OP^3$, and wherein $P^3$ is a suitable protective group such as, for instance, tert-butyldimethylsilyl (TBS), can be prepared according to Scheme 8,

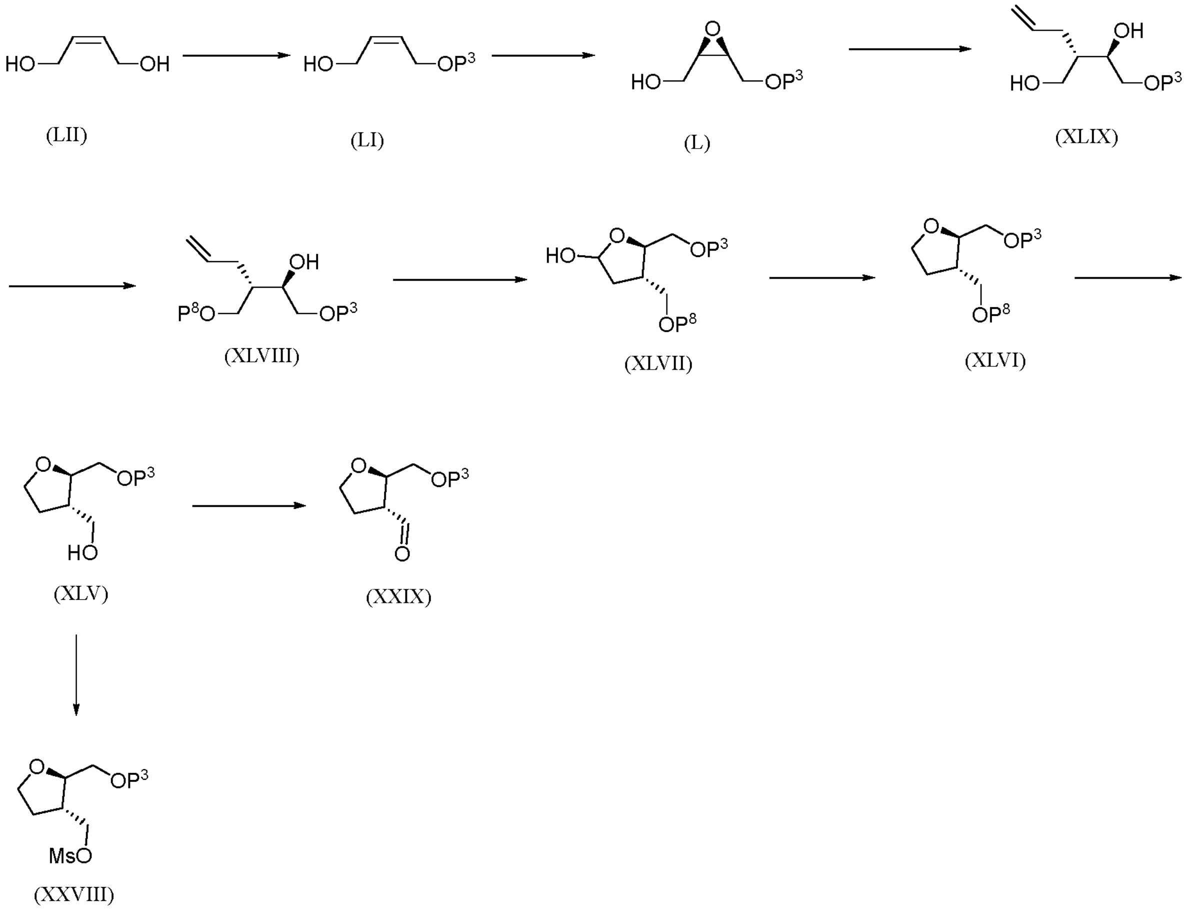

(LII) (LI) (L) (XLIX)

(XLVIII) (XLVII) (XLVI)

(XLV) (XXIX)

(XXVIII)

Scheme 8

- Intermediates of Formula (XXVIII) can be prepared by reacting an intermediate of Formula (XLV) with mesyl chloride in presence of a suitable base such as, for example, triethylamine, in a suitable solvent such as, for example, DCM, at a suitable temperature such as, for example, 0 °C.
- Intermediates of Formula (XXIX) can be prepared by reacting an intermediate of Formula (XLV), with a suitable oxidant such as, for example, DMP, and in a suitable solvent such as, for example, DCM, at a suitable temperature such as, for example, room temperature.
- Intermediates of Formula (XLV) can be prepared by reacting an intermediate of Formula (XLVI), wherein $P^8$ is a suitable protecting group such as, for example, Bz, with a suitable deprotecting agent such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.
- Intermediates of Formula (XLVI) can be prepared by reacting an intermediate of Formula (XLVII) with suitable reductant such as, for example, triethylsilane, with a suitable acid such as boron trifluoride etherate, in a suitable mixture of solvents such as, for example, DCM and acetonitrile, at a suitable temperature such as, for example, 0 °C.
- Intermediates of Formula (XLVII) can be prepared by reacting an intermediate of Formula (XLVIII) with a suitable oxidizing reagent such as, for instance, ozone, in presence of a suitable reductant such as $Me_2S$, or triphenylphosphine ($Ph_3P$), in a suitable mixture solvent such as DCM and MeOH, at a suitable temperature such as, for example, -78 °C.
- Intermediates of Formula (XI,VIII), wherein $P^8$ is a suitable protective group such as, for instance, benzoyl (Bz), can be prepared by reacting an intermediate of Formula (XLIX) with a suitable protecting reagent such as, for instance benzoyl chloride (BzCl), in the presence of suitable base such as, for instance, pyridine, in a suitable solvent such as, for instance, DCM, at a suitable temperature such as, for instance, room temperature.
- Intermediates of Formula (XLIX) can be prepared by reacting an intermediate of Formula (L) with a suitable alkylating reagent such as allylmagnesium bromide, with suitable Lewis acid such as, for example, CuCN, in a suitable solvent such as THF, at a suitable temperature such as, for example, -40°C.
- Intermediates of Formula (L) can be prepared by reacting an intermediates of Formula (LI) with a suitable oxidant such

as, for example, *tert*-butyl hydrogen peroxide, with a suitable catalyst such as, for instance, titanium(IV)isopropoxide ($Ti(OiPr)_4$), in the presence of a suitable ligand such as, for instance, (-)-diethyl-D-tartrate, in a suitable solvent such as, for instance, DCM, at a suitable temperature such as, for instance, -23 °C.

- Intermediates of Formula (LI) can be prepared by reacting an intermediate of Formula (LII) with a suitable protecting reagent such as, for instance BnBr, in the presence of suitable base such as, for instance, NaH, in a suitable mixture of solvent such as, for instance, THF and DMSO, at a suitable temperature such as, for instance, room temperature or 60 °C.

Alternatively, intermediates of Formula (XXIX), wherein $R^1$ is $OP^3$ can also be prepared according to Scheme 9,

Scheme 9

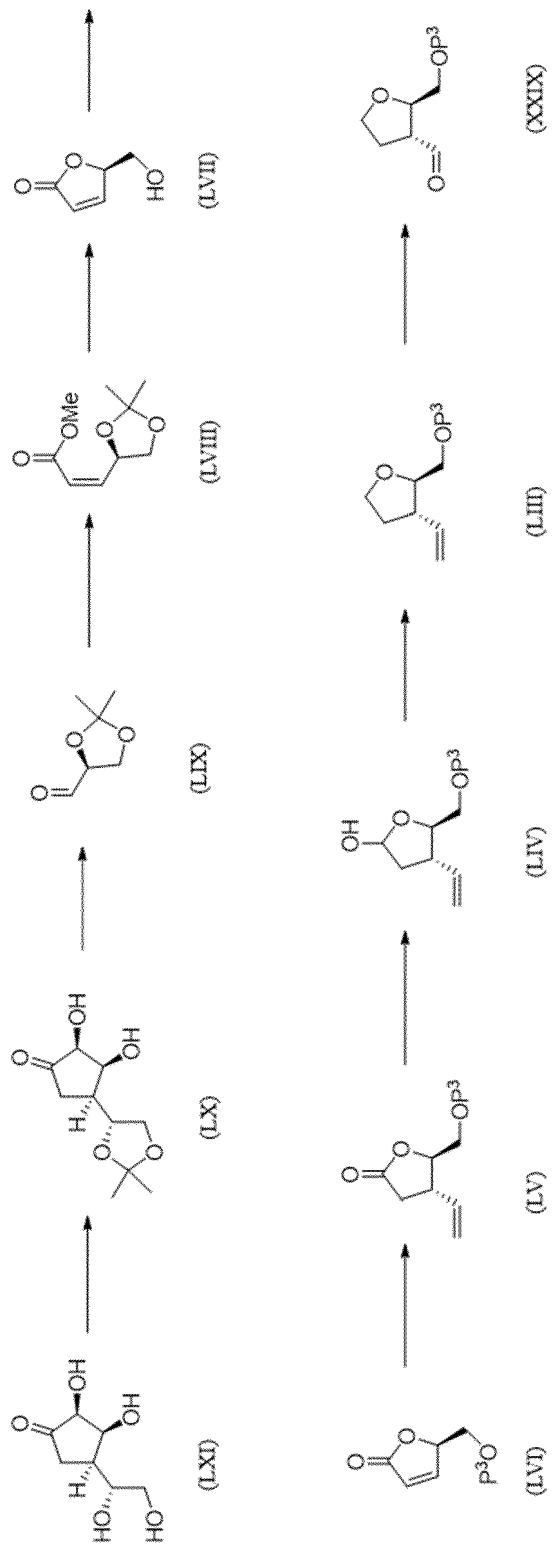
(LXI) (LX) (LIX) (LVIII) (LVII) (LVI) (LV) (LIV) (LIII) (XXIX)

- Intermediates of Formula (XXIX) can also be prepared by reacting an intermediate of Formula (LIII) with a suitable oxidizing reagent such as, for instance, ozone, in presence of a suitable reductant such as $Me_2S$, or $Ph_3P$, in a suitable mixture of solvents such as, for instance, DCM and MeOH, at a suitable temperature such as, for example, -78 °C.
- Intermediates of Formula (LIII) can be prepared by reacting an intermediate of Formula (LIV) with suitable reductant

such as, for example, triethylsilane, with a suitable acid such as boron trifluoride etherate and in a suitable mixture of solvent such as, for example, DCM and acetonitrile, at a suitable temperature such as, for example, 0 °C.

- Intermediates of Formula (LIV) can be prepared by reacting an intermediate of Formula (LV) with suitable reductant such as, for example, di-isobutylaluminiumhydride (DIBAL), in a suitable of solvent such as, for example, $Et_2O$, at a suitable temperature such as, for example, -78 °C.
- Intermediates of Formula (LV) can be prepared by reacting an intermediate of Formula (LVI) with a suitable alkylating reagent such as vinylmagnesium bromide, with suitable Lewis acid such as, for example, copper(I) bromide dimethyl sulfide complex ($CuBrMe_2S$ complex), in a suitable solvent such as THF, at a suitable temperature such as, for example, -78 °C.
- Intermediates of Formula (LVI) can be prepared by reacting an intermediate of Formula (LVII) with a suitable protecting reagent such as, for instance tert-butyl(chloro)diphenylsilane (TBDPSCl), in the presence of suitable base such as, for instance imidazole, in a suitable solvent such as, for instance, DMF, at a suitable temperature such as, for instance, room temperature.
- Intermediates of Formula (LVII) can be prepared by reacting an intermediate of Formula (LVIII), with a suitable acid such as, for example, $H_2SO_4$, and in a suitable solvent such as, for example, MeOH, at a suitable temperature such as, for example, room temperature.
- Intermediates of Formula (LVIII) can be prepared by reacting an intermediate of Formula (LIX) with suitable Wittig reagent such as, for example, methyl (triphenylphosphoranylidene), with a suitable base such as NaOH and in a suitable of solvent mixture such as, for example, water and DCM, at a suitable temperature such as, for example, 0 °C
- Intermediates of Formula (LIX) can be prepared by reacting an intermediate of Formula (LX) with suitable oxidant such as, for example, $NaIO_4$, with a suitable base such as NaOH and in a suitable of solvent such as, for example, water, at a suitable temperature such as, for example, 0 °C.
- Intermediates of Formula (LX) can be prepared by reacting an intermediate of Formula (LXI), with a suitable protecting reagent such as, for example, dimethoxypropane, with a suitable catalyst such as, for instance, p-toluenesulfonic acid (pTSA), and in a suitable solvent such as, for example, THF and DMF, at a suitable temperature such as, for example, 0 °C.
- Intermediate (LXI) can be prepared from ascorbic acid using conditions described in literature.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to methods for treating and / or preventing a cancer, wherein the cancer is selected from those described herein, comprising administering to a subject in need thereof (preferably a human), a therapeutically effective amount of a compound of Formula (I), or pharmaceutically acceptable salt, or a solvate thereof.

In an embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of adenocarcinoma, benign monoclonal gammopathy, biliary cancer (including, but not limited to, cholangiocarcinoma), bladder cancer, breast cancer (including, but not limited to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi’s sarcoma, multiple idiopathic hemorrhagic sarcoma), endometrial cancer (including, but not limited to, uterine cancer, uterine sarcoma), esophageal cancer (including, but not limited to, adenocarcinoma of the esophagus, Barrett’s adenocarinoma), Ewing sarcoma, gastric cancer (including, but not limited to, stomach adenocarcinoma), gastrointestinal stromal tumor (GIST), head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom’s macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell

lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms' tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma,* lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of benign monoclonal gammopathy, breast cancer (including, but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), and prostate cancer (including, but not limited to, prostate adenocarcinoma).

In another embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to a method for treating and / or preventing cancer comprising administering to a subject in need thereof, preferably a human, a therapeutically effective amount of a compound of Formula (I), or a pharmaceutically acceptable salt, or a solvate thereof, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said

subject's cancer or for the treatment or prevention of side effects associated with the treatment of said subject's cancer.

The compounds according to the present invention or pharmaceutical compositions comprising said compounds, may also be combined with other agents that stimulate or enhance the immune response, such as vaccines.

In an embodiment, the present invention is directed to methods for treating and / or preventing a cancer (wherein the cancer is selected from those described herein) comprising administering to a subject in need thereof (preferably a human), a therapeutically effective amount of co-therapy or combination therapy; wherein the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for treating and / or preventing diseases (preferably cancers) mentioned above.

The present invention is directed to compounds of Formula (I) and pharmaceutically acceptable salts, and solvates thereof, for treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for treating and / or preventing, in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for treating and / or preventing of any one of the diseases mentioned hereinbefore.

In view of the utility of the compounds of Formula (I) and pharmaceutically acceptable salts, and solvates thereof, there is provided a method of treating subjects, preferably mammals such as humans, suffering from any of the diseases mentioned hereinbefore; or a method of slowing the progression of any of the diseases mentioned hereinbefore in subject, humans; or a method of preventing subjects, preferably mammals such as humans, from suffering from any one of the diseases mentioned hereinbefore.

Said methods comprise the administration, i.e. the systemic or topical administration, preferably oral or intravenous administration, more preferably oral administration, of an effective amount of a compound of Formula (I) or a pharmaceutically acceptable salt, or a solvate thereof, to subjects such as humans.

One skilled in the art will recognize that a therapeutically effective amount of the compounds of the present

invention is the amount sufficient to have therapeutic activity and that this amount varies *inter alias*, depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound, the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, in an embodiment, the present invention is directed to a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

EXAMPLES

Several methods for preparing the Compounds of this invention are illustrated in the following examples. Unless otherwise noted, all starting materials were obtained from commercial suppliers and used without further purification, or alternatively can be synthesized by a skilled person by using published methods.

Table 1: Abbreviations

| Abbreviation | Meaning |
|---|---|
| **AcOH** | acetic acid |
| **Boc** | tert-butyloxycarbonyl |
| **Bn** | benzyl |
| **Bz** | benzoyl |
| **Celite®** | diatomaceous earth |
| **DBU** | 1,8-diazabicyclo[5.4.0]undec-7-ene |
| **DCE** | 1,2-dichloroethane |

(continued)

| Abbreviation | Meaning |
|---|---|
| **DCM** | dichloromethane |
| **DECP** | diethyl cyanophosphonate |
| **DMAP** | 4-dimethylaminopyridine |
| **DMP** | Dess-Martin periodinane |
| **DMF** | *N,N*-dimethylformamide |
| **DMSO** | dimethyl sulfoxide |
| **MeI** | methyl iodide |
| **EDC.HCl** | N-(3-dimethylaminopropyl)-N'-ethylcarbodiimide hydrochloride |
| **eq. or equiv.** | equivalent(s) |
| **Et** | ethyl |
| **EtOAc** | ethyl acetate |
| **EtOH** | ethanol |
| **$Et_2O$** | diethyl ether |
| **$Et_3N$** | triethylamine |
| **g** | gram(s) |
| **h** | hour(s) |
| **HPLC** | high performance liquid chromatography |
| **$iPrNH_2$ or $i\text{-}PrNH_2$** | isopropylamine |
| **iPrOH or i-PrOH** | isopropylalcohol |
| **KHMDS** | potassium bis(trimethylsilyl)amide |
| **LDA** | lithium diisopropylamide |
| **M** | molar |
| **Me** | methyl |
| **MeOH** | methanol |
| **MeI** | methyl iodide |
| **MeCN** | acetonitrile |
| **mg** | milligram(s) |
| **min** | minute(s) |
| **$NaBH(OAc)_3$** | sodium triacetoxyborohydride |
| **NaOMe** | sodium methoxide |
| **$Pd_2dba_3$•$CHCl_3$** | dipalladium-tris(dibenzylideneacetone)chloroform complex |
| **PMB** | p-methoxybenzyl |
| **Prep** | preparative |
| **quant.** | quantitative yield |
| **RM** | reaction mixture |
| **RP** | reversed phase |
| **rt** | room temperature |
| **SFC** | supercritical fluid chromatography |
| **TBAF** | tetrabutylammonium fluoride |

(continued)

| Abbreviation | Meaning |
|---|---|
| **TBS** | tert-butyldimethylsilyl |
| **TBSCI** | tert-butyldimethylsilyl chloride |
| **TEMPO** | 2,2,6,6-tetramethylpiperidine-1-oxyl |
| **TFA** | trifluoroacetic acid |
| **THF** | tetrahydrofuran |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

## Intermediate 1

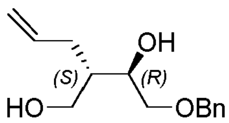

To a cooled (-25 °C) solution of CuCN (3.46 g, 38.6 mmol, 0.3 equiv.) and allylmagnesium bromide (1.0 M solution in diethyl ether, 386 mL, 386 mmol, 3.0 equiv.) in anhydrous THF (1.2 L) under a $N_2$ atmosphere was added a solution of ((2*R*,3*S*)-3-((benzyloxy)methyl)oxiran-2-yl)methanol (CAS: 84621-89-6, 25.0 g, 129 mmol, 1.0 equiv.) in anhydrous THF (80 mL) *via* syringe pump (addition rate: 25 mL/h). After the addition, the reaction mixture was stirred at -25 °C for 12 h. Next, it was quenched with saturated aqueous NaHCOs, warmed to rt, and the layers were separated. The aqueous layer was extracted with EtOAc (3x), and the combined organic layers were dried over anhydrous $MgSO_4$, filtered, and concentrated under reduced pressure. The crude residue was purified by reverse-phase HPLC (Stationary phase: RP XBridge Prep C18 OBD-10 μm, 50 × 150 mm; Mobile phase: 0.25 % $NH_4HCO_3$ solution in water, MeCN) to give **Intermediate 1** (12.1 g, yield: 40 %) as a colourless oil.

## Intermediate 2

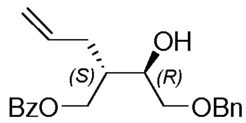

To a cooled (0 °C) solution of **Intermediate 1** (20.4 g, 86.3 mmol, 1.0 equiv.) in dry pyridine (154 mL) was added benzoyl chloride (10.5 mL, 90.6 mmol, 1.05 equiv.) dropwise *via* syringe. The reaction mixture was then stirred at this temperature for 2 h. Next, 1 N aqueous HCl was added to the reaction, and the mixture was extracted with DCM (3×). The combined organic layers were washed with 1 N aqueous HCl followed by brine. The organic layer was then dried over anhydrous $MgSO_4$, filtered, and evaporated under reduced pressure. The crude residue was purified by flash column chromatography (silica gel 300 g, 0-30 % EtOAc in heptane) to give **Intermediate 2** (19.2 g, yield: 65 %) as a colourless oil.

## Intermediate 3

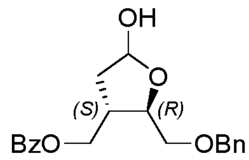

**Intermediate** 2 (14.2 g, 41.7 mmol, 1.0 equiv.) was dissolved in a mixture of dry MeOH (300 mL) and DCM (250 mL), and the solution was cooled down to -78 °C. Ozone was flowed through the reaction mixture until the solution turned blue. Nitrogen was then bubbled through the solution to remove excess ozone. Dimethyl sulfide (15.3 mL, 208 mmol, 5.0 equiv.) was added, and the reaction was allowed to reach rt and stirred for 4 h. Solvents were removed under reduced pressure to give the crude **Intermediate 3** which was used in the next step without further purification.

## Intermediate 4

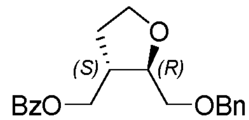

**Intermediate 3** (2.55 g, 7.45 mmol, 1.0 equiv.) was dissolved in a mixture of anhydrous DCM (30 mL) and MeCN (30 mL) under a $N_2$ atmosphere at rt. The solution was then cooled down to -20 °C and triethylsilane (CAS: 617-86-7, 1.75 mL, 11.0 mmol, 1.5 equiv.) was added, followed by dropwise addition of boron trifluoride diethyl etherate (CAS: 109-63-7, 2.82 mL, 11.0 mmol, 1.5 equiv.). After the addition, the reaction mixture was slowly warmed to 0 °C and stirred for 45 min. Saturated aqueous NaHCOs was added, and the mixture was extracted with DCM (3x). The organic layers were separated, dried over anhydrous $MgSO_4$, filtered, and the solvents were evaporated *in vacuo* to afford **Intermediate 4** (2.33 g, yield: 96 %) as a colourless oil.

## Intermediate 5

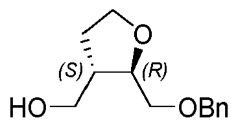

To a solution of **Intermediate 4** (3.66 g, 11.2 mmol, 1.0 equiv.) in dry MeOH (20 mL) was added NaOH (1.0 M solution in $H_2O$, 20 mL, 20.0 mmol, 1.8 equiv.) at rt, and the reaction mixture was stirred for 1 h. Upon completion, the reaction was neutralized by addition of 1 N aqueous HCl, and MeOH was removed under reduced pressure. The resultant aqueous solution was extracted with EtOAc (3x). The organic layers were combined, washed with brine, dried over anhydrous $MgSO_4$, filtered, and concentrated under reduced pressure. The crude residue was purified by flash column chromatography (silica gel 40 g, 0-100 % EtOAc in heptane) to give **Intermediate 5** (2.33 g, yield: 93 %) as colourless oil.

## Intermediate 6

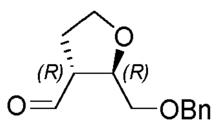

To a solution of **Intermediate 5** (1.20 g, 5.40 mmol, 1.0 equiv.) in anhydrous DCM (48 mL) was added Dess-Martin periodinane (CAS: 87413-09-0, 2.75 g, 6.48 mmol, 1.2 equiv.) at rt. The reaction mixture was then stirred for 2 h. Saturated aqueous NaHCOs was added to the reaction and it was stirred for a further 10 min. The reaction mixture was filtered through a short pad of Celite. The organic layer was separated, and the aqueous layer was extracted with DCM (2x). The combined organic layers were washed with brine, dried over anhydrous $MgSO_4$, filtered, and concentrated under reduced pressure. The crude residue was purified by flash column chromatography (silica gel 40 g, 0-50 % EtOAc in heptane) to give **Intermediate 6** (952 mg, yield: 87 %) as a colourless oil.

## Intermediate 7

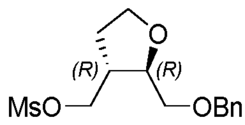

Dry pyridine (2.21 mL, 27.4 mmol, 3.0 equiv.) and DMAP (CAS: 1122-58-3, 230 mg, 1.88 mmol, 0.20 equiv.) were added to a cooled (0 °C) solution of **Intermediate 5** (2.04 g, 9.18 mmol, 1.0 equiv.) in anhydrous DCM (40 mL) under a $N_2$ atmosphere, and the reaction mixture was stirred at 0 °C for 15 min. Next, methanesufonyl chloride (CAS: 124-63-0, 1.06 mL, 13.7 mmol, 1.5 equiv.) was added dropwise at 0 °C, and the reaction mixture was stirred at 10 °C for 2 h, followed by 16 h at rt. Upon completion, the reaction was quenched with 1 N aqueous HCl and extracted with DCM (3x). The organic layers were combined, washed with 1 N aqueous HCl and then brine, dried over anhydrous $MgSO_4$, filtered, and concentrated under reduced pressure. The crude **Intermediate 7** (2.97 g, yield: 93 %) was used in the next step without further purification.

## Intermediate 8

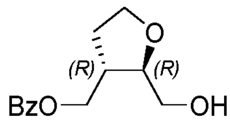

To a 500 mL Schlenk flask charged with Pd/C (10 % w/w, 1.96 g, 1.84 mmol, 0.03 equiv.) was added a solution of **Intermediate 4** (20 g, 61.3 mmol, 1.0 equiv.) in a mixture of dry EtOAc (125 mL) and MeOH (125 mL) under a $H_2$ atmosphere (1 bar). The reaction mixture was then stirred at rt for 4 h. Next, the reaction mixture was filtered through a pad of Celite and washed with MeOH. The filtrate was evaporated under reduced pressure to afford **Intermediate 8** (14.3 g, yield: 98 %) as a pale-yellow oil, which was used in the next step without further purification.

## Intermediate 9

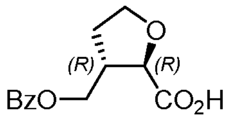

A solution of **Intermediate 8** (12.3 g, 52.1 mmol, 1.0 equiv.) and (diacetoxyiodo)benzene (CAS: 3240-34-4, 36.9 g, 115 mmol, 2.2 equiv.) in a mixture of MeCN (55 mL) and water (55 mL) was treated with TEMPO (CAS: 2564-83-2, 1.61 g, 10.4 mmol, 0.2 equiv.), and the reaction mixture was stirred at rt for 2.5 h. Ethanol (30 mL) and 1 N aqueous HCl (500 mL) were then added, and the mixture was extracted with EtOAc (3x). The combined organic layers were washed with brine, dried over anhydrous $MgSO_4$, filtered, and concentrated *in vacuo.* The crude residue was purified by flash column chromatography (silica gel 100 g, 0-40 % MeOH in DCM) to afford **Intermediate 9** (10.0 g, yield: 77 %) as a pale-yellow oil.

## Intermediate 10

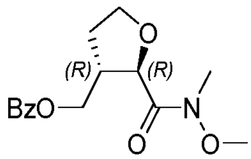

A solution of **Intermediate 9** (10.0 g, 39.9 mmol, 1.0 equiv.) and *N,O*-dimethylhydroxylamine hydrochloride (CAS: 6638-79-5, 4.29 g, 43.9 mmol, 1.1 equiv.) in anhydrous DCM (167 mL) was treated with N-methylmorpholine (CAS: 109-02-4, 4.85 mL, 43.9 mmol, 1.1 equiv.), and the reaction mixture was cooled down to 0 °C and treated with EDC.HCl (CAS: 25952-53-8, 8.42 g, 43.9 mmol, 1.1 equiv.). The resultant mixture was stirred at rt for 2.5 h. Upon completion, the mixture was treated with 1 N aqueous HCl and extracted with DCM (3x). The combined organic layers were washed with water and then brine, dried over anhydrous $MgSO_4$, filtered, and evaporated *in vacuo.* The crude **Intermediate 10** (9.99 g, yield: 85 %) was obtained as a pale-yellow oil and used in the next step without further purification.

## Intermediate 11

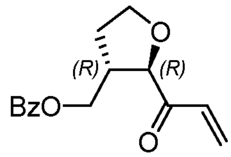

A cooled (-78 °C) solution of **Intermediate 10** (9.99 g, 34.2 mmol, 1.0 equiv.) in anhydrous THF (111 mL) was treated with vinylmagnesium bromide (CAS: 1826-67-1, 1.0 M solution in THF, 68.4 mL, 68.4 mmol, 2.0 equiv.) dropwise over 45 min. Stirring was continued at -78 °C for 1 h, and then the reaction mixture was poured into 1 N aqueous HCl (600 mL) at 0-5 °C. Next, part of the THF was carefully evaporated under reduced pressure, and the remaining mixture was extracted with $Et_2O$ (4x). The combined organic layers were washed with brine, dried over anhydrous $MgSO_4$, filtered, and concentrated under reduced pressure. The crude **Intermediate 11** (9.81 g, yield: 98 %) was obtained as a yellow oil, which was used in the next step without further purification.

## Intermediate 12

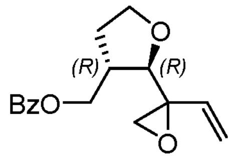

A cooled (0 °C) solution of trimethylsulfonium iodide (CAS: 1774-47-6, 9.23 g, 45.2 mmol, 1.2 equiv.) in anhydrous THF (162 mL) was slowly treated with KHMDS (1 M solution in THF, 44.5 mL, 44.5 mmol, 1.18 equiv.) over 25 min. The resultant mixture was stirred at 0 °C for another 30 min, and then a solution of **Intermediate 11** (9.81 g, 37.7 mmol, 1.0 equiv.) in anhydrous THF (60 mL) was added dropwise over 40 min. After stirring for an additional 40 min at 0 °C, the reaction mixture was poured into ice-cold water (100 mL), and most of the THF was evaporated *in vacuo.* The remaining mixture was diluted with $Et_2O$ and washed with water (2x). The aqueous layer was back-extracted with $Et_2O$. The combined organic layers were washed with brine, dried over anhydrous $MgSO_4$, filtered, and evaporated under reduced pressure. The crude **Intermediate 12** (5.37 g, yield: 52 %) was obtained as a colourless oil, which was used in the next step without further purification.

## Intermediate 13

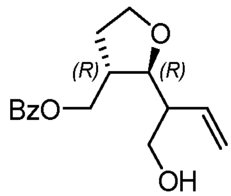

$Pd_2dba_3 \cdot CHCl_3$ (CAS: 52522-40-4, 507 mg, 0.49 mmol, 0.025 equiv.) was suspended in dry THF (20 mL) under a $N_2$ atmosphere. Next, tri(n-butyl)phosphine (186 μL, 0.744 mmol, 0.035 equiv.) was added followed by slow addition of a solution of formic acid (3.70 mL, 97.9 mmol, 5.0 equiv.) and $Et_3N$ (5.45 mL, 39.2 mmol, 2.0 equiv.) in dry THF (41 mL). After being stirred at rt for 5 min, the reaction mixture was treated by a solution of **Intermediate 12** (5.37 g, 19.6 mmol, 1.0 equiv.) in dry THF (51 mL) over a period of 30 min. The resultant mixture was then stirred at rt for 3 h. After being quenched with water, the layers were separated, and the aqueous layer was extracted with EtOAc (3x). The combined organic layers were washed with brine, dried over anhydrous $MgSO_4$, filtered, and evaporated *in vacuo.* The crude residue was purified by flash column chromatography (silica gel 80 g, 0-50 % EtOAc in heptane) to give **Intermediate 13** (851 mg, yield: 16 %) as a clear yellow oil.

## Intermediate 14

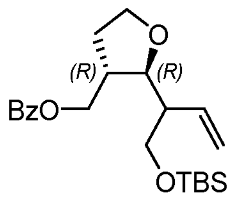

TBSCl (565 mg, 3.75 mmol, 1.3 equiv.) was added to a cooled (0 °C) solution of **Intermediate 13** (797 mg, 2.88 mmol, 1.0 equiv.) and imidazole (255 mg, 3.75 mmol, 1.3 equiv.) in dry DMF (15 mL). After stirring at 0 °C for 5 min, the reaction mixture was brought to rt and stirred for 3 h. The reaction mixture was then quenched with saturated aqueous NaHCOs and extracted with $Et_2O$. The combined organic layers were washed with brine, dried over anhydrous $MgSO_4$, filtered, and evaporated under reduced pressure. The crude residue was purified by flash column chromatography (silica gel 40 g, 0-15 % EtOAc in heptane) to give **Intermediate 14** (866 mg, yield: 77 %) as a colourless oil.

## Intermediate 15

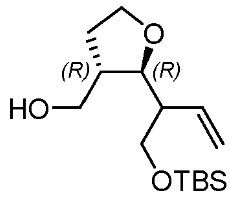

A solution of **Intermediate 14** (876 mg, 2.24 mmol, 1.0 equiv.) in dry MeOH (15 mL) was treated with NaOMe (25 % w/w in MeOH, 0.615 mL, 2.69 mmol, 1.2 equiv.), and the resultant mixture was stirred at rt for 1.5 h. Upon completion, the reaction mixture was diluted with saturated aqueous $NH_4Cl$ (150 mL) and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-50 % EtOAc in heptane) to afford **Intermediate 15** (668 mg, yield: 98 %) as a colourless oil.

## Intermediate 16

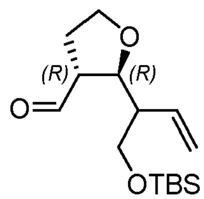

A solution of **Intermediate 15** (661 mg, 2.31 mmol, 1.0 equiv.) in dry DCM (18 mL) was treated with Dess-Martin periodinane (CAS: 87413-09-0, 1.22 g, 2.88 mmol, 1.25 equiv.), and the resultant mixture was stirred at rt for 1 h. The reaction was quenched by the addition of saturated aqueous $NaHCO_3$ (40 mL) and 10 % aqueous $Na_2S_2O_3$ (40 mL), and the mixture was extracted with DCM (3x). The combined organic layers were washed with saturated aqueous $NaHCO_3$ and then brine, dried over anhydrous $MgSO_4$, filtered, and concentrated *in vacuo.* The crude **Intermediate 16** (589 mg, yield: 90 %) was obtained as a colourless oil, which was used in the next step without further purification.

## Intermediate 16

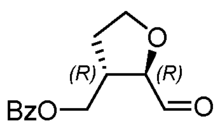

To a cooled (-78 °C) solution of DMSO (241 μL, 3.39 mmol, 4.0 equiv.) in anhydrous DCM (4.0 mL) was added oxalyl chloride (143 μL, 1.69 mmol, 2.0 equiv.) under a $N_2$ atmosphere. The reaction mixture was stirred at -78 °C for 15 min. Next, a solution of **Intermediate 8** (200 mg, 0.846 mmol, 1.0 equiv.) in anhydrous DCM (2.0 mL) was added dropwise at -78 °C. The reaction mixture was stirred for a further 1 h, and then $Et_3N$ (706 μL, 5.08 mmol, 6.0 equiv.) was added

dropwise at -78 °C. The reaction mixture was stirred at -78 °C for a further 1 h. Water (30 mL) was added at -20 °C to quench the reaction, and the mixture was brought to rt. The mixture was extracted with EtOAc. The organic layers were combined, washed with brine, dried over anhydrous $MgSO_4$, filtered, and concentrated under reduced pressure. The crude residue was purified by flash column chromatography (silica gel 24 g, 0-50 % EtOAc in heptane) to give **Intermediate 16** (2.87 mg, yield: 98 %) as a white gum.

## Intermediate 17

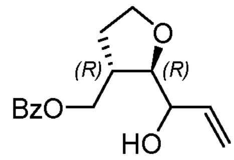

Vinylmagnesium bromide (1.0 M solution in diethyl ether, 49.3 mL, 49.3 mmol, 68 equiv.) was added dropwise to a stirred solution of $ZnCl_2$ (1.9 M solution in 2-methyltetrahydrofuran, 13.0 mL, 24.6 mmol, 34 equiv.) at rt under a $N_2$ atmosphere. The reaction mixture was then stirred at rt for 1 h. Next, the reaction mixture was cooled down to -20 °C, and a solution of **Intermediate 16** (339 mg, 0.721 mmol, 1.0 equiv.) in dry toluene (9.0 mL) was added dropwise while keeping the reaction temperature below -15 °C. The reaction mixture was stirred for a further 20 min and then quenched by the addition of saturated aqueous $NH_4Cl$. The mixture was extracted with EtOAc (2x), and the combined organic layers were washed with brine, dried over anhydrous $MgSO_4$, filtered, and concentrated *in vacuo.* The crude residue was purified by flash column chromatography (silica gel 24 g, 0-80 % EtOAc in heptane) to give **Intermediate 17** (1.07 g, yield: 27 %) as a colourless oil. Inseparable mixture of epimers at the hydroxy-substituted carbon.

## Intermediate 18

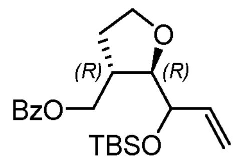

A cooled (0 °C) solution of **Intermediate 17** (953 mg, 2.94 mmol, 1.0 equiv.) in dry DMF (15 mL) was treated with imidazole (260 mg, 3.83 mmol, 1.3 equiv.), followed by portionwise addition of TBSCl (577 mg, 3.83 mmol, 1.3 equiv.) over 15 min. The resultant mixture was stirred at rt for 26 h. Upon completion, the reaction mixture was quenched with saturated aqueous NaHCOs (100 mL), diluted with water (50 mL), and extracted with $Et_2O$ (3x). The combined organic layers were washed with brine, dried over anhydrous $MgSO_4$, filtered, and evaporated under reduced pressure. The crude residue was purified by flash column chromatography (silica gel 24 g, 0-15 % EtOAc in heptane) to give **Intermediate 18** (800 mg, yield: 72 %) as a colourless oil.

## Intermediates 19 and 20

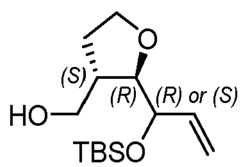

**19**

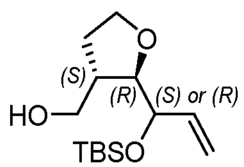

**20**

A solution of **Intermediate 18** (730 mg, 1.94 mmol, 1.0 equiv.) in dry MeOH (13 mL) was treated with NaOMe (25 % w/w in MeOH, 0.532 mL, 2.33 mmol, 1.2 equiv.), and the resultant mixture was stirred at rt for 1.5 h. The reaction was then quenched by dropwise addition of glacial AcOH (0.50 mL), and the mixture was diluted with saturated aqueous $NH_4Cl$ (150 mL) and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-40 % EtOAc in heptane) to afford diastereomerically pure **Intermediate 19** (219 mg, yield: 41 %) and **Intermediate 20** (268 mg, yield: 51 %) as colourless oils.

## Intermediate 21

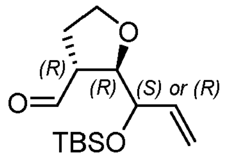

A solution of **Intermediate 20** (173 mg, 0.635 mmol, 1.0 equiv.) in dry DCM (5.0 mL) was treated with Dess-Martin periodinane (CAS: 87413-09-0, 404 mg, 0.952 mmol, 1.5 equiv.), and the resultant mixture was stirred at rt for 1.5 h. The reaction was quenched by the addition of saturated aqueous NaHCOs (40 mL) and 10 % aqueous $Na_2S_2O_3$ (40 mL), and the mixture was extracted with DCM (3x). The combined organic layers were washed with saturated aqueous $NaHCO_3$ and then brine, dried over anhydrous $MgSO_4$, filtered, and concentrated *in vacuo.* The crude **Intermediate 21** (185 mg, yield: 98 %) was obtained as a white solid, which was used in the next step without further purification.

## Intermediate 22

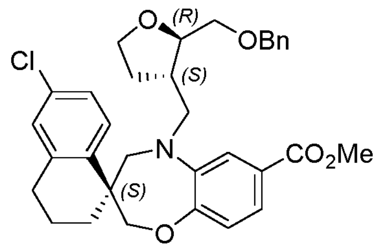

Methyl (*S*)-6'-chloro-3',4,4',5-tetrahydro-2H,2'H-spiro[benzo[b][1,4]oxazepine-3,1'-naphthalene]-7-carboxylate (10.5 g, 28.5 mmol, 1.6 equiv.), **Intermediate 6** (8.28 g, 17.3 mmol, 1.0 equiv.), and anhydrous molecular sieves 4 Å (10 g) were stirred in anhydrous DCM (285 mL) under a $N_2$ atmosphere at 5 °C. Glacial acetic acid (30 mL, 525 mmol, 30 equiv.) was then added at 5 °C, and the reaction mixture was stirred for 15 min. Next, $NaBH(OAc)_3$ (7.42 g, 35.1 mmol, 2.0 equiv.) was added to the reaction in portions over 2 h at 5 °C. After addition, the reaction mixture was stirred at this temperature for a further 14 h. Upon completion, molecular sieves were filtered off, and saturated aqueous $NaHCO_3$ was added. The reaction mixture was extracted with DCM (2x). The combined organic layers were washed with brine, dried over anhydrous $MgSO_4$, filtered, and concentrated under reduced pressure. The crude residue was purified by flash column chromatography (silica gel 330 g, 0-40 % EtOAc in heptane) to afford **Intermediate 22** (13.4 g, yield: 84 %) as a white foam.

## Intermediate 23

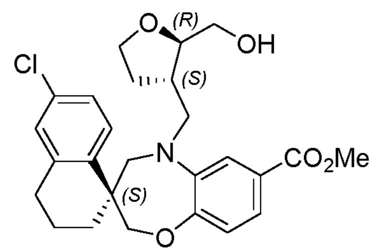

A suspension of **Intermediate 22** (13.3 g, 23.7 mmol, 1.0 equiv.), $Pd(OH)_2$ on carbon (CAS: 12135-22-7, 831 mg, 1.18 mmol, 0.05 equiv.) in dry EtOAc (300 mL) was charged into a 1.0 L Schlenk tube and stirred at rt under a $H_2$ atmosphere (1 bar) for 2 h. Additional $Pd(OH)_2$ on carbon (415 mg, 0.59 mmol, 0.025 equiv.) was added to the reaction mixture and it was stirred for an additional 1 h. The reaction mixture was then filtered through a short pad of Celite, and the filter cake was washed with EtOAc. The solvent was removed under reduced pressure to give **Intermediate 23** (10.4 g, yield: 93 %) as a white foam.

## Intermediate 24

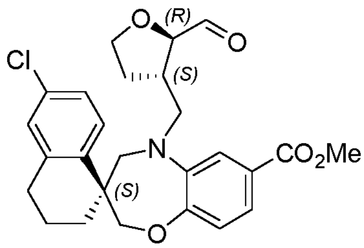

To a cooled (-78 °C) solution of DMSO (3.01 mL, 42.4 mmol, 4.0 equiv.) in anhydrous DCM (80 mL) under a $N_2$ atmosphere was added oxalyl chloride (CAS: 79-37-8, 1.79 mL, 21.2 mmol, 2.0 equiv.). After 30 min of stirring, a solution of **Intermediate 23** (5.0 g, 10.6 mmol, 1.0 equiv.) in anhydrous DCM (20 mL) was added dropwise at -78 °C. The reaction mixture was stirred for 1 h, and then $Et_3N$ (8.84 mL, 63.6 mmol, 6.0 equiv.) was added dropwise at -78 °C. The reaction mixture was stirred for 1 h at -78 °C, and then slowly warmed up to -20 °C during 1 h. Water was added in -20 °C to quench the reaction, and the mixture was brought back to rt. The reaction mixture was extracted with EtOAc (3x), and the combined organic layers were washed with brine, dried over anhydrous $MgSO_4$, filtered, and concentrated under reduced pressure. The crude residue was purified by flash column chromatography (silica gel 80 g, 0-70 % EtOAc in heptane) to give **Intermediate 24** (3.80 g, yield: 76 %) as a pale-yellow foam.

## Intermediate 25

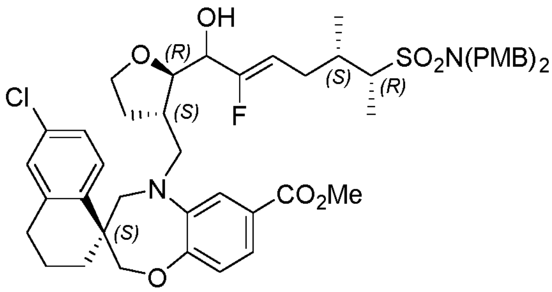

A mixture of anhydrous $CrCl_2$ (CAS: 10049-05-5, 1.27 g, 10.3 mmol, 3.0 equiv.) and $NiBr_2$ (CAS: 13462-88-9, 225 mg, 1.03 mmol, 0.3 equiv.) in dry DMF (10 mL) was degassed by bubbling $N_2$ through the reaction solution. To this was added a solution of **Intermediate 24** (1.62 g, 3.44 mmol, 1.0 equiv.) and (2*R*,3*S*,*E*)-6-bromo-6-fluoro-N,N-bis(4-methoxybenzyl)-3-methylhex-5-ene-2-sulfonamide (2.65 g, 5.16 mmol, 1.5 equiv.) in dry DMF (10 mL) dropwise *via* syringe pump (addition rate: 10 mL/h) at rt under a $N_2$ atmosphere. After the addition, the reaction mixture was stirred at rt for 12 h. Next, ethylenediamine (CAS: 107-15-3, 0.70 mL, 10.4 mmol, 3.0 equiv.) was added to the reaction and it was stirred for 10 min until the colour turned purple. Next, the reaction mixture was filtered through a short pad of Celite, the filter cake was washed with EtOAc, and the filtrate was concentrated under reduced pressure. The crude residue was purified by flash column chromatography (silica gel 24 g, 0-60 % EtOAc in heptane) to give **Intermediate 25** (1.46 g, yield: 47 %) as a white foam. Inseparable mixture of epimers at the methoxy-substituted carbon.

## Intermediate 26

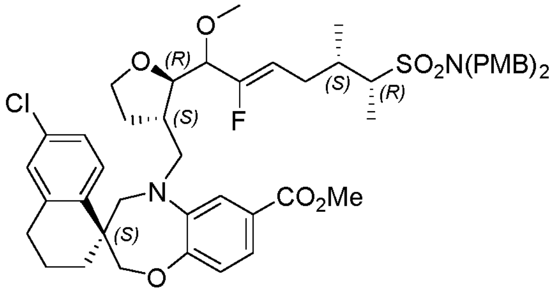

To a solution of **Intermediate 25** (354 mg, 0.297 mmol, 1.0 equiv.) and MeI (CAS: 74-88-4, 111 μL, 1.78 mmol, 6.0 equiv.) in anhydrous DMF (8 mL) was added NaH (60 % dispersion in mineral oil, 71 mg, 1.78 mmol, 6.0 equiv.) at rt under a

$N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-60 % EtOAc in heptane) to give **Intermediate 26** (201 mg, yield: 74 %) as a white foam. Inseparable mixture of epimers at the methoxy-substituted carbon.

## Intermediates 27 and 28

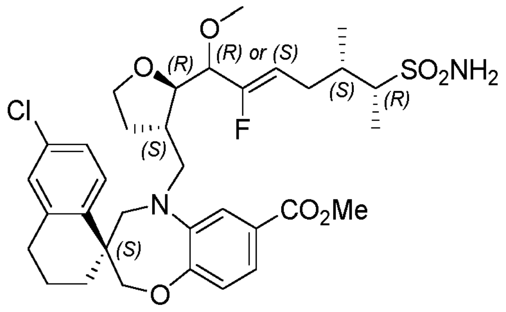

**27**

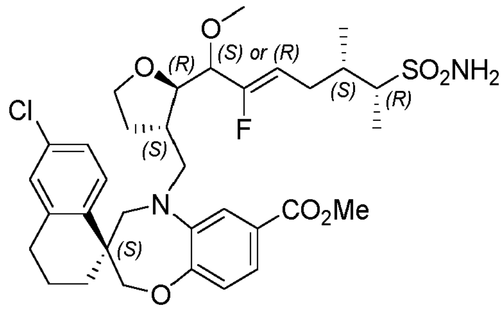

**28**

To a cooled (0 °C) solution of **Intermediate 26** (160 mg, 0.174 mmol, 1.0 equiv.) in anhydrous DCM (3.20 mL) was added TFA (CAS: 76-05-1, 1.60 mL, 20.9 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. The crude residue was purified by flash column chromatography (silica gel 12 g, 0-5 % MeOH in DCM) to give **Intermediate 27** (67 mg, yield: 57 %) as a white gum, and **Intermediate 28** (33 mg, yield: 28 %) as a white solid.

## Intermediate 29

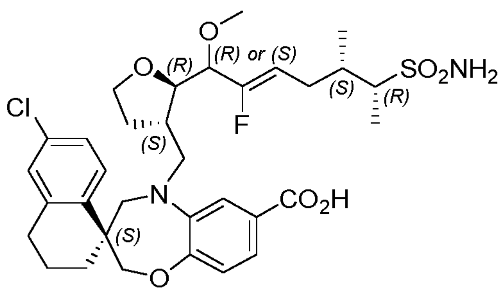

To a solution of **Intermediate** 27 (67 mg, 0.099 mmol, 1.0 equiv.) in a mixture of THF (1.70 mL) and distilled water (1.70 mL) was added LiOH (9.5 mg, 0.395 mmol, 4.0 equiv.) at rt. The reaction mixture was then heated at 40 °C and stirred for 16 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude residue was purified by flash column chromatography (silica gel 12 g, 0-5 % MeOH in DCM) to give **Intermediate 29** (53 mg, yield: 81 %) as a white foam.

## Intermediate 30

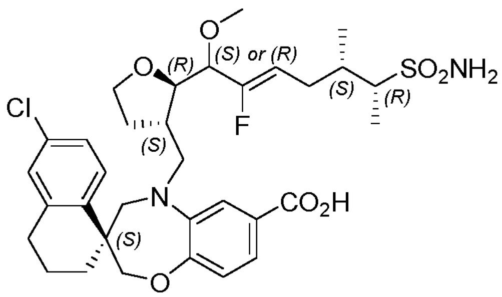

To a solution of **Intermediate 28** (33 mg, 0.049 mmol, 1.0 equiv.) in a mixture of THF (0.80 mL) and distilled water (0.80 mL) was added LiOH (4.7 mg, 0.194 mmol, 4.0 equiv.) at rt. The reaction mixture was then heated at 40 °C and stirred for 16 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude residue was purified by flash column chromatography (silica gel 12 g, 0-5 % MeOH in DCM) to give **Intermediate 30** (20 mg, yield: 75 %) as a white foam.

## Intermediates 31 and 32

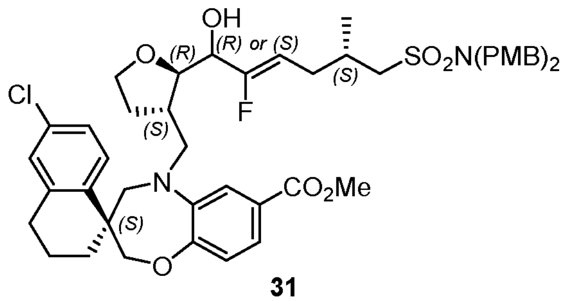

**31**

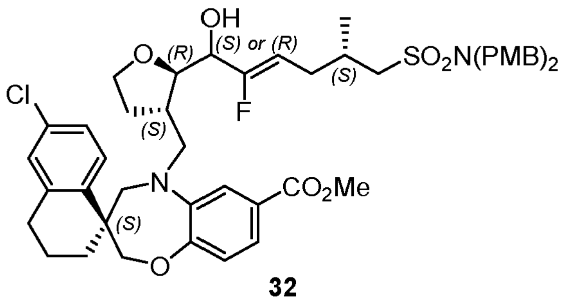

**32**

A mixture of anhydrous $CrCl_2$ (CAS: 10049-05-5, 1.27 g, 10.3 mmol, 3.0 equiv.) and $NiBr_2$ (CAS: 13462-88-9, 225 mg, 1.03 mmol, 0.3 equiv.) in dry DMF (10 mL) was degassed by bubbling $N_2$ through the reaction solution. To this was added a solution of **Intermediate 24** (1.62 g, 3.44 mmol, 1.0 equiv.) and (S,E)-5-bromo-5-fluoro-N,N-bis(4-methoxybenzyl)-2-methylpent-4-ene-1-sulfonamide (2.65 g, 5.16 mmol, 1.5 equiv.) in dry DMF (10 mL) dropwise *via* syringe pump (addition rate: 10 mL/h) at rt under a $N_2$ atmosphere. After the addition, the reaction mixture was stirred at rt for 12 h. Next, ethylenediamine (CAS: 107-15-3, 0.70 mL, 10.4 mmol, 3.0 equiv.) was added to the reaction and it was stirred for 10 min until the colour turned purple. Next, the reaction mixture was filtered through a short pad of Celite, the filter cake was washed with EtOAc, and the filtrate was concentrated under reduced pressure. The crude residue was purified by flash column chromatography (silica gel 80 g, 0-50 % EtOAc in heptane) to give diastereomerically pure **Intermediate 31** (1.22 g, yield: 52 %) and **Intermediate 32** (812 g, yield: 34 %) as white foams.

## Intermediate 33

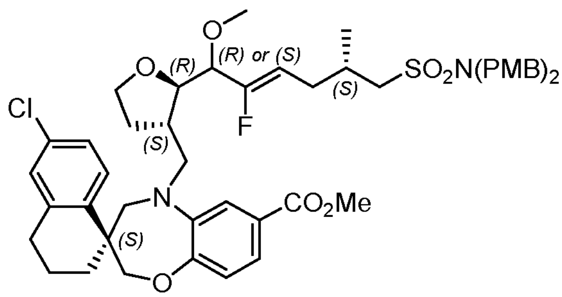

To a solution of **Intermediate 31** (500 mg, 0.561 mmol, 1.0 equiv.) and MeI (CAS: 74-88-4, 105 µL, 1.68 mmol, 3.0 equiv.) in anhydrous DMF (10 mL) was added NaH (60 % dispersion in mineral oil, 67.3 mg, 1.68 mmol, 3.0 equiv.) at rt under a $N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 40 g, 0-50 % EtOAc in heptane) to give **Intermediate 33** (511 mg, yield: 98 %) as a white foam.

**Intermediate 34**

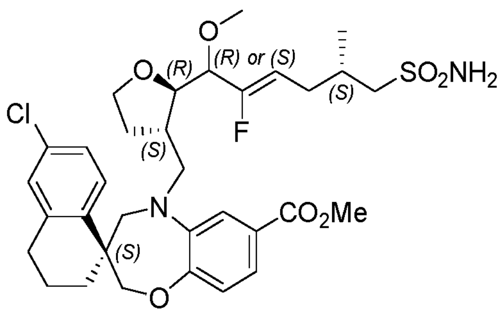

To a cooled (0 °C) solution of **Intermediate 33** (1.42 g, 1.57 mmol, 1.0 equiv.) in anhydrous DCM (125 mL) was added TFA (CAS: 76-05-1, 35 mL, 188 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. The crude residue was purified by flash column chromatography (silica gel 80 g, 0-5 % MeOH in DCM) to give **Intermediate 34** (1.26 g, yield: 98 %) as a pale-yellow foam.

**Intermediate 35**

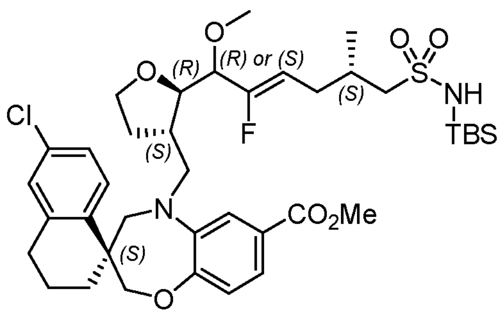

To a cooled (0 °C) solution of **Intermediate 34** (1.05 g, 1.58 mmol, 1.0 equiv.) in anhydrous THF (60 mL) was added NaH (60 % dispersion in mineral oil, 126 mg, 3.16 mmol, 2.0 equiv.) under a $N_2$ atmosphere. The resultant suspension was then stirred at 0 °C for 1 h. Next, a solution of TBSCl (357 mg, 2.37 mmol, 1.5 equiv.) in anhydrous THF (7.0 mL) was added dropwise *via* syringe. The reaction mixture was then stirred at rt for 16 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 80 g, 0-60 % EtOAc in heptane) to give **Intermediate 35** (1.05 g, yield: 85 %) as a white foam.

**Intermediate 36**

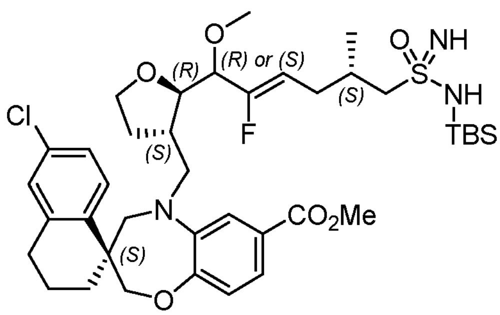

To a cooled (0 °C) solution of dichlorotriphenylphosphorane (CAS: 2526-64-9, 900 mg, 2.70 mmol, 2.0 equiv.) in anhydrous DCM (25 mL) was added $Et_3N$ (753 $\mu$L, 5.40 mmol, 4.0 equiv.) under a $N_2$ atmosphere. After stirring at 0 °C for 15 min, a solution of **Intermediate 35** (1.05 g, 1.35 mmol, 1.0 equiv.) in anhydrous DCM (5 mL) was added dropwise. After stirring at 0 °C for 1 h, gaseous ammonia was bubbled through the reaction mixture during 5 min, followed by stirring at 0 °C

for an additional 2 h. Upon completion, the reaction mixture was filtered through a pad of Celite (the filter cake was rinsed with DCM), and the filtrate was evaporated under reduced pressure. The crude residue was purified by flash column chromatography (silica gel 40 g, 0-100 % EtOAc in heptane) to give **Intermediate 36** (673 mg, yield: 64 %) as a colourless oil. Inseparable mixture of epimers at the chiral sulfur atom.

## Intermediate 37

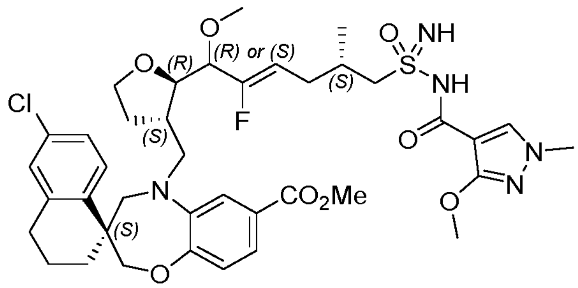

First, the acid chloride was formed separately from 3-methoxy-1-methyl-1H-pyrazole-4-carboxylic acid. To this end, a solution of 3-methoxy-1-methyl-1H-pyrazole-4-carboxylic acid (CAS: 113100-56-4, 60 mg, 0.387 mmol, 1.4 equiv.) in thionyl chloride (2.5 mL) was stirred and heated at 60 °C for 8 h. The reaction mixture was then cooled to ambient temperature, and volatiles were removed under reduced pressure. Next, the freshly prepared acid chloride was dissolved in anhydrous MeCN (5.0 mL) and treated with pyridazine (50 μL, 0.69 mmol, 2.5 equiv.). A solution of **Intermediate 36** (215 mg, 0.276 mmol, 1.0 equiv.) in anhydrous MeCN (4.0 mL) was then added at rt. The resultant reaction mixture was stirred at rt for 24 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude residue was purified by flash column chromatography (silica gel 24 g, 0-5 % MeOH in DCM) to give **Intermediate 37** (168 mg, yield: 75 %) as a white foam.

## Intermediate 38

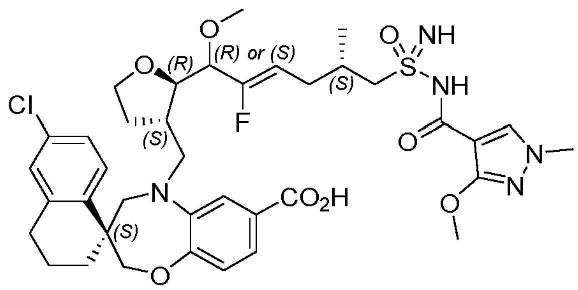

To a solution of **Intermediate 37** (168 mg, 0.209 mmol, 1.0 equiv.) in a mixture of THF (4.5 mL), MeOH (1.0 mL), and distilled water (1.0 mL) was added LiOH (50 mg, 2.09 mmol, 10 equiv.) at rt. The reaction mixture was then stirred at rt for 48 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The obtained crude **Intermediate 38** (161 mg, yield: 97 %) was used in the next step as such, without further purification.

## Intermediate 39

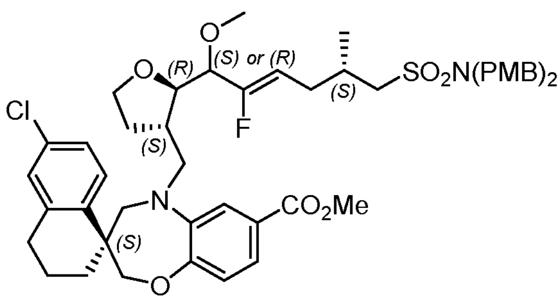

To a solution of **Intermediate 32** (500 mg, 0.561 mmol, 1.0 equiv.) and MeI (CAS: 74-88-4, 105 μL, 1.68 mmol, 3.0 equiv.) in anhydrous DMF (10 mL) was added NaH (60 % dispersion in mineral oil, 67.3 mg, 1.68 mmol, 3.0 equiv.) at rt under a $N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 40 g, 0-50 % EtOAc in heptane) to give **Intermediate 39** (511 mg, yield: 98 %) as a white foam.

## Intermediate 40

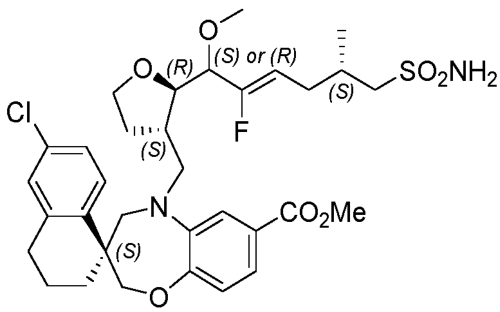

To a cooled (0 °C) solution of **Intermediate 39** (1.42 g, 1.57 mmol, 1.0 equiv.) in anhydrous DCM (125 mL) was added TFA (CAS: 76-05-1, 35 mL, 188 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. The crude residue was purified by flash column chromatography (silica gel 12 g, 0-5 % MeOH in DCM) to give **Intermediate 40** (1.26 g, yield: 98 %) as a pale-yellow foam.

## Intermediate 41

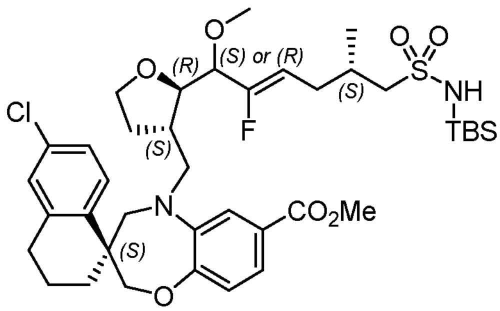

To a cooled (0 °C) solution of **Intermediate 40** (1.05 g, 1.58 mmol, 1.0 equiv.) in anhydrous THF (60 mL) was added NaH (60 % dispersion in mineral oil, 126 mg, 3.16 mmol, 2.0 equiv.) under a $N_2$ atmosphere. The resultant suspension was then stirred at 0 °C for 1 h. Next, a solution of TBSCl (357 mg, 2.37 mmol, 1.5 equiv.) in anhydrous THF (7.0 mL) was added dropwise *via* syringe. The reaction mixture was then stirred at rt for 16 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 80 g, 0-60 % EtOAc in heptane) to give **Intermediate 41** (1.05 g, yield: 85 %) as a white foam.

## Intermediate 42

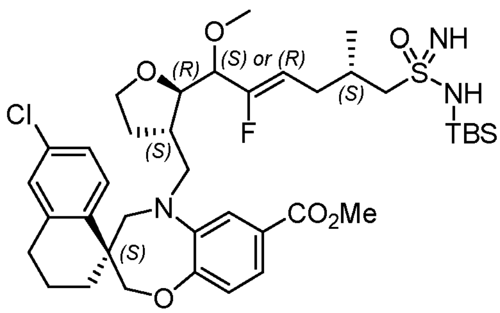

To a cooled (0 °C) solution of dichlorotriphenylphosphorane (CAS: 2526-64-9, 900 mg, 2.70 mmol, 2.0 equiv.) in anhydrous DCM (25 mL) was added $Et_3N$ (753 μL, 5.40 mmol, 4.0 equiv.) under a $N_2$ atmosphere. After stirring at 0 °C for 15 min, a solution of **Intermediate 41** (1.05 g, 1.35 mmol, 1.0 equiv.) in anhydrous DCM (5 mL) was added dropwise. After stirring at 0 °C for 1 h, gaseous ammonia was bubbled through the reaction mixture during 5 min, followed by stirring at 0 °C for an additional 2 h. Upon completion, the reaction mixture was filtered through a pad of Celite (the filter cake was rinsed with DCM), and the filtrate was evaporated under reduced pressure. The crude residue was purified by flash column chromatography (silica gel 40 g, 0-100 % EtOAc in heptane) to give **Intermediate 42** (673 mg, yield: 64 %) as a colourless oil. Inseparable mixture of epimers at the chiral sulfur atom.

## Intermediate 43

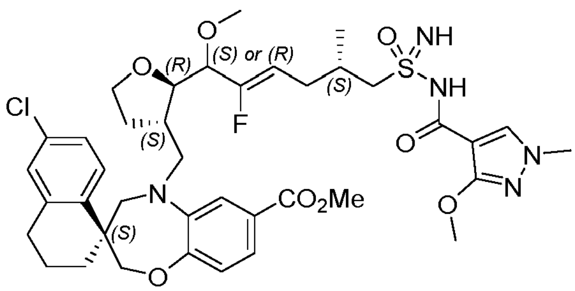

First, the acid chloride was formed separately from 3-methoxy-1-methyl-1H-pyrazole-4-carboxylic acid. To this end, a solution of 3-methoxy-1-methyl-1H-pyrazole-4-carboxylic acid (CAS: 113100-56-4, 53 mg, 0.342 mmol, 1.4 equiv.) in thionyl chloride (2.5 mL) was stirred and heated at 60 °C for 8 h. The reaction mixture was then cooled to ambient temperature, and volatiles were removed under reduced pressure. Next, the freshly prepared acid chloride was dissolved in anhydrous MeCN (4.5 mL) and treated with pyridazine (50 μL, 0.69 mmol, 2.5 equiv.). A solution of **Intermediate 42** (190 mg, 0.244 mmol, 1.0 equiv.) in anhydrous MeCN (3.5 mL) was then added at rt. The resultant reaction mixture was stirred at rt for 24 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude residue was purified by flash column chromatography (silica gel 24 g, 0-5 % MeOH in DCM) to give **Intermediate 43** (160 mg, yield: 81 %) as a white foam.

## Intermediate 44

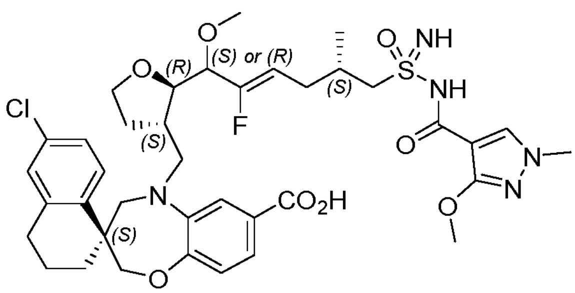

To a solution of **Intermediate 43** (160 mg, 0.199 mmol, 1.0 equiv.) in a mixture of THF (4.5 mL), MeOH (1.0 mL), and distilled water (1.0 mL) was added LiOH (48 mg, 1.99 mmol, 10 equiv.) at rt. The reaction mixture was then stirred at rt

for 48 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The obtained crude **Intermediate 44** (155 mg, yield: 98 %) was used in the next step as such, without further purification.

## Intermediate 45

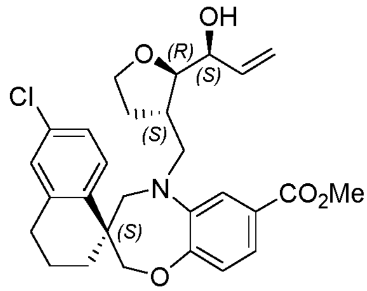

Vinylmagnesium bromide (1.0 M solution in diethyl ether, 4.33 mL, 4.33 mmol, 28 equiv.) was added dropwise to a stirred solution of $ZnCl_2$ (1.9 M solution in 2-methyltetrahydrofuran, 1.14 mL, 2.16 mmol, 14 equiv.) at rt under a $N_2$ atmosphere. The reaction mixture was then stirred at rt for 1 h. Next, the reaction mixture was cooled down to -20 °C, and **Intermediate 24** (72 mg, 0.153 mmol, 1.0 equiv.) in dry toluene (9 mL) was added dropwise while keeping the reaction temperature below -15 °C. The reaction mixture was stirred for a further 20 min and then quenched by the addition of saturated aqueous $NH_4Cl$. The mixture was extracted with EtOAc (2x), and the combined organic layers were washed with brine, dried over anhydrous $MgSO_4$, filtered, and concentrated *in vacuo.* The crude residue was purified by flash column chromatography (silica gel 12 g, 0-80 % EtOAc in heptane) to give **Intermediate 45** (238 mg, yield: 35 %) as a white gum. A single diastereomer was obtained.

## Intermediate 46

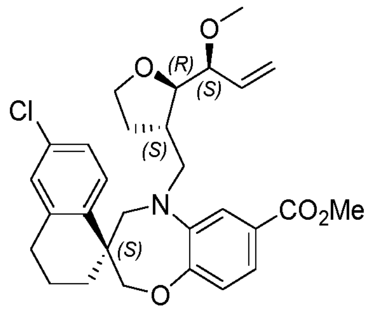

To a solution of **Intermediate 45** (250 mg, 0.371 mmol, 1.0 equiv.) and MeI (CAS: 74-88-4, 139 μL, 2.23 mmol, 6.0 equiv.) in anhydrous DMF (5.0 mL) was added NaH (60 % dispersion in mineral oil, 89.1 mg, 2.23 mmol, 6.0 equiv.), and the reaction mixture was stirred at rt for 1 h. Upon completion, the reaction mixture was diluted with water and extracted with EtOAc (3x). The organic layers were combined, washed with brine, dried over anhydrous $MgSO_4$, filtered, and concentrated under reduced pressure. The crude **Intermediate 46** was used in the next step without further purification.

## Intermediate 47

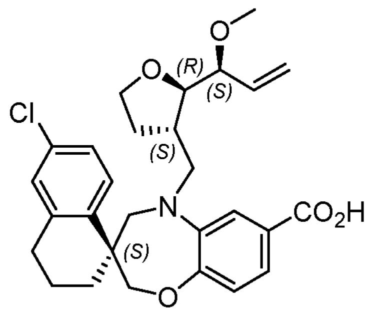

To a solution of **Intermediate 46** (190 mg, 0.371 mmol, 1.0 equiv.) in a mixture of THF (3.0 mL) and distilled water (3.0 mL) was added LiOH (44.4 mg, 1.86 mmol, 5.0 equiv.), and reaction mixture was stirred at 60 °C for 5 h. Upon completion, the reaction was neutralized with 1 N aqueous HCl and extracted with EtOAc (3x). The organic layers were combined, washed with brine, dried over anhydrous $MgSO_4$, filtered, and concentrated under reduced pressure. The crude residue was purified by reverse-phase HPLC (Stationary phase: RP XBridge Prep C18 OBD-10 μm, 50 x 150 mm; Mobile phase: 0.25 % $NH_4HCO_3$ solution in water, MeCN) to give **Intermediate 47** (45 mg, yield: 24 %) as a colourless oil.

## Intermediate 48

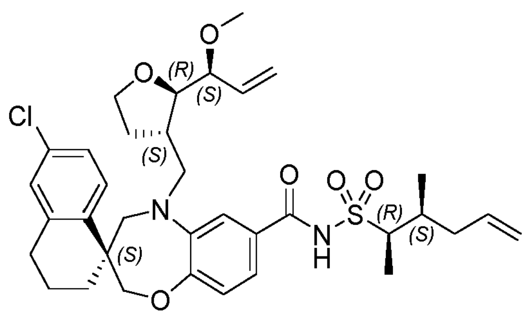

To a solution of **Intermediate 47** (45.0 mg, 0.090 mmol, 1.0 equiv.), (2*R*,3*S*)-3-methylhex-5-ene-2-sulfonamide (41.6 mg, 0.235 mmol, 2.6 equiv.), DMAP (CAS: 1122-58-3, 17.4 mg, 0.142 mmol, 1.6 equiv.). and $Et_3N$ (52.1 μL, 0.375 mmol, 4.2 equiv.) in anhydrous DCM (2.5 mL) was added EDC.HCl (CAS: 25952-53-8, 35.8 mg, 0.186 mmol, 2.1 equiv.), and the reaction mixture was stirred under a $N_2$ atmosphere at rt for 16 h. The reaction was quenched with 1 N aqueous HCl and extracted with DCM (3x). The combined organic layers were washed with brine, dried over anhydrous $MgSO_4$, filtered, and concentrated *in vacuo.* The crude product was purified by flash column chromatography (silica gel 12 g, 0-2 % MeOH in DCM) to afford **Intermediate 48** (32 mg, yield: 54 %) as a white solid.

## Intermediate 49

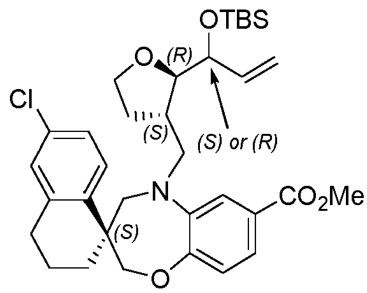

Methyl (*S*)-6'-chloro-3',4,4',5-tetrahydro-2H,2'H-spiro[benzo[b][1,4]oxazepine-3,1'-naphthalene]-7-carboxylate (213 mg, 0.577 mmol, 1.0 equiv.), **Intermediate 21** (172 mg, 0.635 mmol, 1.1 equiv.), and anhydrous 4 Å molecular sieves (250 mg) were stirred in anhydrous DCE (5.0 mL) under a $N_2$ atmosphere at 5 °C. Glacial acetic acid (165 μL, 2.89 mmol, 5.0 equiv.) was then added at 5 °C, and the reaction mixture was stirred for 15 min. Next, $NaBH(OAc)_3$ (CAS: 56553-60-7, 171 mg, 0.808 mmol, 1.4 equiv.) was added to the reaction in portions over 2 h at 5 °C. After addition, the reaction mixture was stirred at this temperature for a further 78 h. Upon completion, molecular sieves were filtered off, and saturated aqueous $NaHCO_3$ was added. The reaction mixture was extracted with DCM (2x). The combined organic layers were washed with brine, dried over anhydrous $MgSO_4$, filtered, and concentrated under reduced pressure. The crude residue was purified by flash column chromatography (silica gel 12 g, 20-80 % DCM in heptane) to afford **Intermediate 49** (128 mg, yield: 36 %) as a colourless waxy solid.

## Intermediate 50

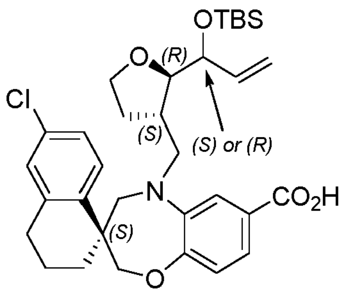

To a solution of **Intermediate 49** (109 mg, 0.178 mmol, 1.0 equiv.) in a mixture of THF (0.7 mL), MeOH (0.7 mL), and distilled water (0.2 mL) was added LiOH (17.1 mg, 0.712 mmol, 4.0 equiv.) at rt. The reaction mixture was then heated at 40 °C and stirred for 15 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude residue was purified by flash column chromatography (silica gel 4 g, 0-20 % EtOAc in DCM) to give **Intermediate 50** (67 mg, yield: 63 %) as a colourless waxy solid.

## Intermediate 51

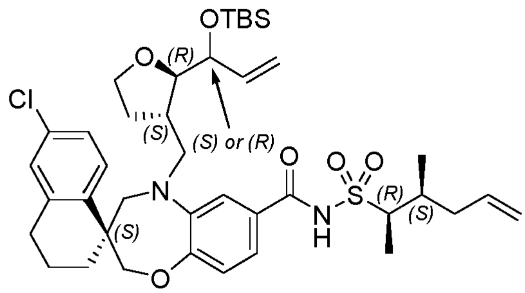

To a solution of **Intermediate 50** (67 mg, 0.112 mmol, 1.0 equiv.), (2*R*,3*S*)-3-methylhex-5-ene-2-sulfonamide (39.7 mg, 0.224 mmol, 2.0 equiv.), DMAP (CAS: 1122-58-3, 20.5 mg, 0.168 mmol, 1.5 equiv.). and $Et_3N$ (62.3 µL, 0.448 mmol, 4.0 equiv.) in anhydrous DCM (1.5 mL) was added EDC.HCl (CAS: 25952-53-8, 42.9 mg, 0.224 mmol, 2.0 equiv.), and the reaction mixture was stirred under a $N_2$ atmosphere at rt for 16 h. The reaction was quenched with 1 N aqueous HCl and extracted with DCM (3x). The combined organic layers were washed with brine, dried over anhydrous $MgSO_4$, filtered, and concentrated *in vacuo.* The crude product was purified by flash column chromatography (silica gel 4 g, 0-30 % EtOAc in heptane) to afford **Intermediate 51** (88 mg, yield: 98 %) as a colourless film.

## Intermediate 52

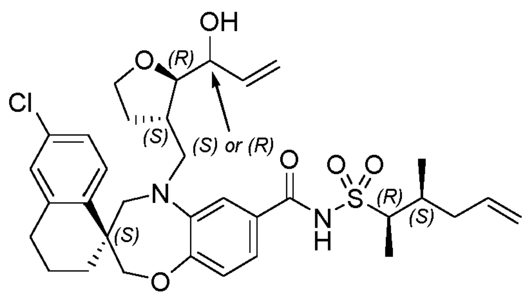

A solution of **Intermediate 51** (88 mg, 0.116 mmol, 1.0 equiv.) in anhydrous THF (1.0 mL) was treated dropwise with TBAF (CAS: 429-41-4, 1.0 M solution in THF, 0.174 mL, 0.174 mmol, 1.5 equiv.), and the resultant mixture was stirred at rt for 24 h. The reaction mixture was then poured into saturated aqueous $NH_4Cl$ (50 mL) and extracted with EtOAc (3x). The combined organic layers were washed with water and then brine, dried over anhydrous $MgSO_4$, filtered, and evaporated under reduced pressure. The crude **Intermediate 52** (76 mg, yield: 98 %) was obtained as a thick colourless oil which was used in the next step without further purification.

## Intermediate 53

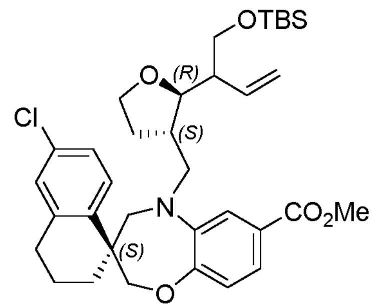

A solution of methyl (*S*)-6'-chloro-3',4,4',5-tetrahydro-2H,2'H-spiro[benzo[b][1,4]oxazepine-3,1'-naphthalene]-7-carboxylate (500 mg, 1.40 mmol, 1.0 equiv.), **Intermediate 16** (587 mg, 2.06 mmol, 1.45 equiv.), and 4 Å molecular sieves (300 mg) in anhydrous DCM (15 mL) was cooled down to 0 °C (ice bath) and treated with AcOH (0.788 mL, 13.8 mmol, 10 equiv.). The reaction mixture was stirred at 0 °C for 15 min. Sodium triacetoxyborohydride (CAS: 56553-60-7, 380 mg, 1.79 mmol, 1.3 equiv.) was then added as a solid in *ca.* 16 portions over a period of 3.5 h. Stirring was continued at 0 °C for a further 16 h, during which the ice bath was removed to reach a final temperature of 20 °C. The 4 Å molecular sieves were removed by filtration (the filter cake was rinsed with DCM), and the filtrate was washed with saturated aqueous NaHCOs. The layers were separated, and the aqueous layer was extracted with DCM (2x). The combined organic layers were washed with brine, dried over anhydrous $MgSO_4$, filtered, and evaporated *in vacuo.* The crude residue was purified by flash column chromatography (silica gel 40 g, 0-60 % DCM in heptane) to afford **Intermediate 53** (1.02 g, yield: 98%) as a colourless oil.

## Intermediate 54

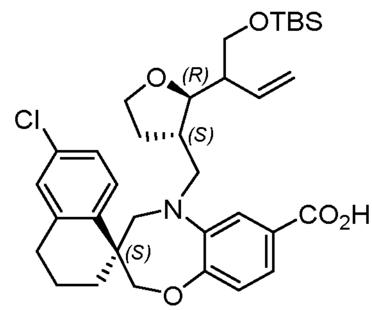

To a solution of **Intermediate 53** (945 mg, 1.51 mmol, 1.0 equiv.) in a mixture of THF (7.0 mL), distilled water (7.0 mL), and MeOH (1.0 mL) was added LiOH (181 mg, 7.54 mmol, 5.0 equiv.) at rt. The reaction mixture was then heated at 50 °C and stirred for 16 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude residue was purified by flash column chromatography (silica gel 12 g, 0-5 % MeOH in DCM) to give **Intermediate 54** (900 mg, yield: 97 %) as a white foam.

## Intermediate 55

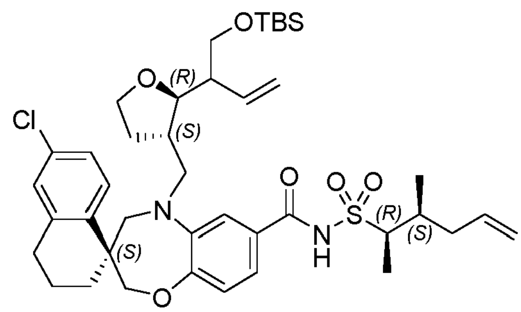

To a solution of **Intermediate 54** (718 mg, 1.17 mmol, 1.0 equiv.), (2*R*,3*S*)-3-methylhex-5-ene-2-sulfonamide

(416 mg, 2.35 mmol, 2.0 equiv.), DMAP (CAS: 1122-58-3, 215 mg, 1.76 mmol, 1.5 equiv.). and $Et_3N$ (652 µL, 4.69 mmol, 4.0 equiv.) in anhydrous DCM (12 mL) was added EDC.HCl (CAS: 25952-53-8, 450 mg, 2.35 mmol, 2.0 equiv.), and the reaction mixture was stirred under a $N_2$ atmosphere at rt for 16 h. The reaction was quenched with 1 N aqueous HCl and extracted with DCM (3x). The combined organic layers were washed with brine, dried over anhydrous $MgSO_4$, filtered, and concentrated *in vacuo.* The crude product was purified by flash column chromatography (silica gel 24 g, 0-40 % EtOAc in heptane) to afford **Intermediate 55** (672 mg, yield: 74 %) as a colourless film.

## Intermediate 56

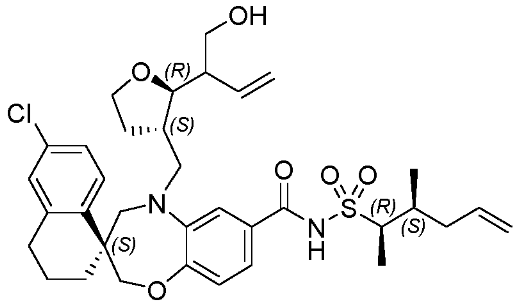

A solution of **Intermediate 55** (649 mg, 0.841 mmol, 1.0 equiv.) in anhydrous THF (8.0 mL) was treated dropwise with TBAF (CAS: 429-41-4, 1.0 M solution in THF, 2.52 mL, 2.52 mmol, 3.0 equiv.), and the resultant mixture was stirred at rt for 48 h. The reaction mixture was then poured into saturated aqueous $NH_4Cl$ (200 mL) and extracted with EtOAc (3x). The combined organic layers were washed with water and then brine, dried over anhydrous $MgSO_4$, filtered, and evaporated under reduced pressure. The crude **Intermediate 56** (706 mg, yield: 98 %) was obtained as a thick colourless oil which was used in the next step without further purification.

## Intermediate 57

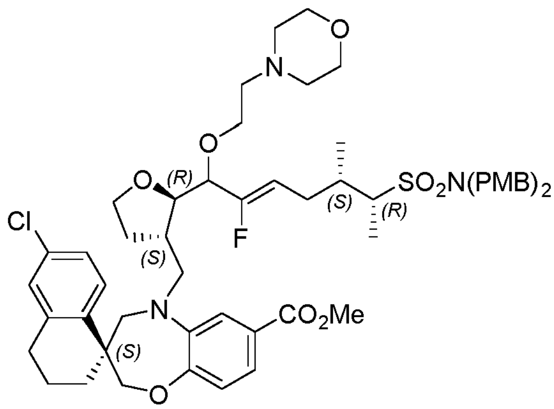

To a solution of **Intermediate 25** (137 mg, 0.151 mmol, 1.0 equiv.) and 4-(2-bromoethyl)morpholine (CAS: 89583-07-3, 88.1 mg, 0.454 mmol, 3.0 equiv.) in dry DMF (3.0 mL) was added NaH (60 % dispersion in mineral oil, 18.2 mg, 0.454 mmol, 3.0 equiv.), and the reaction mixture was stirred at rt for 15 min. Next, the reaction mixture was heated at 50 °C for 1 h, and then cooled down to rt, quenched with saturated aqueous $NH_4Cl$, and extracted with EtOAc. The organic layers were combined, washed with brine, dried over anhydrous $MgSO_4$, filtered, and concentrated under reduced pressure. The crude **Intermediate 57** (150 mg, yield: 97 %) was obtained as a pale-yellow gum and used in the next step without further purification.

## Intermediate 58

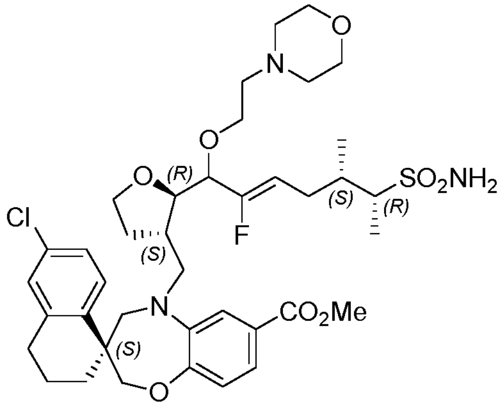

To a cooled (0 °C) solution of **Intermediate 57** (180 mg, 0.177 mmol, 1.0 equiv.) in anhydrous DCM (3.50 mL) was added TFA (CAS: 76-05-1, 1.60 mL, 20.9 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. The crude residue was purified by flash column chromatography (silica gel 12 g, 0-5 % MeOH in DCM) to give **Intermediate 58** (75 mg, yield: 54 %) as a white foam.

## Intermediate 59

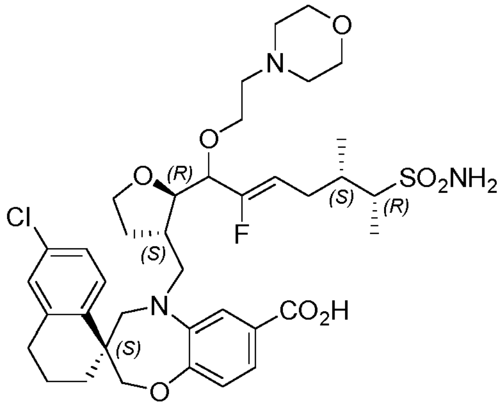

To a solution of **Intermediate 58** (75 mg, 0.096 mmol, 1.0 equiv.) in a mixture of THF (1.50 mL) and distilled water (1.50 mL) was added LiOH (9.23 mg, 0.385 mmol, 4.0 equiv.) at rt. The reaction mixture was then heated at 40 °C and stirred for 16 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude residue was purified by flash column chromatography (silica gel 12 g, 0-5 % MeOH in DCM) to give **Intermediate 59** (59 mg, yield: 80 %) as a white solid.

## Intermediate 60

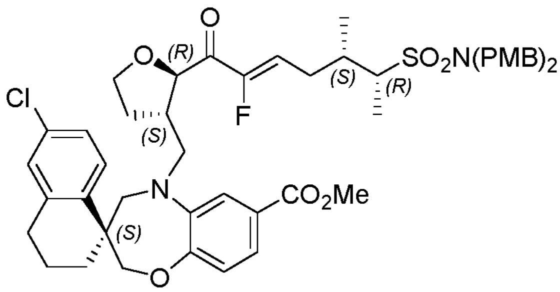

To a solution of **Intermediate 25** (730 mg, 0.806 mmol, 1.0 equiv.) in anhydrous DCM (20 mL) was added Dess-Martin periodinane (CAS: 87413-09-0, 1.37 g, 3.23 mmol, 4.0 equiv.) at rt. The reaction mixture was then stirred for 2 h. Saturated aqueous NaHCOs was added to the reaction and it was stirred for a further 10 min. The reaction mixture was filtered through a short pad of Celite. The organic layer was separated, and the aqueous layer was extracted with DCM (2x). The combined organic layers were washed with brine, dried over anhydrous $MgSO_4$, filtered, and concentrated under reduced pressure. The crude residue was purified by flash column chromatography (silica gel 24 g, 0-50 % EtOAc in heptane) to give **Intermediate 60** (585 mg, yield: 80 %) as a white foam.

## Intermediate 61

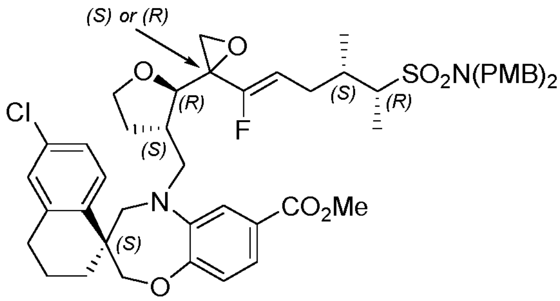

To a solution of **Intermediate 60** (690 mg, 0.764 mmol, 1.0 equiv.) and trimethylsulfoxonium iodide (CAS: 1774-47-6, 176 mg, 0.802 mmol, 1.05 equiv.) in dry DMSO (8.0 mL) was added potassium tert-butoxide (1.0 M solution in THF, 1.15 mL, 1.15 mmol, 1.5 equiv.), and the reaction mixture was stirred at rt under a $N_2$ atmosphere for 1 h. Upon completion, the reaction mixture was quenched with water and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-50 % EtOAc in heptane) to give **Intermediate 61** (390 mg, yield: 56 %) as a white foam. A single diastereomer was obtained.

## Intermediate 62

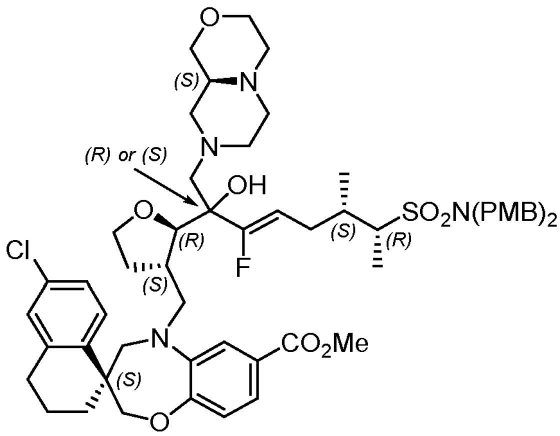

To a solution of **Intermediate 61** (340 mg, 0.371 mmol, 1.0 equiv.) and (*S*)-octahydropyrazino[2,1-c][1,4]oxazine dihydrochloride (CAS: 1089280-14-7, 239 mg, 1.11 mmol, 3.0 equiv.) in dry MeOH (5.0 mL) was added $Et_3N$ (515 μL, 3.71 mmol, 10 equiv.) at rt, and the reaction mixture was heated at 70 °C for 16 h. Next, the reaction mixture was cooled down to rt, and water was added. The reaction mixture was extracted with EtOAc (3x). The organic layers were combined, washed with brine, dried over anhydrous $MgSO_4$, filtered, and concentrated under reduced pressure. The crude residue was purified by flash column chromatography (silica gel 24 g, 0-80 % EtOAc in heptane) to afford **Intermediate 62** (270 mg, yield: 69 %) as a white gum.

## Intermediate 63

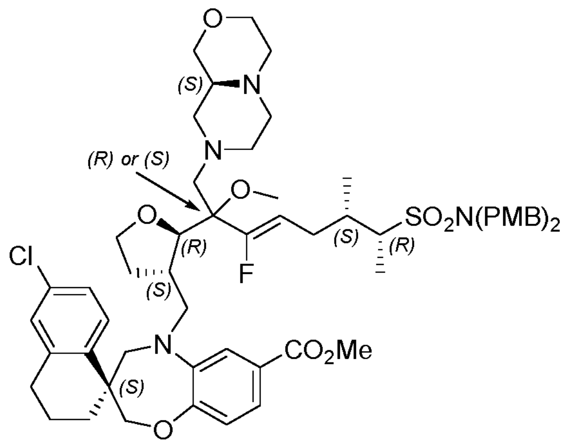

To a solution of **Intermediate 62** (270 mg, 0.255 mmol, 1.0 equiv.) and MeI (CAS: 74-88-4, 47.6 μL, 0.76 mmol, 3.0 equiv.) in anhydrous DMF (5.0 mL) was added NaH (60 % dispersion in mineral oil, 30.6 mg, 0.76 mmol, 3.0 equiv.) at rt under a $N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 12 g, 0-80 % EtOAc in heptane) to give **Intermediate 63** (309 mg, yield: 90 %) as a pale-yellow gum.

## Intermediate 64

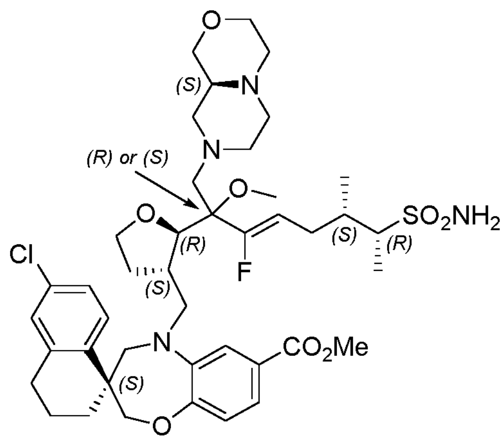

To a cooled (0 °C) solution of **Intermediate 63** (309 mg, 0.23 mmol, 1.0 equiv.) in anhydrous DCM (5.0 mL) was added TFA (CAS: 76-05-1, 2.11 mL, 27.6 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. The crude residue was purified by flash column chromatography (silica gel 24 g, 0-10 % MeOH in DCM) to give **Intermediate 64** (125 mg, yield: 65 %) as a white solid.

## Intermediate 65

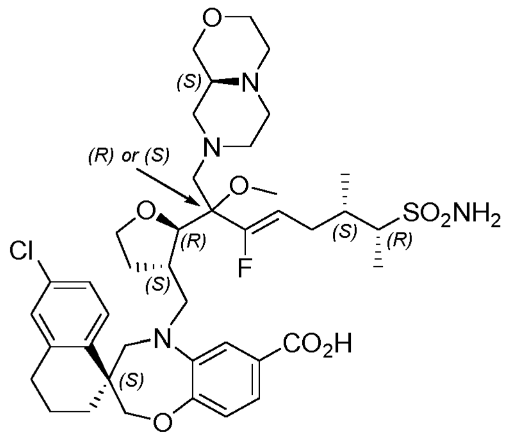

To a solution of **Intermediate 64** (125 mg, 0.15 mmol, 1.0 equiv.) in a mixture of THF (1.50 mL) and distilled water (1.50 mL) was added LiOH (14.4 mg, 0.60 mmol, 4.0 equiv.) at rt. The reaction mixture was then heated at 40 °C and stirred for 16 h. Upon completion, the reaction mixture was diluted with water, acidified by adding 1 N aqueous HCl, and extracted with EtOAc. The organic layers were combined, dried over anhydrous $MgSO_4$, filtered, and concentrated under reduced pressure. The crude **Intermediate 65** (100 mg, yield: 81 %) was used in the next step without further purification.

## Intermediate 66

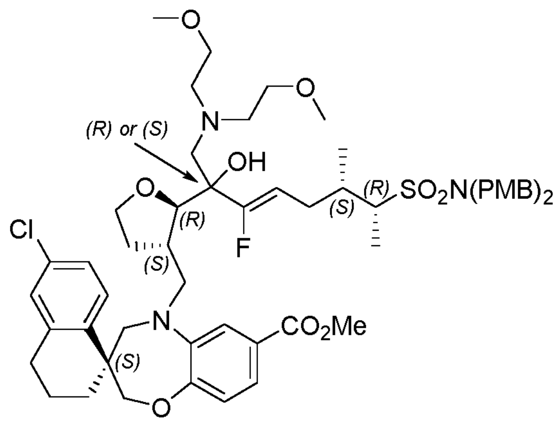

A solution composed of **Intermediate 61** (300 mg, 0.327 mmol, 1.0 equiv.) and bis(2-methoxyethyl)amine (CAS: 111-95-5, 131 mg, 0.981 mmol, 3.0 equiv.) in dry MeOH (6.0 mL) was heated at 70 °C for 16 h. Next, the reaction mixture was cooled down to rt, and water was added. The reaction mixture was extracted with EtOAc (3x). The organic layers were combined, washed with brine, dried over anhydrous $MgSO_4$, filtered, and concentrated under reduced pressure. The crude residue was purified by flash column chromatography (silica gel 24 g, 0-80 % EtOAc in heptane) to afford **Intermediate 66** (250 mg, yield: 73 %) as a pale-yellow solid.

## Intermediate 67

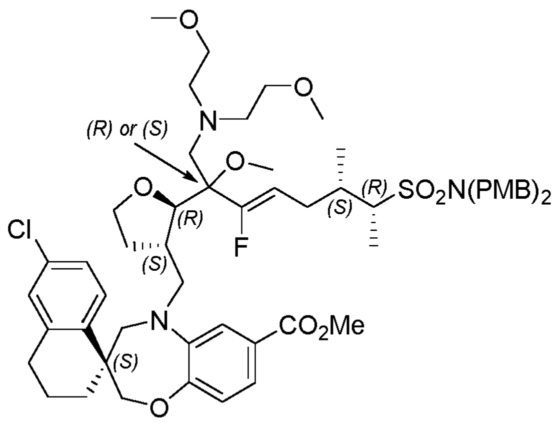

To a solution of **Intermediate 66** (250 mg, 0.238 mmol, 1.0 equiv.) and MeI (CAS: 74-88-4, 88.9 μL, 1.43 mmol, 6.0 equiv.) in anhydrous DMF (6.0 mL) was added NaH (60 % dispersion in mineral oil, 62 mg, 1.43 mmol, 6.0 equiv.) at rt under a $N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-80 % EtOAc in heptane) to give **Intermediate 67** (200 mg, yield: 79 %) as a white foam.

## Intermediate 68

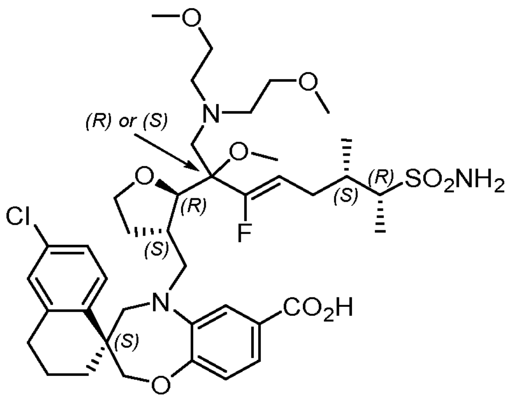

To a cooled (0 °C) solution of **Intermediate 67** (235 mg, 0.221 mmol, 1.0 equiv.) in anhydrous DCM (4.0 mL) was added TFA (CAS: 76-05-1, 1.92 mL, 26.5 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. Next, the crude residue was dissolved in a mixture of THF (5.0 mL) and water (5.0 mL) and LiOH (10.0 mg, 0.418 mmol, 1.9 equiv.) was added. The reaction mixture was stirred at rt for 14 h. The mixture was diluted with water (10 mL), acidified with 1 N aqueous HCl (0.5 mL), and then extracted with EtOAc (3x). The combined organic layers were dried over anhydrous $MgSO_4$, filtered, and concentrated under reduced pressure. The crude residue was purified by flash column chromatography (silica gel 12 g, 0-5 % MeOH in DCM) to give **Intermediate 68** (60 mg, yield: 33 %) as a colourless glassy solid.

## Intermediate 69

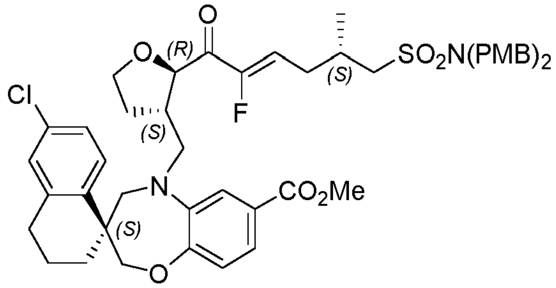

To a solution of **Intermediate 31** (360 mg, 0.404 mmol, 1.0 equiv.) in anhydrous DCM (10 mL) was added Dess-Martin periodinane (CAS: 87413-09-0, 257 mg, 0.606 mmol, 1.5 equiv.) at rt. The reaction mixture was then stirred for 2 h. Saturated aqueous $NaHCO_3$ was added to the reaction and it was stirred for a further 10 min. The reaction mixture was filtered through a short pad of Celite. The organic layer was separated, and the aqueous layer was extracted with DCM (2x). The combined organic layers were washed with brine, dried over anhydrous $MgSO_4$, filtered, and concentrated under reduced pressure. The crude residue was purified by flash column chromatography (silica gel 24 g, 0-50 % EtOAc in heptane) to give **Intermediate 69** (287 mg, yield: 97 %) as a white foam.

## Intermediate 70

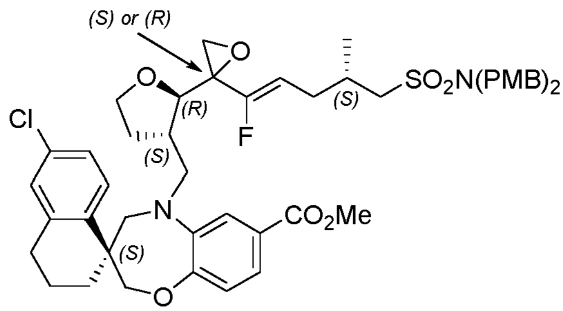

To a solution of **Intermediate 69** (280 mg, 0.315 mmol, 1.0 equiv.) and trimethylsulfoxonium iodide (CAS: 1774-47-6, 72 mg, 0.331 mmol, 1.05 equiv.) in dry DMSO (5.0 mL) was added potassium tert-butoxide (1.0 M solution in THF, 473 μL, 0.473 mmol, 1.5 equiv.), and the reaction mixture was stirred at rt under a $N_2$ atmosphere for 1 h. Upon completion, the reaction mixture was quenched with water and extracted with EtOAc (3x). The combined organic layers were washed with brine, dried over anhydrous $MgSO_4$, filtered, and concentrated under reduced pressure. The crude residue was purified by flash column chromatography (silica gel 12 g, 0-50 % EtOAc in heptane) to give **Intermediate 70** (97 mg, yield: 34 %) as a white foam. A single diastereomer was obtained.

## Intermediate 71

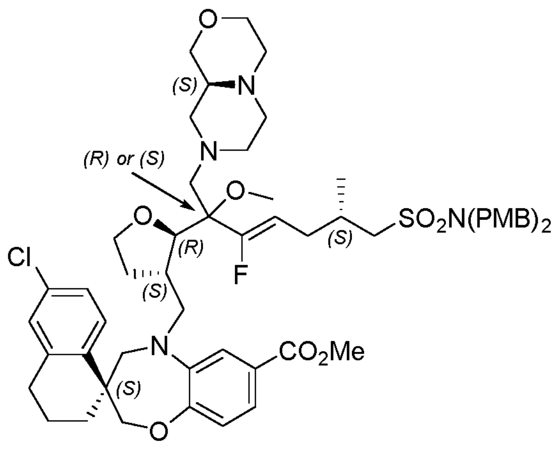

To a solution of **Intermediate 70** (95 mg, 0.105 mmol, 1.0 equiv.) and (S)-octahydropyrazino[2,1-c][1,4]oxazine dihydrochloride (CAS: 1089280-14-7, 68 mg, 0.315 mmol, 3.0 equiv.) in dry MeOH (2.0 mL) was added $Et_3N$ (146 μL, 1.05 mmol, 10 equiv.) at rt, and the reaction mixture was heated at 70 °C for 16 h. Next, the reaction mixture was cooled down to rt, and water was added. The reaction mixture was extracted with EtOAc (3x). The organic layers were combined, washed with brine, dried over anhydrous $MgSO_4$, filtered, and concentrated under reduced pressure. Next, the crude product was dissolved in anhydrous DMF (1.5 mL) and MeI (CAS: 74-88-4, 19.6 μL, 0.315 mmol, 3.0 equiv.) was added. After 10 min of stirring at rt under a $N_2$ atmosphere, NaH (60 % dispersion in mineral oil, 14 mg, 0.315 mmol, 3.0 equiv.) was added. The reaction mixture was stirred at 50 °C for 3 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 12 g, 0-60 % EtOAc in heptane) to give **Intermediate 71** (62 mg, yield: 56 %) as a pale-yellow gum.

## Intermediate 72

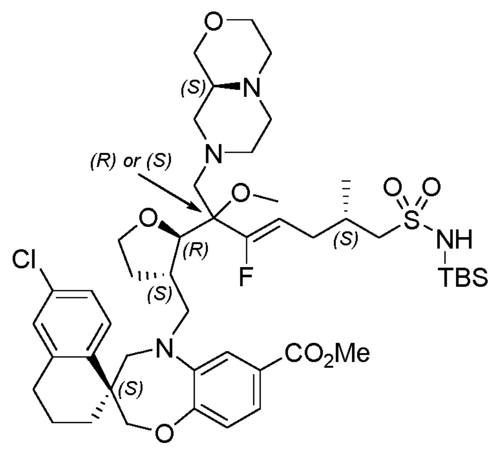

To a cooled (0 °C) solution of **Intermediate 71** (60 mg, 0.057 mmol, 1.0 equiv.) in anhydrous DCM (2.0 mL) was added TFA (CAS: 76-05-1, 523 μL, 6.84 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. Next, the crude residue was dissolved in anhydrous THF (5.0 mL) and NaH (60 % dispersion in mineral oil, 5 mg, 0.114 mmol, 2.0 equiv.) was added. The resultant suspension was then stirred at 0 °C for 1 h. Next, a solution of TBSCl (13 mg, 0.086 mmol, 1.5 equiv.) in anhydrous THF (0.5 mL) was added dropwise *via* syringe. The reaction mixture was then stirred at rt for 16 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 4 g, 0-60 % EtOAc in heptane) to give **Intermediate 72** (44 mg, yield: 83 %) as a white foam.

## Intermediate 73

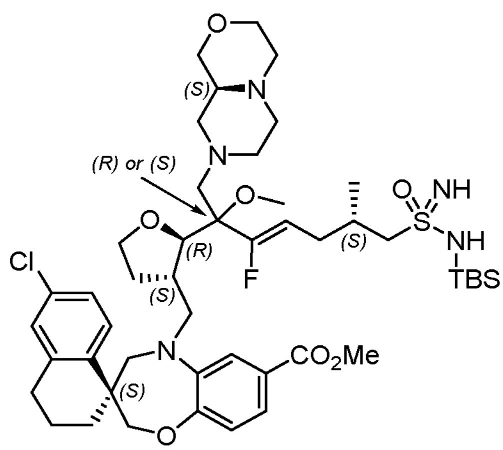

To a cooled (0 °C) solution of dichlorotriphenylphosphorane (CAS: 2526-64-9, 31 mg, 0.094 mmol, 2.0 equiv.) in anhydrous DCM (1.5 mL) was added $Et_3N$ (26 µL, 0.188 mmol, 4.0 equiv.) under a $N_2$ atmosphere. After stirring at 0 °C for 15 min, a solution of **Intermediate 72** (44 mg, 0.047 mmol, 1.0 equiv.) in anhydrous DCM (0.5 mL) was added dropwise. After stirring at 0 °C for 1 h, gaseous ammonia was bubbled through the reaction mixture during 5 min, followed by stirring at 0 °C for an additional 2 h. Upon completion, the reaction mixture was filtered through a pad of Celite (the filter cake was rinsed with DCM), and the filtrate was evaporated under reduced pressure. The crude residue was purified by flash column chromatography (silica gel 12 g, 0-100 % EtOAc in heptane) to give **Intermediate 73** (35 mg, yield: 80 %) as a colourless oil. Inseparable mixture of epimers at the chiral sulfur atom.

## Intermediate 74

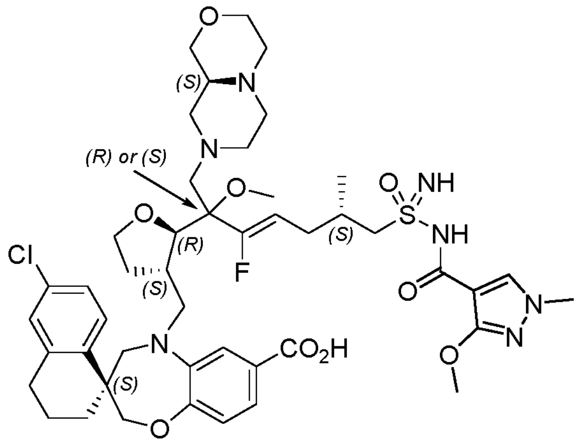

First, the acid chloride was formed separately from 3-methoxy-1-methyl-1H-pyrazole-4-carboxylic acid. To this end, a solution of 3-methoxy-1-methyl-1H-pyrazole-4-carboxylic acid (CAS: 113100-56-4, 8.5 mg, 0.053 mmol, 1.4 equiv.) in thionyl chloride (1.0 mL) was stirred and heated at 60 °C for 8 h. The reaction mixture was then cooled to ambient temperature, and volatiles were removed under reduced pressure. Next, the freshly prepared acid chloride was dissolved in anhydrous MeCN (2.0 mL) and treated with pyridazine (7 µL, 0.095 mmol, 2.5 equiv.). A solution of **Intermediate 73** (35 mg, 0.038 mmol, 1.0 equiv.) in anhydrous MeCN (1.5 mL) was then added at rt. The resultant reaction mixture was stirred at rt for 24 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude product was taken up in a mixture of THF (1.0 mL), MeOH (1.0 mL), and distilled water (0.2 mL), and LiOH (9.2 mg, 0.38 mmol, 10 equiv.) was added. The reaction mixture was then stirred at rt for 48 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The obtained crude **Intermediate 74** (27 mg, yield: 75 %) was used in the next step as such, without further purification.

## Intermediate 75

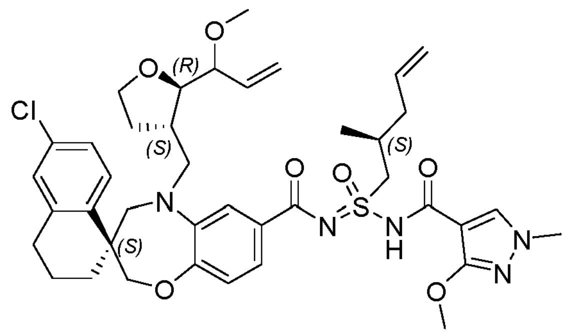

To a cooled (0 °C) solution of **Intermediate 47** (83 mg, 0.167 mmol, 1.0 equiv.) in anhydrous DCM (3.0 mL) was added $SOCl_2$ (0.70 mL, 9.65 mmol, 58 equiv.), and the reaction mixture was stirred at rt for 2 h. Next, the mixture was evaporated *in vacuo*, followed by co-evaporation with toluene to give the corresponding acyl chloride intermediate. It was then dissolved in anhydrous DCM (1.5 mL), and 3-methoxy-1-methyl-1H-pyrazole-4-carboxylic acid (CAS: 113100-56-4,

40 mg, 0.256 mmol, 1.5 equiv.) was added, followed by EDC.HCl (CAS: 25952-53-8, 50 mg, 0.261 mmol, 1.55 equiv.). After stirring at rt for 5 min, DMAP (30 mg, 0.246 mmol, 1.45 equiv.) was added to the reaction mixture, which was then stirred for 4 h. Upon completion, water was added, and the reaction mixture was extracted with EtOAc (3x). The combined organic layers were dried over anhydrous $MgSO_4$, filtered, and concentrated under reduced pressure. The crude residue was purified by flash column chromatography (silica gel 12 g, 0-5 % MeOH in DCM) to afford **Intermediate 75** (87 mg, yield: 91 %) as a white solid. Inseparable mixture of diastereomers at the methoxy-substituted carbon and the chiral sulfur atoms.

## Intermediate 76

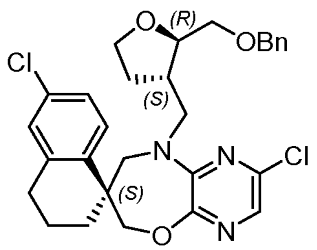

To a solution of **Intermediate 7** (2.71 g, 8.05 mmol, 1.0 equiv.) and (*S*)-2',6-dichloro-3,4,8',9'-tetrahydro-2H,6'H-spiro[naphthalene-1,7'-pyrazino[2,3-b][1,4]oxazepine] (2.66 g, 8.85 mmol, 1.1 equiv.) in dry DMF (45 mL) was added NaH (60 % dispersion in mineral oil, 810 mg, 20.3 mmol, 2.5 equiv.), and the reaction mixture was heated at 50 °C for 2 h. The reaction mixture was then cooled down to rt, quenched with water, and extracted with EtOAc (3x). The organic layers were combined, washed with brine, dried over anhydrous $MgSO_4$, filtered, and concentrated under reduced pressure. The crude residue was purified by flash column chromatography (silica gel 40 g, 0-40 % EtOAc in heptane) to give **Intermediate 76** (3.29 g, yield: 76 %) as a pale-yellow solid.

## Intermediate 77

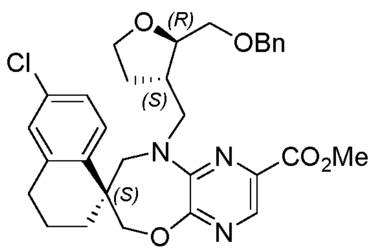

An autoclave was charged with **Intermediate 76** (4.04 g, 7.48 mmol, 1.0 equiv.), $Et_3N$ (2.37 mL, 17.1 mmol, 2.3 equiv.), and [1,1'-bis(diphenylphosphino)ferrocene]dichloropalladium(II) (CAS: 72287-26-4, 65.5 mg, 0.748 mmol, 0.1 equiv.) in dry MeOH (100 mL). The autoclave was then sealed, flushed once with carbon monoxide, and pressurized with carbon monoxide (30 bar). The resultant mixture was heated at 135 °C for 24 h. After cooling sown to rt, the reaction mixture was filtered through a short pad of Celite, and the filter cake was rinsed with MeOH. The volatiles were then removed under reduced pressure. The crude residue was purified by flash column chromatography (silica gel 100 g, 0-60 % EtOAc in heptane) to give **Intermediate 77** (3.14 g, yield: 74 %) as a pale-yellow solid.

## Intermediate 78

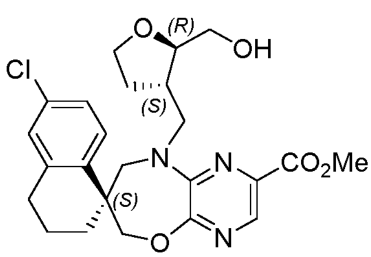

**Intermediate 77** (2.90 g, 5.14 mmol, 1.0 equiv.), $Pd(OH)_2$ on carbon (CAS: 12135-22-7, 144 mg, 1.03 mmol, 0.2 equiv.), and dry EtOAc (75 mL) were charged into a 500 mL Schlenk tube, and the resultant mixture was stirred at rt under a $H_2$ atmosphere for 2 h. The reaction progress was monitored by LCMS. An additional portion of $Pd(OH)_2$ on carbon (CAS:

12135-22-7, 72 mg, 0.51 mmol, 0.1 equiv.) was added, and the reaction mixture was stirred for another 2 h. Upon completion, the reaction mixture was filtered through a short pad of Celite, and the filter cake was rinsed with EtOAc. The filtrate was then concentrated under reduced pressure. The crude residue was purified by flash column chromatography (silica gel 40 g, 0-100 % EtOAc in heptane) to afford **Intermediate 78** (2.12 g, yield: 87 %) as a white foam.

## Intermediate 79

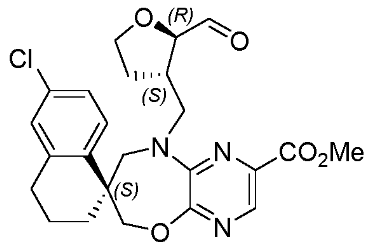

To a cooled (-78 °C) solution of DMSO (299 µL, 4.21 mmol, 4.0 equiv.) in anhydrous DCM (8.0 mL) under a $N_2$ atmosphere was added oxalyl chloride (CAS: 79-37-8, 178 µL, 2.11 mmol, 2.0 equiv.). After 30 min of stirring, a solution of **Intermediate 78** (500 mg, 1.06 mmol, 1.0 equiv.) in anhydrous DCM (2.0 mL) was added dropwise at -78 °C. The reaction mixture was stirred for 1 h, and then $Et_3N$ (863 µL, 6.21 mmol, 6.0 equiv.) was added dropwise at -78 °C. The reaction mixture was stirred for 1 h at -78 °C, and then slowly warmed up to -20 °C during 1 h. Water was added in -20 °C to quench the reaction, and the mixture was brought back to rt. The reaction mixture was extracted with EtOAc (3x), and the combined organic layers were washed with brine, dried over anhydrous $MgSO_4$, filtered, and concentrated under reduced pressure. The crude residue was purified by flash column chromatography (silica gel 24 g, 0-70 % EtOAc in heptane) to give **Intermediate 79** (485 mg, yield: 97 %) as a colourless gum.

## Intermediate 80

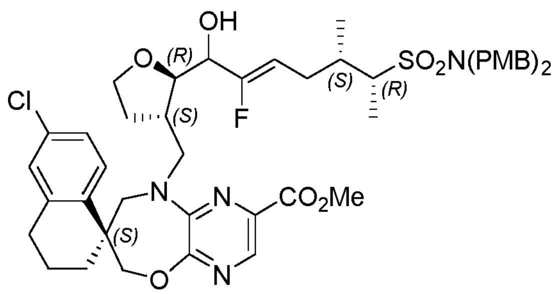

A mixture of anhydrous $CrCl_2$ (CAS: 10049-05-5, 340 mg, 2.77 mmol, 3.0 equiv.) and $NiBr_2$ (CAS: 13462-88-9, 60 mg, 0.277 mmol, 0.3 equiv.) in dry DMF (3.0 mL) was degassed by bubbling $N_2$ through the reaction solution. To this was added a solution of **Intermediate 79** (435 mg, 0.922 mmol, 1.0 equiv.) and (2*R*,3*S*,*E*)-6-bromo-6-fluoro-N,N-bis(4-methoxybenzyl)-3-methylhex-5-ene-2-sulfonamide (711 mg, 1.38 mmol, 1.5 equiv.) in dry DMF (3.0 mL) dropwise *via* syringe pump (addition rate: 10 mL/h) at rt under a $N_2$ atmosphere. After the addition, the reaction mixture was stirred at rt for 12 h.

Next, ethylenediamine (CAS: 107-15-3, 293 µL, 4.61 mmol, 3.0 equiv.) was added to the reaction and it was stirred for 10 min until the colour turned purple. Next, the reaction mixture was filtered through a short pad of Celite, the filter cake was washed with EtOAc, and the filtrate was concentrated under reduced pressure. The crude residue was purified by flash column chromatography (silica gel 24 g, 0-60 % EtOAc in heptane) to give **Intermediate 80** (642 mg, yield: 77 %) as a white foam. Inseparable mixture of epimers at the hydroxy-substituted carbon.

## Intermediate 81

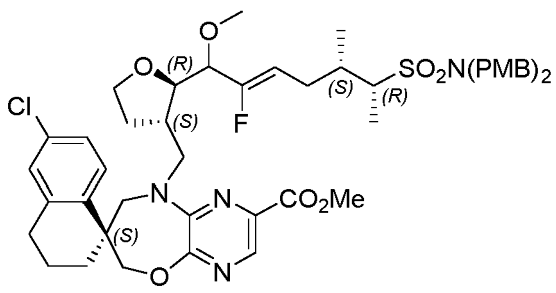

To a solution of **Intermediate 80** (640 mg, 0.705 mmol, 1.0 equiv.) and MeI (CAS: 74-88-4, 263 μL, 4.23 mmol, 6.0 equiv.) in anhydrous DMF (14 mL) was added NaH (60 % dispersion in mineral oil, 182 mg, 4.23 mmol, 6.0 equiv.) at rt under a $N_2$ atmosphere. The resultant suspension was then stirred at 50 °C for 1 h. Upon completion, the reaction mixture was quenched with saturated aqueous $NH_4Cl$ and extracted with EtOAc (3x). The combined organic layers were washed with brine, dried over anhydrous $MgSO_4$, filtered, and concentrated under reduced pressure. The crude residue was purified by flash column chromatography (silica gel 24 g, 0-60 % EtOAc in heptane) to give **Intermediate 81** (520 mg, yield: 80 %) as a white foam.

## Intermediate 82

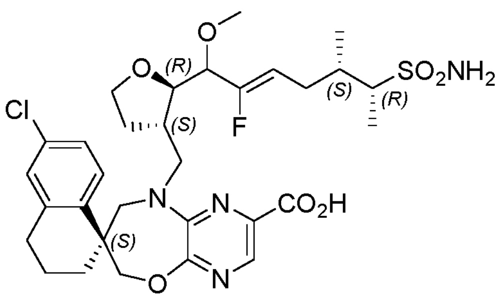

To a cooled (0 °C) solution of **Intermediate 81** (249 mg, 0.217 mmol, 1.0 equiv.) in anhydrous DCM (3.5 mL) was added TFA (CAS: 76-05-1, 1.86 mL, 26.0 mmol, 120 equiv.). The resultant mixture was then stirred at ambient temperature for 16 h. After that, saturated aqueous $Na_2CO_3$ was added to quench the reaction, and the mixture was extracted with DCM (3x). The organic layers were combined, washed with brine, dried over anhydrous $MgSO_4$, filtered, and concentrated under reduced pressure. Next, the crude residue was dissolved in a mixture of THF (5.0 mL) and water (5.0 mL) and LiOH (7.03 mg, 0.294 mmol, 1.35 equiv.) was added. The reaction mixture was stirred at rt for 14 h. The mixture was diluted with water (10 mL), acidified with 1 N aqueous HCl (0.5 mL), and then extracted with EtOAc (3x). The combined organic layers were dried over anhydrous $MgSO_4$, filtered, and concentrated under reduced pressure. The crude residue was purified by flash column chromatography (silica gel 12 g, 0-5 % MeOH in DCM) to give **Intermediate 82** (124 mg, yield: 60 %) as a colourless glassy solid.

## Intermediate 83

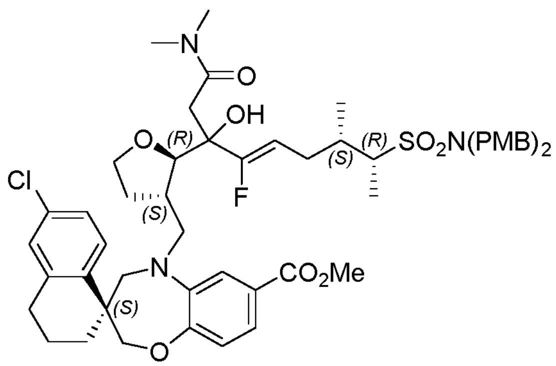

LDA (CAS: 4111-54-0, 2.0 M solution in THF/heptane/ethylbenzene, 392 μL, 0.781 mmol, 2.5 equiv.) was added

to a cooled (-78 °C) solution of anhydrous DMA (73 μL, 0.781 mmol, 2.5 equiv.) in anhydrous THF (4.0 mL). The resultant mixture was stirred at -78 °C for 1 h. Next, a solution of **Intermediate 60** (284 mg, 0.311 mmol, 1.0 equiv.) in anhydrous THF (6.0 mL) was introduced dropwise at -78 °C. The reaction mixture was stirred at -78 °C for 1 h, and then quenched with saturated aqueous $NH_4Cl$ (50 mL). The mixture was extracted with EtOAc (2x). The combined organic layers were dried over anhydrous $Na_2SO_4$, filtered, and concentrated under reduced pressure. The crude residue was purified by flash column chromatography (silica gel 12 g, 0-40 % EtOAc in heptane) to give **Intermediate 83** (132 mg, yield: 42 %) as a white foam. Inseparable mixture of two epimers at the hydroxy-substituted quaternary carbon atom.

## Intermediate 84

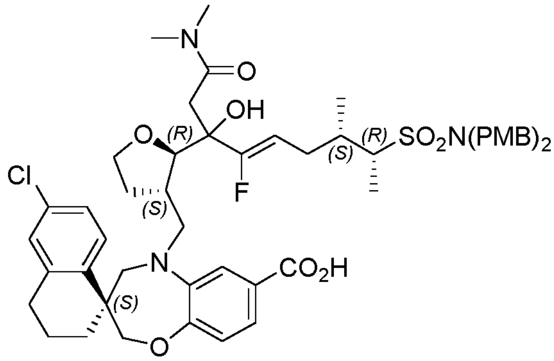

A solution of **Intermediate 83** (120 mg, 0.12 mmol, 1.0 equiv.) and LiOH monohydrate (51 mg, 1.21 mmol, 10 equiv.) was stirred in a mixture of THF/MeOH/water 3:1:1 (5.0 mL) at 50 °C for 12 h. Next, the reaction mixture was diluted with water, cooled to 0 °C, and acidified by slow addition of concentrated aqueous HCl (pH *ca.* 3). Next, the reaction mixture was extracted with diethyl ether.

The combined organic layers were washed with brine (10 mL), dried over anhydrous $Na_2SO_4$ and concentrated under reduced pressure to give the product **Intermediate 84** (115 mg, 87 %) as a white foam. The crude product was used in the next step as such without further purification.

## Intermediate 85

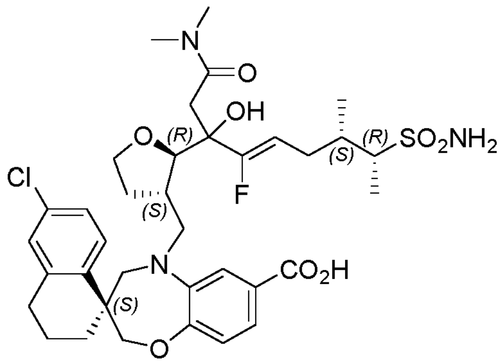

TFA (1.0 mL, 11.3 mmol, 240 equiv.) was added to a solution of **Intermediate 84** (46 mg, 0.047 mmol, 1.0 equiv.) in anhydrous DCM (5.0 mL), and the reaction mixture was stirred at rt for 16 h. Volatiles were then removed under reduced pressure, and the residue was diluted with DCM (50 mL) and saturated aqueous NaHCOs (50 mL). The organic layer was separated, and the aqueous layer was extracted with DCM (2x). The combined organic layers were washed with brine, dried over anhydrous $MgSO_4$, filtered, and concentrated under reduced pressure. The crude **Intermediate** 85 (31 mg, yield: 89 %) was obtained as a colourless film and used in the next step without further purification.

**Preparation of final compounds**

## Compound 1

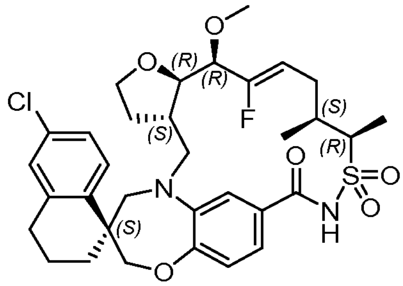

DBU (CAS: 6674-22-2, 89.7 μL, 0.601 mmol, 10 equiv.) was added to a stirred solution of **Intermediate 29** (40 mg, 0.060 mmol, 1.0 equiv.) in anhydrous DMF (6.0 mL) under a $N_2$ atmosphere. After addition, the reaction mixture was stirred at rt for 15 min, and then diethyl cyanophosphonate (CAS: 2942-58-7, 27 μL, 0.18 mmol, 3.0 equiv.) was added gently in one portion. After addition, the reaction mixture was stirred at rt for 40 min. Saturated aqueous $NH_4Cl$ was added to quench the reaction, and the mixture was extracted with EtOAc (3x). The organic layers were combined, washed with brine, dried over anhydrous $MgSO_4$, filtered, and concentrated under reduced pressure. The crude residue was purified by preparative SFC (Stationary phase: Chiralcel Diacel OJ 20 x 250 mm; Mobile phase: $CO_2$, EtOH + 0.4 % *i*-$PrNH_2$) to give **Compound** 1 (20 mg, yield: 51 %) as a white solid.

$^1H$ NMR δ 1.07 (d, *J*=5.85 Hz, 3 H) 1.38 - 1.51 (m, 4 H) 1.72 - 1.88 (m, 1 H) 1.75 - 1.84 (m, 1 H) 1.92 - 2.16 (m, 5 H) 2.27 (br dd, *J*=12.65, 8.67 Hz, 1 H) 2.59 - 2.68 (m, 1 H) 2.72 - 2.84 (m, 2 H) 3.05 (br dd, *J*=15.21, 10.19 Hz, 1 H) 3.35 (br d, *J*=14.32 Hz, 1 H) 3.40 (s, 3 H) 3.61 - 3.74 (m, 2 H) 3.87 (dd, *J*=7.84, 2.61 Hz, 1 H) 3.90 - 3.99 (m, 3 H) 4.03 (d, *J*=12.23 Hz, 1 H) 4.05 - 4.11 (m, 1 H) 4.17 (d, *J*=12.12 Hz, 1 H) 4.89 - 5.13 (m, 1 H) 6.96 (d, *J*=8.26 Hz, 2 H) 6.99 - 7.05 (m, 2 H) 7.10 (d, *J*=2.19 Hz, 1 H) 7.16 - 7.23 (m, 1 H) 7.71 (d, *J*=8.47 Hz, 1 H); LCMS confirms the MW (RT: 1.98, MW: 646.30, $[M+H]^+$ 647, Method 1).

## Compound 2

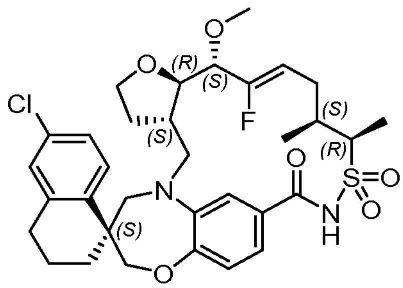

DBU (CAS: 6674-22-2, 34 μL, 0.225 mmol, 10 equiv.) was added to a stirred solution of **Intermediate 30** (15 mg, 0.023 mmol, 1.0 equiv.) in anhydrous DMF (2.25 mL) under a $N_2$ atmosphere. After addition, the reaction mixture was stirred at rt for 15 min, and then diethyl cyanophosphonate (CAS: 2942-58-7, 10 μL, 0.068 mmol, 3.0 equiv.) was added gently in one portion. After addition, the reaction mixture was stirred at rt for 40 min. Saturated aqueous $NH_4Cl$ was added to quench the reaction, and the mixture was extracted with EtOAc (3x). The organic layers were combined, washed with brine, dried over anhydrous $MgSO_4$, filtered, and concentrated under reduced pressure. The crude residue was purified by flash column chromatography (silica gel 4 g, 0-50 % EtOAc in heptane) to give **Compound** 2 (7.2 mg, yield: 48 %) as a white solid.

$^1H$ NMR δ 1.08 (d, *J*=6.38 Hz, 3 H) 1.46 (d, *J*=7.21 Hz, 3 H) 1.68 - 1.87 (m, 2 H) 1.89 - 1.99 (m, 1 H) 2.00 - 2.12 (m, 3 H) 2.12 - 2.19 (m, 1 H) 2.66 - 2.84 (m, 3 H) 2.85 - 2.99 (m, 1 H) 3.31 (d, *J*=14.53 Hz, 1 H) 3.41 - 3.51 (m, 1 H) 3.55 (s, 3 H) 3.80 - 3.85 (m, 1 H) 3.87 (br d, *J*=10.24 Hz, 1 H) 3.89 - 3.96 (m, 1 H) 3.97 - 4.20 (m, 5 H) 4.24 - 4.32 (m, 1 H) 4.84 (dt, *J*=39.43, 4.95 Hz, 1 H) 6.83 - 6.91 (m, 1 H) 6.92 - 6.98 (m, 1 H) 7.09 (d, *J*=2.19 Hz, 1 H) 7.20 (dd, *J*=8.47, 2.40 Hz, 1 H) 7.46 (d, *J*=1.99 Hz, 1 H) 7.71 (d, *J*=8.57 Hz, 1 H); LCMS confirms the MW (RT: 1.09, MW: 646.00, $[M+H]^+$ 647, Method 2).

## Compound 3

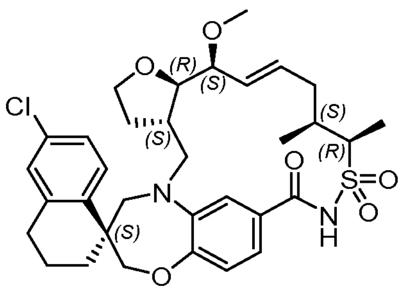

A solution of **Intermediate 48** (22 mg, 0.033 mmol, 1.0 equiv.) and Hoveyda-Grubbs 2nd generation catalyst (CAS: 301224-40-8, 8.4 mg, 0.013 mmol, 0.4 equiv.) in anhydrous DCE (13 mL) was degassed by bubbling a stream of dry $N_2$ through the solution for *ca.* 15 min. The reaction vessel was then sealed, and the reaction mixture was heated at 60 °C for 24 h. After cooling down to rt, ethyl vinyl ether (200 μL) was added to quench the metathesis catalyst. After 1 h of vigorous stirring, the reaction mixture was filtered over Celite (the filter cake was rinsed with DCM), and the filtrate was evaporated under reduced pressure. The crude residue was purified by flash column chromatography (silica gel 4 g, 0-50 % EtOAc in heptane), followed by a second purification by reverse-phase preparative HPLC (Stationary phase: C18 column; Mobile phase: MeCN + $NH_4HCO_3$ solution) to give **Compound** 3 (11.2 mg, yield: 54 %) as a white solid.

$^1$H NMR δ 1.04 (d, *J*=6.79 Hz, 3 H) 1.40 - 1.52 (m, 5 H) 1.55 - 1.73 (m, 3 H) 1.75 - 1.89 (m, 3 H) 1.92 - 2.10 (m, 6 H) 2.14 - 2.23 (m, 1 H) 2.34 - 2.46 (m, 1 H) 2.69 - 2.85 (m, 2 H) 2.97 (dd, *J*=15.21, 10.71 Hz, 1 H) 3.27 - 3.34 (m, 4 H) 3.57 (ddd, *J*=10.76, 8.47, 5.12 Hz, 1 H) 3.70 (d, *J*=9.20 Hz, 1 H) 3.79 (d, *J*=9.72 Hz, 1 H) 3.92 (br d, *J*=14.11 Hz, 1 H) 3.95 - 4.03 (m, 3 H) 4.17 (d, *J*=12.23 Hz, 1 H) 4.25 - 4.37 (m, 1 H) 5.51 - 5.59 (m, 1 H) 5.94 (ddd, *J*=15.42, 9.82, 2.87 Hz, 1 H) 6.85 (s, 1 H) 6.94 (s, 2 H) 7.09 (d, J 2.30 Hz, 1 H) 7.19 (dd, J=8.47, 2.19 Hz, 1 H) 7.70 (d, J=8.47 Hz, 1 H); LCMS confirms the MW (RT: 1.03, MW: 629.00, $[M+H]^+$ 629, Method 2).

## Compounds 4 and 5

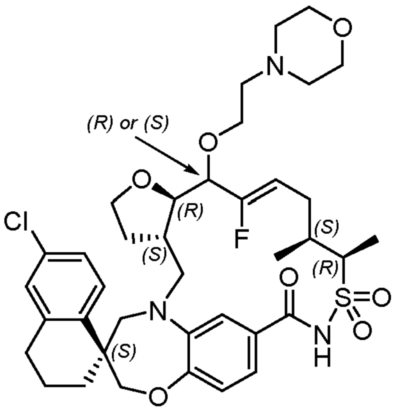

4

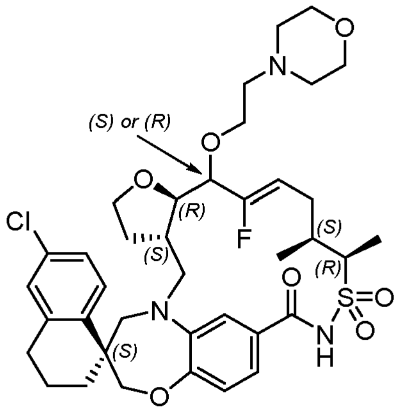

5

DBU (CAS: 6674-22-2, 95.7 μL, 0.641 mmol, 10 equiv.) was added to a stirred solution of **Intermediate 59** (49 mg, 0.064 mmol, 1.0 equiv.) in anhydrous DMF (5.0 mL) under a $N_2$ atmosphere. After addition, the reaction mixture was stirred at rt for 15 min, and then diethyl cyanophosphonate (CAS: 2942-58-7, 28.8 μL, 0.192 mmol, 3.0 equiv.) was added gently in one portion. After addition, the reaction mixture was stirred at rt for 40 min. Saturated aqueous $NH_4Cl$ was added to quench the reaction, and the mixture was extracted with EtOAc (3x). The organic layers were combined, washed with brine, dried over anhydrous $MgSO_4$, filtered, and concentrated under reduced pressure. The crude residue was purified by flash column chromatography (silica gel 4 g, 0-60 % EtOAc in heptane), followed by a second purification by reverse-phase preparative HPLC (Stationary phase: C18 column; Mobile phase: MeCN + $NH_4HCO_3$ solution) to give **Compound 4** (17 mg, yield: 36 %) and **Compound 5** (10 mg, yield: 21 %) as white solids.

**Compound 4.** $^1$H NMR δ 0.95 - 1.11 (m, 3 H) 1.40 - 1.48 (m, 3 H) 1.73 - 1.84 (m, 2 H) 1.92 - 2.13 (m, 6 H) 2.31 - 2.87 (m, 13 H) 2.91 - 3.05 (m, 1 H) 3.31 (br d, *J*=14.21 Hz, 1 H) 3.53 - 4.24 (m, 14 H) 4.91 - 5.16 (m, 1 H) 6.86 - 6.98 (m, 1 H) 6.98 - 7.12 (m, 2 H) 7.14 - 7.21 (m, 1 H) 7.70 (d, *J*=8.47 Hz, 1 H); LCMS confirms the MW (RT: 1.00, MW: 745.00, $[M+H]^+$ 746, Method 2).

**Compound 5.** $^1$H NMR δ 1.06 (br d, *J*=6.06 Hz, 3 H) 1.41 - 1.48 (m, 3 H) 1.54 - 2.30 (m, 10 H) 2.45 - 2.64 (m, 4 H)

2.68 - 2.83 (m, 4 H) 2.83 - 2.96 (m, 2 H) 3.30 - 3.40 (m, 2 H) 3.43 - 3.59 (m, 2 H) 3.60 - 3.69 (m, 3 H) 3.80 - 3.94 (m, 3 H) 3.96 - 4.14 (m, 53H) 4.24 (br t, *J*=5.33 Hz, 1 H) 4.42 - 4.50 (m, 1 H) 4.87 - 5.09 (m, 1 H) 6.88 - 7.01 (m, 2 H) 7.08 - 7.25 (m, 2 H) 7.41 - 7.47 (m, 1 H) 7.72 (d, *J*=8.57 Hz, 1 H); LCMS confirms the MW (RT: 1.04, MW: 745.00, $[M+H]^+$ 747, Method 2).

## Compounds 6 and 7

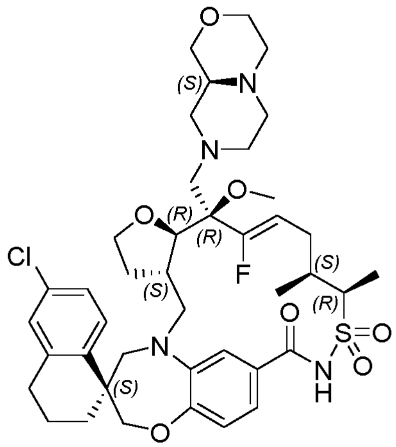

**6**

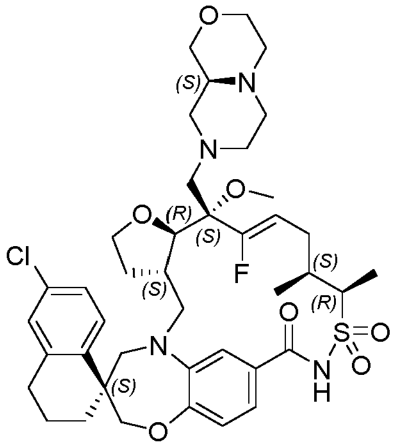

**7**

DBU (CAS: 6674-22-2, 146 μL, 0.976 mmol, 10 equiv.) was added to a stirred solution of **Intermediate 65** (80 mg, 0.098 mmol, 1.0 equiv.) in anhydrous DMF (10 mL) under a $N_2$ atmosphere. After addition, the reaction mixture was stirred at rt for 15 min, and then diethyl cyanophosphonate (CAS: 2942-58-7, 43.8 μL, 0.293 mmol, 3.0 equiv.) was added gently in one portion. After addition, the reaction mixture was stirred at rt for 40 min. Saturated aqueous $NH_4Cl$ was added to quench the reaction, and the mixture was extracted with EtOAc (3x). The organic layers were combined, washed with brine, dried over anhydrous $MgSO_4$, filtered, and concentrated under reduced pressure. The crude residue was purified by flash column chromatography (silica gel 4 g, 0-60 % EtOAc in heptane), followed by a second purification by reverse-phase preparative HPLC (Stationary phase: C18 column; Mobile phase: MeCN + $NH_4HCO_3$ solution) to give **Compound** 6 (19 mg, yield: 24 %) and **Compound 7** (11 mg, yield: 14 %) as white solids.

**Compound** 6. $^1$H NMR (400 MHz, CHLOROFORM-*d*) δ ppm 1.04 (d, *J*=5.9 Hz, 3 H) 1.37 (d, *J*=7.1 Hz, 3 H) 1.39 - 1.46 (m, 1 H) 1.68 - 1.88 (m, 2 H) 1.88 - 1.98 (m, 3 H) 2.02 - 2.19 (m, 6 H) 2.36 - 2.56 (m, 5 H) 2.70 - 2.97 (m, 10 H) 3.06 - 3.15 (m, 1 H) 3.24 (t, *J*=10.8 Hz, 1 H) 3.33 - 3.41 (m, 2 H) 3.51 (s, 3 H) 3.62 - 3.71 (m, 2 H) 3.75 - 3.96 (m, 4 H) 3.97 - 4.07 (m, 3 H) 4.09-4.15 (m, 1 H) 4.25 (br d, *J*=13.2 Hz, 1 H) 4.94-5.11 (m, 1 H) 6.90 - 6.93 (m, 1 H) 6.97 - 7.03 (m, 1 H) 7.08 (d, *J*=2.2 Hz, 1 H) 7.17 (dd, *J*=8.5, 2.3 Hz, 1 H) 7.35 (d, *J*=1.7 Hz, 1 H) 7.74 (d, *J*=8.5 Hz, 1 H).

LCMS confirms the MW (RT: 1.03, MW: 800.00, $[M+H]^+$ 801, Method 2).

**Compound** 7. $^1$H NMR (400 MHz, CHLOROFORM-*d*) δ ppm 1.05 (d, *J*=6.4 Hz, 3 H) 1.39 (d, *J*=7.1 Hz, 3 H) 1.50 (br dd, *J*=14.4, 7.5 Hz, 1 H) 1.82 - 2.09 (m, 8 H) 2.19 - 2.31 (m, 1 H) 2.34 - 2.47 (m, 3 H) 2.56 (br t, *J*=11.1 Hz, 1 H) 2.71 - 2.96 (m, 8 H) 3.08 - 3.19 (m, 1 H) 3.23 (s, 3 H) 3.37 - 3.47 (m, 1 H) 3.55 - 3.72 (m, 3 H) 3.76 - 3.88 (m, 2 H) 3.92 - 4.07 (m, 4 H) 4.14 - 4.28 (m, 2 H) 5.11 - 5.29 (m, 1 H) 6.92 (d, J=8.0 Hz, 1 H) 7.04 - 7.14 (m, 3 H) 7.18 (dd, *J*=8.5, 2.2 Hz, 1 H) 7.74 (d, *J*=8.6 Hz, 1 H).

LCMS confirms the MW (RT: 1.12, MW: 800.00, $[M+H]^+$ 801, Method 2).

## Compounds 8, 9, 10, and 11

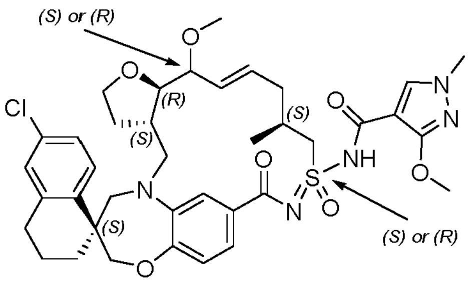

**8**

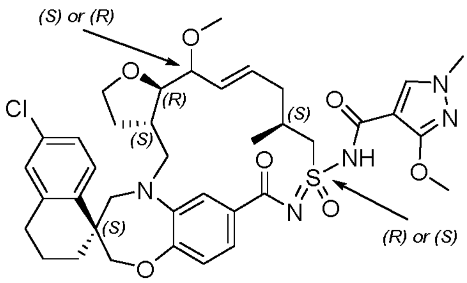

**9**

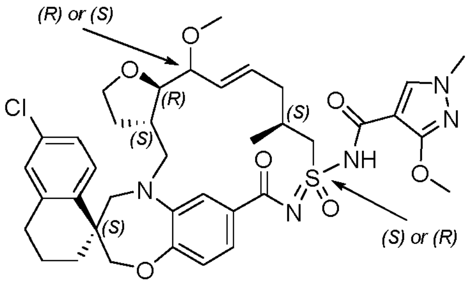

**10**

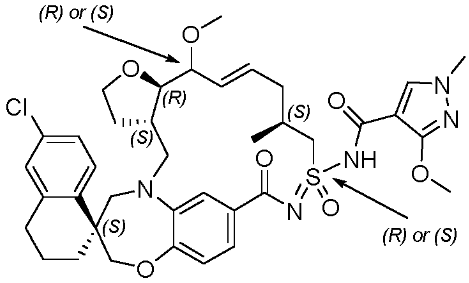

**11**

A solution of **Intermediate 75** (87 mg, 0.111 mmol, 1.0 equiv.) and Hoveyda-Grubbs 2nd generation catalyst (CAS: 301224-40-8, 14 mg, 0.022 mmol, 0.4 equiv.) in anhydrous DCE (50 mL) was degassed by bubbling a stream of dry $N_2$ through the solution for *ca.* 15 min. The reaction vessel was then sealed, and the reaction mixture was heated at 60 °C for 24 h. After cooling down to rt, ethyl vinyl ether (500 μL) was added to quench the metathesis catalyst. After 1 h of vigorous stirring, the reaction mixture was filtered over Celite (the filter cake was rinsed with DCM), and the filtrate was evaporated under reduced pressure. The crude residue was purified by flash column chromatography (silica gel 12 g, 0-80 % EtOAc in heptane), followed by a second purification by preparative SFC (Stationary phase: Chiralcel Diacel IH 20 x 250 mm; Mobile phase: $CO_2$, *i*PrOH/MeOH 1:1 + 0.4 % *i*-$PrNH_2$) to give **Compound 8** (7.5 mg, yield: 9 %), **Compound 9** (5 mg, yield: 5 %), **Compound 10** (7.5 mg, yield: 7 %), and **Compound 11** (4 mg, yield: 4 %) as white solids.

**Compound 8.** LCMS confirms the MW (RT: 1.89, MW: 751.30, $[M+H]^+$ 752, Method 1); SFC (Rt 4.95min 95.05% isomer 1), (Rt 5.16min 4.95% isomer 2) manual integration (RT: 4.95, Area %: 95.05, MW: 751.28, BPM1: 752, BPM2: 750, Method 1).

**Compound** 9. LCMS confirms the MW (RT: 1.97, MW: 751.30, $[M+H]^+$ 752, Method 3); SFC (Rt 5.30 min 0.00% isomer 1) (Rt 5.58 min 0.00% isomer 2), (Rt 5.65 min 100.00% isomer 3) (RT: 5.65, Area %: 100.00, Method 1).

**Compound 10.** LCMS confirms the MW (RT: 1.87, MW: 751.30, $[M+H]^+$ 752, Method 1); SFC (Rt 5.30 min 0.00% isomer 1) (Rt 5.58 min 0.00% isomer 2), (Rt 5.65 min 0.00% isomer 3) (RT: 6.31, Area %: 85.59, Method 1).

**Compound 11.** LCMS confirms the MW (RT: 1.98, MW: 751.30, $[M+H]^+$ 752, Method 1); SFC (Rt 5.30 min 0.00% isomer 1) (Rt 5.58 min 0.00% isomer 2), (Rt 5.65 min 0.00% isomer 3) (RT: 6.00, Area %: 99.12, Method 1).

## Compounds 12 and 13

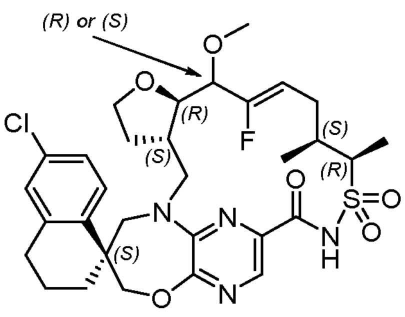

**12**

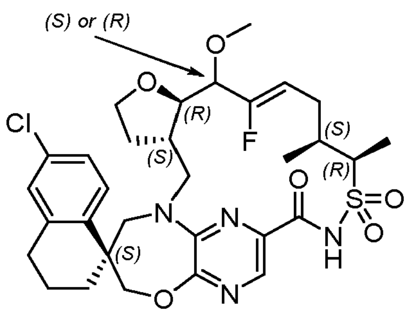

**13**

DBU (CAS: 6674-22-2, 233 μL, 1.56 mmol, 10 equiv.) was added to a stirred solution of **Intermediate 82** (104 mg, 0.156 mmol, 1.0 equiv.) in anhydrous DMF (12 mL) under a $N_2$ atmosphere. After addition, the reaction mixture was stirred at rt for 15 min, and then diethyl cyanophosphonate (CAS: 2942-58-7, 70 μL, 0.468 mmol, 3.0 equiv.) was added gently in one portion. After addition, the reaction mixture was stirred at rt for 40 min. Saturated aqueous $NH_4Cl$ was added to quench the reaction, and the mixture was extracted with EtOAc (3x). The organic layers were combined, washed with brine, dried over anhydrous $MgSO_4$, filtered, and concentrated under reduced pressure. The crude residue was purified by flash column chromatography (silica gel 12 g, 0-60 % EtOAc in heptane), followed by a second purification by preparative SFC (Stationary phase: Chiralcel Diacel OJ 20 × 250 mm; Mobile phase: $CO_2$, EtOH + 0.4 % *i*-$PrNH_2$) to give **Compound 12** (10 mg, yield: 10 %) and **Compound 13** (11 mg, yield: 11 %) as white solids.

**Compound 12.** $^1$H NMR δ 1.10 - 1.19 (m, 3 H) 1.41 (d, *J*=7.26 Hz, 3 H) 1.78 - 1.95 (m, 3 H) 2.06 - 2.33 (m, 8 H) 2.67 (br d, *J*=3.96 Hz, 1 H) 2.75 - 2.88 (m, 2 H) 3.14 - 3.21 (m, 1 H) 3.24 (s, 3 H) 3.38 (br dd, *J*=13.97, 3.19 Hz, 1 H) 3.55 (d, *J*=14.74 Hz, 1 H) 3.71 - 3.86 (m, 2 H) 3.95 - 4.09 (m, 2 H) 4.19 - 4.28 (m, 2 H) 4.38 (d, *J*=12.54 Hz, 1 H) 4.61 - 4.81 (m, 1 H) 7.11 (d, *J*=2.20 Hz, 1 H) 7.20 (dd, *J*=8.47, 2.31 Hz, 1 H) 7.73 (d, *J*=8.36 Hz, 1 H) 8.43 (s, 1 H); LCMS confirms the MW (RT: 1.87, MW: 648.20, $[M+H]^+$ 649, Method 1); SFC (Rt 5.92min 100.00% isomer 1), (Rt 6.37min 0.00% isomer 2) (RT: 5.92, Area %: 100.00, MW: 648.2, BPM1: 708 $[M+iPrNH2]^+$, Method 2).

**Compound 13.** $^1$H NMR δ 1.16 (d, *J*=5.94 Hz, 3 H) 1.39 (d, *J*=7.26 Hz, 3 H) 1.65 - 1.73 (m, 1 H) 1.78 - 2.04 (m, 7 H) 2.08 - 2.21 (m, 3 H) 2.61 - 2.71 (m, 1 H) 2.75 - 2.86 (m, 2 H) 2.86 - 2.96 (m, 1 H) 3.19 (s, 2 H) 3.27 (dd, *J*=12.98, 9.46 Hz, 1 H) 3.51 (d, *J*=14.96 Hz, 1 H) 3.71 (q, *J*=7.19 Hz, 1 H) 3.84 - 3.93 (m, 2 H) 3.96 (td, J=9.52, 3.19 Hz, 1 H) 4.10 - 4.24 (m, 2 H) 4.31 - 4.43 (m, 2 H) 4.55 - 4.71 (m, 1 H) 7.12 (d, *J*=2.20 Hz, 1 H) 7.21 (dd, *J*=8.47, 2.31 Hz, 1 H) 7.77 (d, *J*=8.58 Hz, 1 H) 8.38 (s, 1 H); LCMS confirms the MW (RT: 1.90, MW: 648.20, $[M+H]^+$ 649, Method 1); SFC (Rt 5.93min 2.40% isomer 1), (Rt 6.37min 96.10% isomer 2) (RT: 6.37, Area %: 96.10, MW: 648.22, BPM1: 708 $[M+iPrNH2]^+$, Method 2).

## Compounds 14 and 15

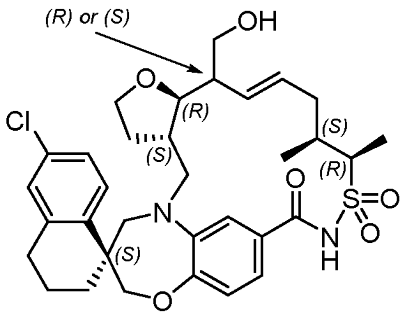

14

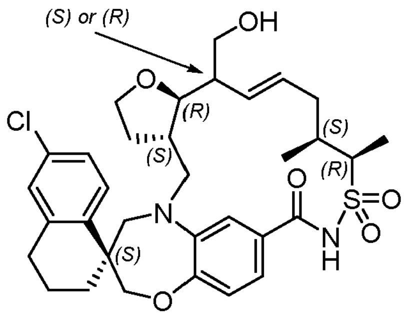

15

A solution of **Intermediate 56** (706 mg, 1.07 mmol, 1.0 equiv.) and Hoveyda-Grubbs 2$^{nd}$ generation catalyst (CAS: 301224-40-8, 135 mg, 0.215 mmol, 0.4 equiv.) in anhydrous DCE (540 mL) was degassed by bubbling a stream of dry $N_2$ through the solution for *ca.* 15 min. The reaction vessel was then sealed, and the reaction mixture was heated at 60 °C for 24 h. After cooling down to rt, ethyl vinyl ether (4.0 mL) was added to quench the metathesis catalyst. After 1 h of vigorous stirring, the reaction mixture was filtered over Celite (the filter cake was rinsed with DCM), and the filtrate was evaporated under reduced pressure. The crude residue was purified by flash column chromatography (silica gel 40 g, 10-90 % EtOAc in heptane). A small sample (50 mg) of this purified product was further purified by reverse-phase preparative HPLC (Stationary phase: RP XBridge Prep C18 OBD 5 μm, 50 x 250 mm; Mobile phase: MeCN + 0.25 % $NH_4HCO_3$ aqueous solution) to give **Compound 14** (6.5 mg, yield: 12 %) and **Compound 15** (7.9 mg, yield: 18 %) as white solids.

**Compound 14.** $^1$H NMR δ 0.98 (br s, 3 H) 1.13 (d, *J*=6.17 Hz, 3 H) 1.72 - 2.12 (m, 8 H) 2.12 - 2.32 (m, 2 H) 2.38 - 2.52 (m, 2 H) 2.77 (br s, 4 H) 2.96 - 3.13 (m, 1 H) 3.30 (br d, *J*=14.11 Hz, 1 H) 3.70 - 4.03 (m, 7 H) 4.05 - 4.23 (m, 1 H) 5.38 - 5.53 (m, 1 H) 5.53 - 5.71 (m, 1 H) 6.79 (br d, *J*=1.25 Hz, 1 H) 7.08 (br s, 2 H) 7.16 (br d, *J*=7.84 Hz, 1 H) 7.31 (br s, 1 H) 7.70 (br d, *J*=8.26 Hz, 1 H); LCMS confirms the MW (RT: 1.77, MW: 628.20, $[M+H]^+$ 629, Method 1).

**Compound** 15. $^1$H NMR δ 1.01 (br s, 3 H) 1.25 (s, 3 H) 1.35 - 1.55 (m, 4 H) 1.65 - 1.85 (m, 3 H) 1.90 - 2.22 (m, 7 H) 2.45 - 2.65 (m, 2 H) 2.78 (br s, 2 H) 2.86 - 2.98 (m, 1 H) 3.32 (br d, *J*=13.80 Hz, 1 H) 3.57 - 3.79 (m, 4 H) 3.86 - 4.07 (m, 4 H) 4.13 (br s, 1 H) 4.29 (br d, *J*=6.38 Hz, 1 H) 5.46 - 5.62 (m, 1 H) 5.84 (br d, *J*=10.35 Hz, 1 H) 6.90 (br d, *J*=14.53 Hz, 2 H) 7.09 (s, 1 H) 7.19 (br d, *J*=7.73 Hz, 1 H) 7.70 (br d, *J*=8.47 Hz, 1 H); LCMS confirms the MW (RT: 1.79, MW: 628.20, $[M+H]^+$ 629, BPM2: 627, Method 1).

## Compound 16

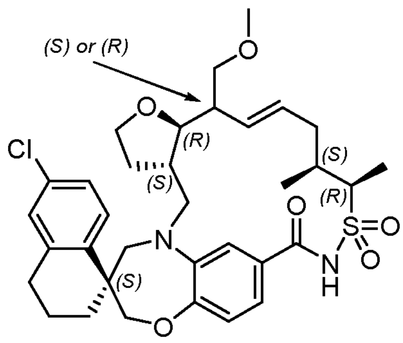

**16**

A solution of **Compound** 14 (55 mg, 0.087 mmol, 1.0 equiv.) in anhydrous THF (2.5 mL) was treated with NaH (60 % dispersion in mineral oil, 14 mg, 0.35 mmol, 4.0 equiv.) at 0 °C followed by addition of MeI (27.2 μL, 0.437 mmol, 5.0 equiv.), and the reaction mixture was heated at 45 °C for 14 h. The reaction mixture was then quenched with saturated aqueous $NH_4Cl$ (30 mL) and extracted with EtOAc (3x). The combined organic layers were washed with brine, dried over anhydrous $MgSO_4$, filtered, and concentrated under reduced pressure to afford **Compound 16** (56 mg, yield: 98 %).

$^1H$ NMR δ 1.02 (br d, *J*=5.5 Hz, 3 H) 1.35 - 1.50 (m, 4 H) 1.71 - 1.85 (m, 3 H) 1.89-2.17 (m, 6 H) 2.43 - 2.63 (m, 1 H) 2.64 - 2.86 (m, 3 H) 2.93 (dd, *J*=15.4, 10.6 Hz, 1 H) 3.29 - 3.35 (m, 2 H) 3.37 (s, 3 H) 3.44 - 3.51 (m, 1 H) 3.55 - 3.65 (m, 1 H) 3.75 (d, *J*=9.7 Hz, 1 H) 3.87 - 3.98 (m, 2 H) 4.01 (br d, *J*=12.1 Hz, 1 H) 4.15 (br d, *J*=12.3 Hz, 1 H) 4.28 (br s, 1 H) 5.45 (dd, *J*=15.3, 8.5 Hz, 1 H) 5.78 - 5.88 (m, 1 H) 6.92 (br s, 2 H) 7.10 (d, *J*=2.2 Hz, 1 H) 7.19 (br d, *J*=7.7 Hz, 1 H) 7.27 (br s, 1 H) 7.71 (d, *J*=8.6 Hz, 1 H); LCMS confirms the MW (RT: 1.93, MW: 642.30, $[M+H]^+$ 643, Method 1).

## Compound 17

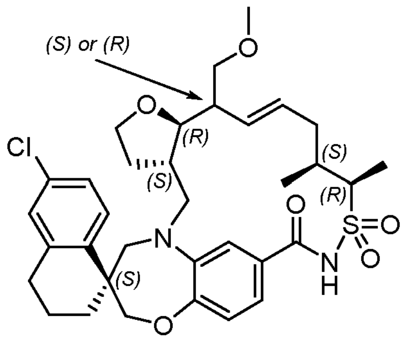

**17**

A solution of **Compound 15** (55 mg, 0.087 mmol, 1.0 equiv.) in anhydrous THF (2.5 mL) was treated with NaH (60 % dispersion in mineral oil, 14 mg, 0.35 mmol, 4.0 equiv.) at 0 °C followed by addition of MeI (27.2 μL, 0.437 mmol, 5.0 equiv.), and the reaction mixture was heated at 45 °C for 14 h. The reaction mixture was then quenched with saturated aqueous $NH_4Cl$ (30 mL) and extracted with EtOAc (3x). The combined organic layers were washed with brine, dried over anhydrous $MgSO_4$, filtered, and concentrated under reduced pressure to afford **Compound 17** (56 mg, yield: 98 %).

$^1H$ NMR δ 1.04 (br d, *J*=5.3 Hz, 3 H) 1.39 - 1.49 (m, 4 H) 1.75 - 1.88 (m, 3 H) 1.91 -2.09 (m, 6 H) 2.56 - 2.66 (m, 1 H) 2.68 - 2.86 (m, 3 H) 3.04 (br dd, *J*=15.4, 10.6 Hz, 1 H) 3.31 (br t, *J*=6.9 Hz, 2 H) 3.38 (s, 3 H) 3.56 - 3.68 (m, 2 H) 3.70 - 3.77 (m, 1 H) 3.78 - 3.91 (m, 2 H) 3.96 - 4.04 (m, 2 H) 4.14 - 4.23 (m, 1 H) 5.54 (br d, *J*=4.4 Hz, 2 H) 6.93 (br s, 2 H) 7.10 (br d, *J*=2.2 Hz, 1 H) 7.13 (br s, 1 H) 7.20 (br dd, *J*=8.5, 1.9 Hz, 1 H) 7.71 (d, *J*=8.4 Hz, 1 H); LCMS confirms the MW (RT: 1.92, MW: 642.30, $[M+H]^+$ 643, Method 1).

## Compound 18

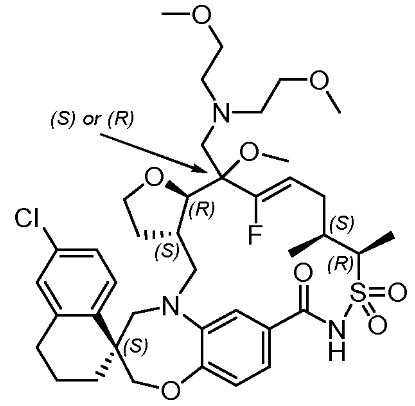

18

DBU (CAS: 6674-22-2, 110 μL, 0.737 mmol, 10 equiv.) was added to a stirred solution of **Intermediate 68** (60 mg, 0.074 mmol, 1.0 equiv.) in anhydrous DMF (2.5 mL) under a $N_2$ atmosphere. After addition, the reaction mixture was stirred at rt for 15 min, and then diethyl cyanophosphonate (CAS: 2942-58-7, 40 μL, 0.267 mmol, 3.0 equiv.) was added gently in one portion. After addition, the reaction mixture was stirred at rt for 1 h. The reaction mixture was diluted with MeCN and directly purified by reverse-phase preparative HPLC (Stationary phase: RP XBridge Prep C18 OBD 10 μm, 30 x 150 mm; Mobile phase: MeCN + 0.5 % $NH_4OAc$ aqueous solution). A second purification by preparative SFC (Stationary phase: Chiralcel Diacel OJ 20 x 250 mm; Mobile phase: $CO_2$, EtOH + 0.4 % *i*-$PrNH_2$) afforded **Compound 18** (4.5 mg, yield: 8 %) as a colourless film. $^1H$ NMR δ 1.04 - 1.10 (m, 3 H) 1.38 - 1.53 (m, 4 H) 1.76 - 1.87 (m, 2 H) 1.90 - 1.97 (m, 2 H) 1.98 - 2.09 (m, 4 H) 2.20 - 2.33 (m, 1 H) 2.71 - 2.87 (m, 5 H) 2.89 - 3.04 (m, 4 H) 3.11 - 3.18 (m, 1 H) 3.26 - 3.32 (m, 7 H) 3.32 - 3.36 (m, 3 H) 3.43 - 3.54 (m, 4 H) 3.75 - 3.87 (m, 2 H) 3.90 - 4.00 (m, 2 H) 4.03 - 4.08 (m, 1 H) 4.09 - 4.12 (m, 1 H) 4.20 - 4.29 (m, 1 H) 5.11 - 5.28 (m, 1 H) 6.88 - 6.95 (m, 2 H) 6.99 - 7.05 (m, 1 H) 7.06 - 7.10 (m, 1 H) 7.15 - 7.22 (m, 1 H) 7.65 - 7.72 (m, 1 H); LCMS confirms the MW (RT: 2.14, MW: 791.30, $[M+H]^+$ 792, Method 1).

## Compound 19

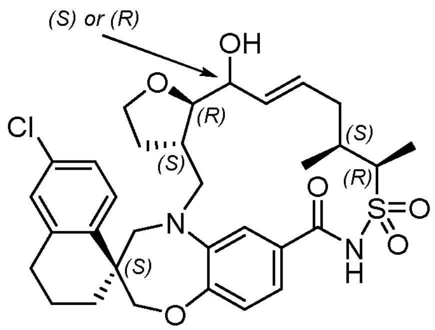

19

A solution of **Intermediate 52** (75 mg, 0.117 mmol, 1.0 equiv.) and Hoveyda-Grubbs 2nd generation catalyst (CAS: 301224-40-8, 14.6 mg, 0.023 mmol, 0.4 equiv.) in anhydrous DCE (58 mL) was degassed by bubbling a stream of dry $N_2$ through the solution for *ca.* 15 min. The reaction vessel was then sealed, and the reaction mixture was heated at 60 °C for 24 h. After cooling down to rt, ethyl vinyl ether (1.0 mL) was added to quench the metathesis catalyst. After 1 h of vigorous stirring, the reaction mixture was filtered over Celite (the filter cake was rinsed with DCM), and the filtrate was evaporated under reduced pressure. The crude residue was purified by flash column chromatography (silica gel 4 g, 0-60 % EtOAc in heptane) to give **Compound 19** (79 mg, yield: 98 %) as a white solid.

$^1H$ NMR δ 1.04 (d, *J*=6.7 Hz, 3 H) 1.41 - 1.45 (m, 1 H) 1.47 (d, *J*=7.2 Hz, 3 H) 1.75 - 1.84 (m, 2 H) 1.98 - 2.03 (m, 2 H) 2.06 - 2.16 (m, 3 H) 2.74 - 2.83 (m, 2 H) 2.98 (br dd, *J*=15.0, 10.8 Hz, 1 H) 3.31 (br d, *J*=14.2 Hz, 1 H) 3.56 - 3.61 (m, 2 H) 3.77 (br d, *J*=9.9 Hz, 2 H) 3.91 (br d, *J*=14.1 Hz, 1 H) 3.96 - 4.05 (m, 3 H) 4.18 (s, 1 H) 4.25 - 4.36 (m, 2 H) 5.69 (br dd, *J*=15.1, 8.2 Hz, 1 H) 5.97 - 6.08 (m, 1 H) 6.82 (s, 1 H) 6.89 - 6.98 (m, 2 H) 7.10 (d, *J*=2.2 Hz, 1 H) 7.20 (dd, *J*=8.5, 2.2 Hz, 1 H) 7.70 (d, *J*=8.5 Hz, 1 H) 8.12 (br s, 1 H); LCMS confirms the MW (RT: 0.96, MW: 615.00, $[M+H]^+$ 615, Method 2).

## Compound 20

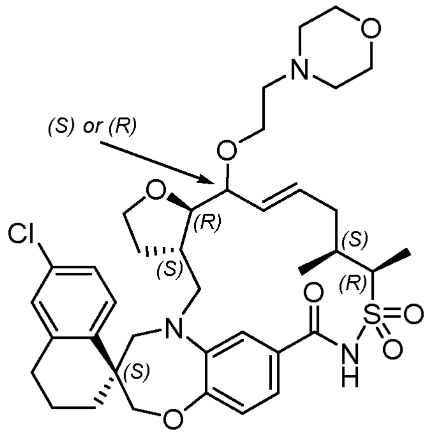

**20**

A solution of **Compound 19** (20 mg, 0.033 mmol, 1.0 equiv.) in anhydrous THF (1.0 mL) was treated with NaH (60 % dispersion in mineral oil, 5.2 mg, 0.13 mmol, 4.0 equiv.) followed by 4-(2-bromoethyl)morpholine (CAS: 89583-07-3, 14.1 μL, 0.094 mmol, 2.85 equiv.), and the reaction mixture was heated at 50 °C for 3 h. Next, the reaction mixture was cooled down to rt, quenched with saturated aqueous $NH_4Cl$ (30 mL), and extracted with EtOAc (3x). The organic layers were combined, washed with brine, dried over anhydrous $MgSO_4$, filtered, and concentrated *in vacuo.* The crude residue was purified by flash column chromatography (silica gel 4 g, 0-10 % MeOH in DCM) to give **Compound 20** (10 mg, yield: 42 %) as a white solid.

$^1$H NMR δ 1.04 (d, *J*=6.8 Hz, 3 H) 1.35 - 1.45 (m, 1 H) 1.48 (d, *J*=7.3 Hz, 3 H) 1.68 - 1.86 (m, 2 H) 1.92 - 2.10 (m, 5 H) 2.10 - 2.21 (m, 1 H) 2.34 - 2.45 (m, 1 H) 2.56 (br s, 4 H) 2.64 (br t, *J*=5.9 Hz, 2 H) 2.74 - 2.84 (m, 2 H) 2.97 (br dd, *J*=15.2, 10.8 Hz, 1 H) 3.31 (br d, *J*=14.1 Hz, 1 H) 3.43 - 3.51 (m, 1 H) 3.56 (ddd, *J*=10.7, 8.6, 5.0 Hz, 1 H) 3.61 - 3.69 (m, 1 H) 3.73 (t, *J*=4.5 Hz, 4 H) 3.79 (br d, *J*=9.7 Hz, 1 H) 3.83 (br d, J=9.2 Hz, 1 H) 3.91 (br d, *J*=14.3 Hz, 1 H) 3.95 - 4.04 (m, 2 H) 3.99 - 4.04 (m, 1 H) 4.17 (d, *J*=12.1 Hz, 1 H) 4.29 (q, *J*=7.0 Hz, 1 H) 5.57 (br dd, *J*=15.4, 9.2 Hz, 1 H) 5.92 (ddd, *J*=15.3, 9.7, 2.8 Hz, 1 H) 6.83 (s, 1 H) 6.90 - 6.99 (m, 2 H) 7.09 (d, *J*=2.0 Hz, 1 H) 7.19 (dd, *J*=8.5, 2.1 Hz, 1 H) 7.69 (d, *J*=8.4 Hz, 1 H); LCMS confirms the MW (RT: 0.99, MW: 728.00, $[M+H]^+$ 728, Method 2).

## Compounds 21 and 22

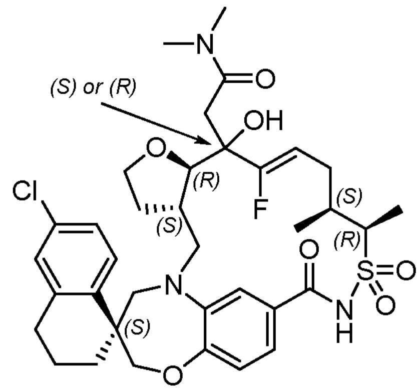

**21**

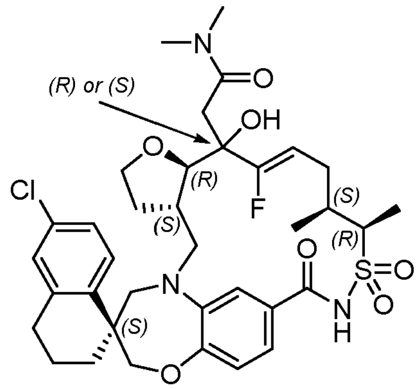

**22**

DBU (CAS: 6674-22-2, 166 μL, 1.11 mmol, 10 equiv.) was added to a stirred solution of **Intermediate 85** (82 mg, 0.111 mmol, 1.0 equiv.) in anhydrous DMF (12 mL) under a $N_2$ atmosphere. After addition, the reaction mixture was stirred at rt for 15 min, and then diethyl cyanophosphonate (CAS: 2942-58-7, 50 μL, 0.334 mmol, 3.0 equiv.) was added gently in one portion. After addition, the reaction mixture was stirred at rt for 30 min. Next, water and 1 N aqueous HCl were added to adjust the pH at *ca.* 4. The mixture was then extracted with EtOAc (3x). The organic layers were combined, washed with brine, dried over anhydrous $MgSO_4$, filtered, and concentrated under reduced pressure. The crude product was purified by reverse-phase preparative HPLC (Stationary phase: RP XBridge Prep C18 OBD 10 μm, 30 x 150 mm; Mobile phase: MeCN + 0.5 % $NH_4HCO_3$ aqueous solution) to give **Compound 21** (9.8 mg, yield: 12 %) and **Compound 22** (7.4 mg, yield: 9 %) both as thick colourless oils.

**Compound 21.** $^1H$ NMR (400 MHz, acetone-$d_6$) δ ppm 1.04 (d, *J*=6.5 Hz, 3 H) 1.39 (d, *J*=6.9 Hz, 3 H) 1.48 - 1.57 (m, 1 H) 1.72 - 1.83 (m, 1 H) 1.88 - 1.94 (m, 2 H) 2.36 - 2.46 (m, 1 H) 2.78 - 2.84 (m, 6 H) 2.87 (s, 3 H) 3.06 (s, 3 H) 3.14 - 3.24 (m, 1 H) 3.42 (d, *J*=13.9 Hz, 1 H) 3.55 - 3.64 (m, 1 H) 3.71 - 3.79 (m, 5 H) 3.84 - 4.11 (m, 6 H) 4.27 (d, *J*=12.2 Hz, 1 H) 5.21 - 5.38 (m, 1 H) 6.62 (d, *J*=0.8 Hz, 1 H) 6.93 (d, *J*=8.2 Hz, 1 H) 7.02 - 7.10 (m, 2 H) 7.13 (d, *J*=2.0 Hz, 1 H) 7.23 (dd, *J*=8.4, 2.2 Hz, 1 H) 7.76 (d, *J*=8.6 Hz, 1 H); LCMS confirms the MW (RT:1.91, MW 718.20, $[M+H]^+$ 719, Method 1).

**Compound** 22. $^1H$ NMR (400 MHz, acetone-$d_6$) δ ppm 0.98 (d, *J*=6.5 Hz, 3 H) 1.35 (d, *J*=6.9 Hz, 3 H) 1.42 - 1.51 (m, 1 H) 1.84 - 2.02 (m, 4 H) 2.14 - 2.27 (m, 4 H) 2.54 (dd, *J*=16.7, 2.0 Hz, 1 H) 2.82 (br s, 12 H) 2.92 (s, 3 H) 3.02 - 3.07 (m, 1 H) 3.09 (s, 3 H) 3.21 (dd, *J*=15.7, 2.2 Hz, 1 H) 3.38 - 3.53 (m, 4 H) 3.72 - 3.81 (m, 6 H) 3.95 (br d, *J*=14.3 Hz, 1 H) 4.04 - 4.09 (m, 1 H) 4.11 - 4.19 (m, 2 H) 4.32 - 4.40 (m, 1 H) 5.27 - 5.45 (m, 1 H) 6.66 (s, 1 H) 6.90 (d, *J*=8.2 Hz, 1 H) 7.08 (dd, *J*=8.0, 1.8 Hz, 1 H) 7.13 (d, *J*=2.4 Hz, 1 H) 7.24 (dd, *J*=8.6, 2.0 Hz, 1 H) 7.65 (d, *J*=1.6 Hz, 1 H) 7.77 (d, *J*=8.6 Hz, 1 H); LCMS confirms the MW (RT:1.91, MW 718.20, $[M+H]^+$ 719, Method 1).

## Compounds 23 and 24

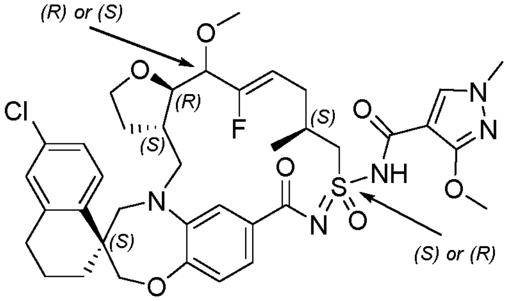

23

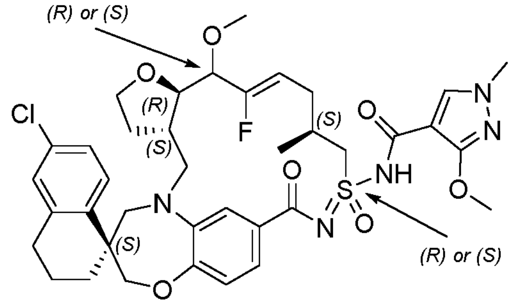

24

A solution of **Intermediate 38** (76 mg, 0.096 mmol, 1.0 equiv.) in anhydrous MeCN (2.0 mL) was added to a well-stirred solution of EDC.HCl (CAS: 25952-53-8, 22 mg, 0.116 mmol, 1.2 equiv.) and DMAP (CAS: 1122-58-3, 28 mg, 0.232 mmol, 2.4 equiv.) in anhydrous MeCN (1.0 mL). After addition, the reaction mixture was stirred at rt for 16 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude product was purified by preparative SFC (Stationary phase: Chiralcel Diacel IH-20, 20 x 250 mm; Mobile phase: $CO_2$, MeOH) to give **Compound 23** (26 mg, yield: 34 %) and **Compound 24** (25 mg, yield: 34 %) as white solids.

**Compound** 23. $^1H$ NMR δ 1.12 (br d, *J*=6.3 Hz, 3 H) 1.26 (s, 2 H) 1.40 - 1.69 (m, 6 H) 1.78 - 2.07 (m, 7 H) 2.12 - 2.23 (m, 2 H) 2.40 - 2.54 (m, 1 H) 2.70 - 2.91 (m, 4 H) 3.18 (br dd, *J*=11.8, 10.2 Hz, 1 H) 3.36 (s, 4 H) 3.67 - 3.76 (m, 3 H) 3.79 (s, 3 H) 3.84 - 4.05 (m, 7H) 4.10 (s, 3 H) 4.18 (br d, *J*=12.1 Hz, 1 H) 4.99 - 5.17 (m, 1 H) 6.95 (br d, *J*=8.3 Hz, 1 H) 7.09 (d, *J*=2.1 Hz, 1 H) 7.19 (dd, *J*=8.6, 2.2 Hz, 1 H) 7.42 - 7.51 (m, 2 H) 7.75 (d, *J*=8.5 Hz, 1 H) 7.80 (s, 1 H); LCMS confirms the MW (RT: 1.85, MW: 769.30, $[M+H]^+$ 770, Method 4).

**Compound** 24. $^1H$ NMR δ 1.18 (br d, *J*=5.5 Hz, 3 H) 1.26 (s, 3 H) 1.60 (br s, 5 H) 1.78 - 2.07 (m, 8 H) 2.45 (br s, 3 H) 2.74 - 2.88 (m, 4 H) 3.06 (br dd, *J*=14.6, 10.4 Hz, 1 H) 3.35 (br d, *J*=14.6 Hz, 1 H) 3.39 (s, 3 H) 3.57 - 3.72 (m, 4 H) 3.79 (s, 3 H) 3.83 - 4.02 (m, 7 H) 4.07 (s, 3 H) 4.16 (d, *J*=12.0 Hz, 1 H) 5.33 - 5.50 (m, 1 H) 6.94 (d, *J*=8.2 Hz, 1 H) 7.08 (d, *J*=2.1 Hz, 1 H) 7.19 (dd, *J*=8.5, 2.1 Hz, 1 H) 7.37 (s, 1 H) 7.49 (br d, *J*=7.7 Hz, 1 H) 7.75 (d, *J*=8.5 Hz, 1 H) 7.79 (s, 1 H); LCMS confirms the MW (RT: 1.81, MW: 769.30, $[M+H]^+$ 770, Method 4).

## Compounds 25 and 26

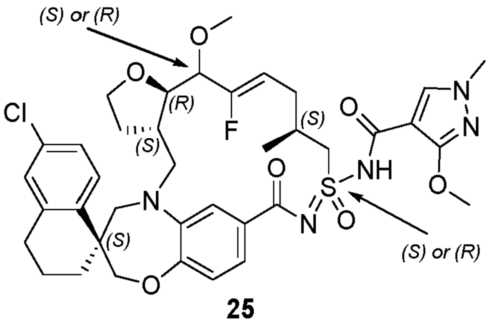

25

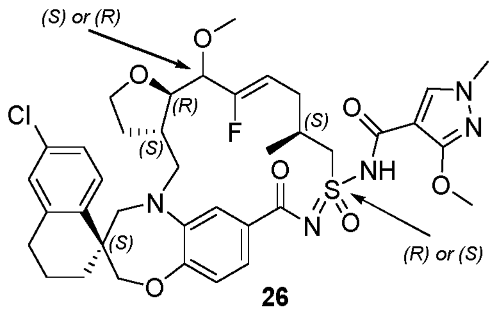

26

A solution of **Intermediate 44** (91 mg, 0.115 mmol, 1.0 equiv.) in anhydrous MeCN (2.5 mL) was added to a well-stirred solution of EDC.HCl (CAS: 25952-53-8, 26 mg, 0.139 mmol, 1.2 equiv.) and DMAP (CAS: 1122-58-3, 34 mg, 0.278 mmol, 2.4 equiv.) in anhydrous MeCN (1.3 mL). After addition, the reaction mixture was stirred at rt for 16 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude product was purified by preparative SFC (Stationary phase: Chiralcel Diacel IH-20, 20 × 250 mm; Mobile phase: $CO_2$, MeOH) to give **Compound 25** (32 mg, yield: 35 %) and **Compound 26** (33 mg, yield: 35 %) as white solids.

**Compound** 25. $^1$H NMR δ 1.18 (br d, *J*=6.6 Hz, 3 H), 1.37 - 1.48 (m, 1 H), 1.75 - 1.97 (m, 4 H), 1.99 - 2.06 (m, 3 H), 2.22 - 2.38 (m, 1 H), 2.45 - 2.62 (m, 2 H), 2.71 - 2.86 (m, 2 H), 2.99 - 3.10 (m, 2 H), 3.38 (br d, *J*=14.4 Hz, 1 H), 3.44 (s, 3 H), 3.65 (br dd, *J*=13.5, 9.1 Hz, 1 H), 3.73 - 3.90 (m, 8 H), 3.97 (br d, *J*=14.2 Hz, 2 H), 4.08 (s, 3 H), 4.17 (d, *J*=12.0 Hz, 1 H), 5.71 (br d, *J*=37.5 Hz, 1 H), 6.94 (br d, *J*=8.3 Hz, 1 H), 7.09 (d, *J*=2.1 Hz, 1 H), 7.20 (dd, *J*=8.5, 2.1 Hz, 1 H), 7.29 (d, *J*=1.6 Hz, 1 H), 7.51 (br d, *J*=7.8 Hz, 1 H), 7.72 - 7.85 (m, 2 H); LCMS confirms the MW (RT: 1.84, Area %: 100.00, MW: 769.30, [M+H]$^+$ 770, Method 4).

**Compound** 26. $^1$H NMR δ 1.14 (br d, *J*=6.2 Hz, 3 H), 1.22 - 1.33 (m, 3 H), 1.39 - 1.47 (m, 1 H), 1.69 - 1.88 (m, 3 H), 1.89 - 2.07 (m, 4 H), 2.18 (br s, 1 H), 2.36 (br s, 2 H), 2.68 - 2.85 (m, 3 H), 3.01 (br d, *J*=14.0 Hz, 1 H), 3.33 (br d, *J*=14.4 Hz, 1 H), 3.39 (br s, 3 H), 3.71 - 4.01 (m, 12 H), 4.05 (br s, 3 H), 4.17 (br d, *J*=12.0 Hz, 1 H), 5.40 (br d, *J*=37.6 Hz, 1 H), 6.91 (br d, *J*=7.6 Hz, 1 H), 7.09 (s, 1 H), 7.19 (br d, *J*=7.9 Hz, 1 H), 7.42 (br d, *J*=7.8 Hz, 1 H), 7.70 - 7.86 (m, 2 H); LCMS confirms the MW (RT: 1.82, Area %: 98.16, MW: 769.30, [M+H]$^+$ 770, Method 4).

## Compounds 27 and 28

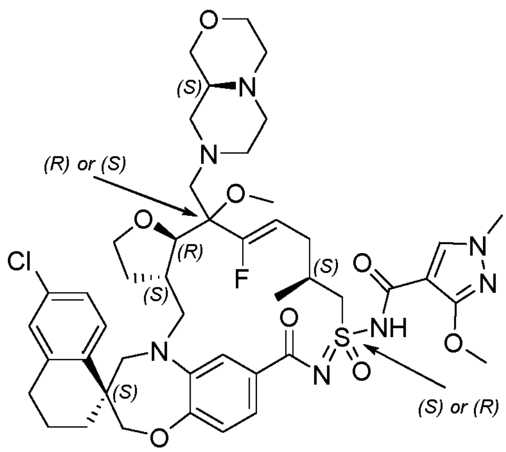

27

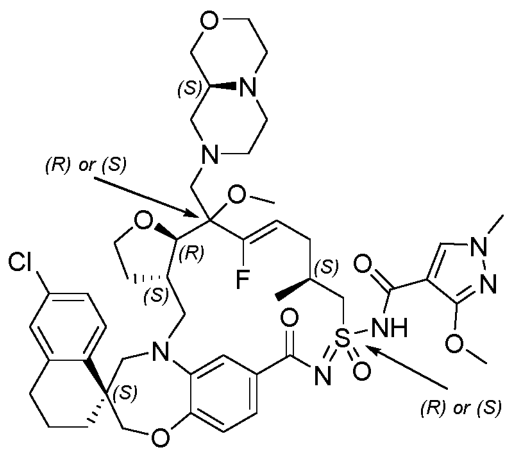

28

A solution of **Intermediate 74** (27 mg, 0.029 mmol, 1.0 equiv.) in anhydrous MeCN (1.5 mL) was added to a well-stirred solution of EDCI (CAS: 25952-53-8, 6.6 mg, 0.034 mmol, 1.2 equiv.) and DMAP (CAS: 1122-58-3, 8.5 mg, 0.07 mmol, 2.4 equiv.) in anhydrous MeCN (0.5 mL). After addition, the reaction mixture was stirred at rt for 16 h. Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude product was purified by preparative SFC (Stationary phase: Chiralcel Diacel IH-20, 20 x 250 mm; Mobile phase: $CO_2$, MeOH) to give **Compound 27** (8.8 mg, yield: 33 %) and **Compound 28** (8.1 mg, yield: 31 %) as white solids.

**Compound** 27. $^1$H NMR (400 MHz, CHLOROFORM-*d*) δ ppm 1.21 (br d, *J*=6.4 Hz, 3 H) 1.40 - 1.52 (m, 2 H) 1.76 - 2.03 (m, 8 H) 2.25 - 2.50 (m, 8 H) 2.61 - 2.88 (m, 8 H) 3.00 (br d, *J*=10.3 Hz, 1 H) 3.08 - 3.17 (m, 2 H) 3.25 (br t, *J*=10.6 Hz, 1 H) 3.44 (s, 4 H) 3.56 - 3.88 (m, 11 H) 3.91 - 4.03 (m, 4 H) 4.09 (s, 3 H) 4.19 (d, *J*=12.1 Hz, 1 H) 5.63 (br dd, *J*=37.7, 4.7 Hz, 1 H) 6.93 (d, *J*=8.1 Hz, 1 H) 7.09 (d, J 2.0 Hz, 1 H) 7.19 (dd, *J*=8.5, 2.1 Hz, 1 H) 7.36 (d, *J*=1.5 Hz, 1 H) 7.48 (dd, *J*=8.3, 1.7 Hz, 1 H) 7.74 (d, *J*=8.6 Hz, 1 H) 7.79 (s, 1 H).

LCMS confirms the MW (RT: 1.88, Area %: 97.92, MW: 923.40, [M+H]$^+$ 924.5, Method 4).

**Compound 28.** $^1$H NMR (400 MHz, CHLOROFORM-*d*) δ ppm 1.21 (br d, *J*=6.8 Hz, 3 H) 1.37 - 1.49 (m, 2 H) 1.78 - 2.05 (m, 8 H) 2.16 - 2.39 (m, 6 H) 2.47 - 2.70 (m, 4 H) 2.70 - 2.87 (m, 6 H) 2.97 - 3.13 (m, 3 H) 3.19 - 3.35 (m, 2 H) 3.38 (s, 3 H) 3.56 - 3.62 (m, 1 H) 3.66 - 3.87 (m, 10 H) 3.92 - 4.04 (m, 4 H) 4.10 (s, 3 H) 4.23 (br d, *J*=12.1 Hz, 1 H) 5.43 (dt, *J*=38.3, 7.0 Hz, 1 H) 6.95 (d, *J*=8.4 Hz, 1 H) 7.09 (d, *J*=1.8 Hz, 1 H) 7.19 (dd, *J*=8.6, 2.0 Hz, 1 H) 7.35 (s, 1 H) 7.46 (br d, *J*=8.1 Hz, 1 H) 7.76 (d, *J*=8.6 Hz, 1 H) 7.80 (s, 1 H).

LCMS confirms the MW (RT: 1.89, Area %: 96.81, MW: 923.40, [M+H]$^+$ 924.5, Method 4).

## Compounds 29 and 30

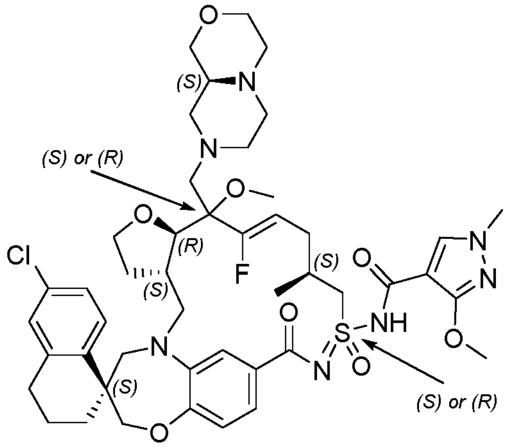

29

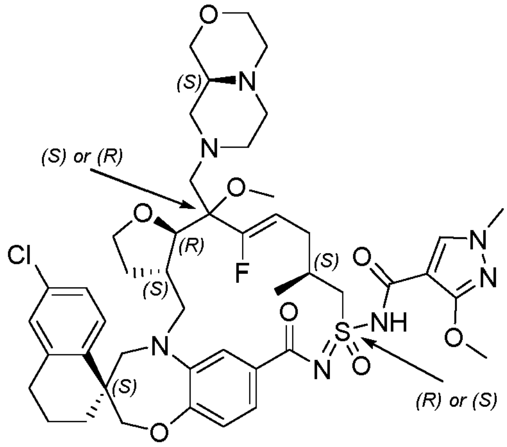

30

A solution of **Intermediate 74** (27 mg, 0.029 mmol, 1.0 equiv.) in anhydrous MeCN (1.5 mL) was added to a well-stirred solution of EDCI (CAS: 25952-53-8, 6.6 mg, 0.034 mmol, 1.2 equiv.) and DMAP (CAS: 1122-58-3, 8.5 mg, 0.07 mmol, 2.4 equiv.) in anhydrous MeCN (0.5 mL). After addition, the reaction mixture was stirred at rt for 16 h.

Upon completion, the reaction mixture was diluted with water, acidified by adding 1 N aqueous HCl until pH *ca.* 5, and extracted with EtOAc. The organic layers were combined, dried over anhydrous $MgSO_4$, filtered, and concentrated under reduced pressure. The crude product was purified by preparative SFC (Stationary phase: Chiralcel Diacel IH-20, 20 x 250 mm; Mobile phase: $CO_2$, MeOH) to give **Compound 29** (8.8 mg, yield: 33 %) and **Compound 30** (8.1 mg, yield: 31 %) as white solids.

**Compound** 29. $^1$H NMR (400 MHz, CHLOROFORM-*d*) δ ppm 1.16 (br d, *J*=6.8 Hz, 3 H) 1.75 - 2.01 (m, 8 H) 2.08 (br d, *J*=11.0 Hz, 3 H) 2.26 - 2.55 (m, 8 H) 2.59 - 2.89 (m, 9 H) 2.91 - 3.10 (m, 4 H) 3.23 - 3.31 (m, 1 H) 3.34 - 3.48 (m, 5 H) 3.67 (br dd, *J*=11.2, 2.5 Hz, 3 H) 3.78 (s, 3 H) 3.80 - 3.99 (m, 7 H) 4.04 (s, 3 H) 4.07 - 4.22 (m, 3 H) 5.37 - 5.54 (m, 1 H) 6.92 (d, *J*=8.2 Hz, 1 H) 7.09 (d, *J*=2.0 Hz, 1 H) 7.20 (dd, *J*=8.5, 2.1 Hz, 1 H) 7.41 (d, *J*=1.5 Hz, 1 H) 7.46 (dd, *J*=8.3, 1.6 Hz, 1 H) 7.75 (s, 1 H) 7.77 (s, 1 H).

LCMS confirms the MW (RT: 1.87, Area %: 100.0, MW: 923.40, $[M+H]^+$ 924.7, Method 4).

**Compound 30.** $^1$H NMR (400 MHz, CHLOROFORM-*d*) δ ppm 1.06 (br d, *J*=6.1 Hz, 3 H) 1.77 - 2.06 (m, 7 H) 2.10 (br d, *J*=13.9 Hz, 3 H) 2.28 - 2.50 (m, 8 H) 2.50 - 2.58 (m, 1 H) 2.62 - 2.88 (m, 8 H) 2.90 - 3.08 (m, 5 H) 3.20 - 3.44 (m, 4 H) 3.46 (s, 3 H) 3.65 - 3.78 (m, 3 H) 3.80 (s, 3 H) 3.81 - 4.04 (m, 7 H) 4.08 (s, 3 H) 4.16 (d, *J*=12.1 Hz, 1 H) 4.29 (br d, *J*=11.3 Hz, 1 H) 5.38 - 5.68 (m, 1 H) 6.95 (d, *J*=8.2 Hz, 1 H) 7.10 (d, *J*=2.1 Hz, 1 H) 7.21 (dd, *J*=8.5, 2.2 Hz, 1 H) 7.46 (s, 2 H) 7.77 (d, *J*=8.5 Hz, 1 H) 7.80 (s, 1 H).

LCMS confirms the MW (RT: 1.87, Area %: 100.0, MW: 923.40, $[M+H]^+$ 924.7, Method 4).

### Analytical Analysis

<u>LCMS</u>

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

| Method Code | Instrument | column | mobile phase | gradient | Flow<br>----------<br>Col T | Run time |
|---|---|---|---|---|---|---|
| 1 | Waters: Acquity® UPLC® - DAD and SQD | Waters :BEH (1.7µm, 2.1 * 100mm) | A: 10mM $CH_3COONH_4$ in 95% H2O + 5% $CH_3CN$<br>B: CH3CN | From 100% A to 5% A in 2.10min, to 0% A in 0.90min, to 5% A in 0.5min | 0.6<br>-------<br>55 | 3.5 |
| 2 | Waters: Acquity® UPLC® - DAD and SQD | BEH C18 column (1.7 µm, 2.1 × 50 mm; Waters Acquity) | A: 10mM $CH_3COONH_4$ in 95% H2O + 5% $CH_3CN$<br>B: CH3CN | 95 % A and 5 % B to 5 % A and 95 % B in 1.3 minutes and hold for 0.7 minutes | 0.8<br>-------<br>55 | 2 |
| 3 | Waters: Acquity® UPLC® - DAD and SQD | Waters :BEH (1.7µm, 2.1 * 100mm) | A: 10mM $CH_3COONH_4$ in 95% H2O + 5% $CH_3CN$<br>B: CH3CN | From 100% A to 5% A in 2.10min, to 0% A in 0.9min, to 5% A in 0.5min | 0.6<br>-------<br>55 | 3.5 |
| 4 | Waters: Acquity® UPLC® - DAD and SQD | Waters :BEH (1.7µm, 2.1 * 100mm) | A: 0.1% $NH_4HCO_3$ in 95% H2O + 5% $CH_3CN$<br>B: CH3CN | From 100% A to 5% A in 2.10min, to 0% A in 0.9min, to 5% A in 0.5min | 0.6<br>-------<br>55 | 3.5 |

SFC-MS methods

The SFC measurement was performed using an Analytical Supercritical fluid chromatography (SFC) system composed by a binary pump for delivering carbon dioxide ($CO_2$) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Analytical SFC-MS Methods (Flow expressed in mL/min; column temperature (T) in °C; Run time in minutes, Backpressure (BPR) in bars. "$iPrNH_2$" means isopropylamine, "iPrOH" means isopropanol, "EtOH" means ethanol, "min" mean minutes.

| SFC Method | Column | mobile phase | gradient | Flow<br>-------<br>Col T | Run time<br>-------<br>BPR |
|---|---|---|---|---|---|
| 1 | Daicel Chiralpak® IH3 column (3.0 µm, 150 x 4.6 mm) | A:$CO_2$<br>B: MeOH-iPrOH (50-50)+0.2% $iPrNH_2$ | 10%-50% B in 6 min, hold 3.5 min | 2.5<br>-------<br>40 | 9.5<br>-------<br>130 |

(continued)

| SFC Method | Column | mobile phase | gradient | Flow<br>-------<br>Col T | Run time<br>-------<br>BPR |
|---|---|---|---|---|---|
| 2 | Daicel Chiralpak® OJ3 column (3.0 μm, 150 x 4.6 mm) | A:$CO_2$<br>B: EtOH+0.2% $iPrNH_2$ | 10%-50% B in 6 min, hold 3.5 min | 2.5<br>-------<br>40 | 9.5<br>-------<br>130 |

NMR

$^1$H NMR spectra were recorded on Bruker Avance III and Avance NEO spectrometers. $CDCl_3$ was used as solvent, unless otherwise mentioned. The chemical shifts are expressed in ppm relative to tetramethylsilane.

**Pharmacological Analysis**

Biological Example 1

Terbium labeled Myeloid Cell Leukemia 1(Mcl-1) homogeneous time-resolved fluorescence (HTRF) binding assay utilizing the BIM BH3 peptide ($H_2N$-(C/Cy5Mal) WIAQELRRIGDEFN-OH) as the binding partner for Mcl-1.

Apoptosis, or programmed cell death, ensures normal tissue homeostasis, and its dysregulation can lead to several human pathologies, including cancer. Whilst the extrinsic apoptosis pathway is initiated through the activation of cell-surface receptors, the intrinsic apoptosis pathway occurs at the mitochondrial outer membrane and is governed by the binding interactions between pro- and anti-apoptotic Bcl-2 family proteins, including Mcl-1. In many cancers, the anti-apoptotic Bcl-2 protein(s), such as the Mcl-1, are upregulated, and in this way the cancer cells can evade apoptosis. Thus, inhibition of the Bcl-2 protein(s), such as Mcl-1, may lead to apoptosis in cancer cells, providing a method for the treatment of said cancers.

This assay evaluated inhibition of the BH3 domain : Mcl-1 interaction by measuring the displacement of Cy5-labeled BIM BH3 peptide ($H_2N$-(C/Cy5Mal) WIAQELRRIGDEFN-OH) in the HTRF assay format.

Assay Procedure

The following assay and stock buffers were prepared for use in the assay: (a) Stock buffer: 10 mM Tris-HCl, pH = 7.5 + 150 mM NaCl, filtered, sterilized, and stored at 4°C; and (b) 1X assay buffer, where the following ingredients were added fresh to stock buffer: 2 mM dithiothreitol (DTT), 0.0025% Tween-20, 0.1 mg/mL bovine serum albumin (BSA). The 1X Tb-Mcl-1 + Cy5 Bim peptide solution was prepared by diluting the protein stock solution using the 1X assay buffer (b) to 25 pM Tb-Mcl-1 and 8 nM Cy5 Bim peptide.

Using the Acoustic ECHO, 100 nL of 100x test compound(s) were dispensed into individual wells of a white 384-well Perkin Elmer Proxiplate, for a final compound concentration of 1x and final DMSO concentration of 1%. Inhibitor control and neutral control (NC, 100 nL of 100% DMSO) were stamped into columns 23 and 24 of assay plate, respectively. Into each well of the plate was then dispensed 10 μL of the 1X Tb-Mcl-1 + Cy5 Bim peptide solution. The plate was centrifuged with a cover plate at 1000 rpm for 1 minute, then incubated for 60 minutes at room temperature with plates covered.

The TR-FRET signal was read on an BMG PHERAStar FSX MicroPlate Reader at room temperature using the HTRF optic module (HTRF: excitation: 337nm, light source: laser, emission A: 665 nm, emission B: 620 nm, integration start: 60 μs, integration time: 400 μs).

Data Analysis

The BMG PHERAStar FSX MicroPlate Reader was used to measure fluorescence intensity at two emission wavelengths - 665 nm and 620 nm - and report relative fluorescence units (RFU) for both emissions, as well as a ratio of the emissions (665 nm/620 nm)* 10,000. The RFU values were normalized to percent inhibition as follows:

$$\% \ inhibition = (((NC - IC) - (compound - IC)) / (NC - IC)) * 100$$

where IC (inhibitor control, low signal) = mean signal of 1X Tb-MCl-1 + Cy5 Bim peptide+ inhibitor control or 100% inhibition of Mcl-1; NC (neutral control, high signal) = mean signal 1X Tb-MCl-1 + Cy5 Bim peptide with DMSO only or 0% inhibition

An 11-point dose response curve was generated to determine $IC_{50}$ values (using GenData) based on the following equation:

$$Y = Bottom + (Top - Bottom)/(1 + 10^{\wedge}((logIC_{50} - X) * HillSlope))$$

where Y = % inhibition in the presence of X inhibitor concentration; Top = 100% inhibition derived from the IC (mean signal of Mcl-1 + inhibitor control); Bottom = 0% inhibition derived from the NC (mean signal of Mcl-1 + DMSO); Hillslope = Hill coefficient; and $IC_{50}$ = concentration of compound with 50% inhibition in relation to top/neutral control (NC).

$$K_i = IC_{50} / (1 + [L]/Kd)$$

In this assay [L] = 8 nM and Kd = 10 nM

Representative compounds of the present invention were tested according to the procedure as described above, with results as listed in the Table below (n.d. means not determined).

| Compound | Tb-MCL1 $K_i$ (nM) |
|---|---|
| **1** | 0.218 |
| **2** | 1.13 |
| **3** | 0.466 |
| **4** | 0.192 |
| **5** | 0.445 |
| **6** | 0.0137 |
| **7** | 0.088 |
| **8** | 0.0231 |
| **9** | 0.050 |
| **10** | 0.401 |
| **11** | 0.94 |
| **12** | 5.23 |
| **13** | 35.5 |
| **14** | 0.521 |
| **15** | 0.636 |
| **16** | 0.673 |
| **17** | 0.678 |
| **18** | 0.096 |
| **19** | 0.717 |
| **20** | 0.223 |
| **21** | 0.182 |
| **22** | 0.981 |
| **23** | 0.25 |
| **24** | 0.011 |
| **25** | 0.025 |
| **26** | 5.12 |

(continued)

| Compound | Tb-MCL1 $K_i$ (nM) |
|---|---|
| **27** | 0.048 |
| **28** | 0.011 |
| **29** | 0.17 |
| **30** | 0.014 |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.
The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.
This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| Product | Units | Storage |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 mL | -20 °C |
| Gentamicin (50 mg/mL) | 100 mL | 4 °C |
| Caspase 3/7 Detection kit | 100 mL 10 x 10 mL | -20 °C |

Cell culture media:

| MOLP8 | |
|---|---|
| | |
| RPMI-1640 medium | 500 mL |
| 20 % FBS (heat inactivated) | 120 mL |
| 2 mM L-Glutamine | 6.2 mL |

(continued)

| | |
|---|---|
| **MOLP8** | |
| 50 μg/mL Gentamicin | 620 μL |
| | |
| **Assay media** | |
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C.

Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2, and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.

The activity of a test compound was calculated as percent change in apoptosis induction as follows:

LC = median of the Low Control values

= Central Reference in Screener

= DMSO

= 0%

HC = Median of the High Control values

= Scale Reference in Screener

= 30 μM of positive control

= 100 % apoptosis induction

$$\%Effect\ (AC_{50}) = 100 - ((sample-LC) / (HC-LC)) * 100$$

$$\%Control = (sample /HC)*100$$

$$\%Control\ min = ((sample-LC) / (HC-LC)) *100$$

Table: Measured $AC_{50}$ for Representative Compounds of Formula (I). Averaged values are reported over all runs on all batches of a particular compound.

| Compound | MOLP8 $AC_{50}$ (nM) |
|---|---|
| **1** | 191 |
| **2** | 1125 |
| **3** | 432 |
| **4** | 314 |
| **5** | 1096 |
| **6** | 69 |
| **7** | 88 |
| **8** | 34 |
| **9** | 119 |
| **10** | 453 |
| **11** | 1308 |
| **12** | 1221 |
| **13** | 15234 |
| **14** | 1250 |
| **15** | 2925 |
| **16** | 1822 |
| **17** | 2816 |
| **18** | 412 |
| **19** | 752 |
| **20** | 616 |
| **21** | 436 |
| **22** | 4828 |
| **23** | 2425 |
| **24** | 44.0 |
| **25** | 486 |
| **26** | >5000 |
| **27** | 424 |
| **28** | 23.2 |
| **29** | 702 |
| **30** | 28.6 |

**Claims**

**1.** A compound of Formula (I)

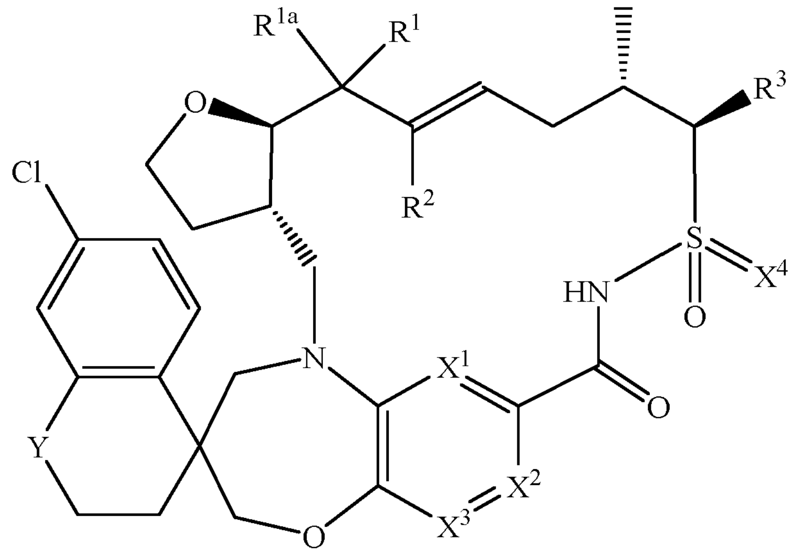

(I) (I),

wherein

$R^1$ represents hydrogen, $-OR^a$, $-CH_2OR^b$, $-CH_2NR^cR^d$, or $-CH_2-C(=O)-NR^cR^d$;
$R^{1a}$ represents hydrogen, $-OR^a$, $-CH_2OR^b$, $-CH_2NR^cR^d$, or $-CH_2-C(=O)-NR^cR^d$;
provided that $R^1$ and $R^{1a}$ cannot both be $-OR^a$;
wherein at least one of $R^1$ or $R^{1a}$ is other than hydrogen;
$R^a$ is selected from the group consisting of hydrogen, $C_{1-4}$alkyl, and $C_{1-4}$alkyl substituted with one substituent selected from the group consisting of $Het^1$, $-OR^e$, and
$-NR^fR^g$;
$R^b$ is selected from the group consisting of hydrogen, $C_{1-4}$alkyl, and $C_{1-4}$alkyl substituted with one substituent selected from the group consisting of $Het^1$, $-OR^e$, and
$-NR^fR^g$;
$R^c$ and $R^d$ are each independently selected from the group consisting of hydrogen;
$C_{1-4}$alkyl; $C_{1-4}$alkyl substituted with $-O-C_{1-4}$alkyl; and $C_{2-4}$alkyl substituted with one $Het^1$;
or $R^c$ and $R^d$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl;
$R^f$ and $R^g$ are each independently selected from the group consisting of hydrogen;
$C_{1-4}$alkyl; $C_{1-4}$alkyl substituted with $-O-C_{1-4}$alkyl; and $C_{2-4}$alkyl substituted with one $Het^1$;
or $R^f$ and $R^g$ are taken together to form together with the N-atom to which they are attached a monocyclic 4- to 7-membered fully saturated heterocyclyl containing one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl;
$R^e$ represents hydrogen or $C_{1-4}$alkyl; $-C_{2-4}$alkyl$-O-C_{1-4}$alkyl, or $-C_{2-4}$alkyl$-O-C_{2-4}$alkyl$-O-C_{1-4}$alkyl;
$R^2$ represents hydrogen or fluoro;
$R^3$ represents hydrogen or methyl;
$R^4$ represents -(C=O)-phenyl, $-(C=O)-Het^2$ or $-(C=O)-Het^3$; wherein said phenyl, $Het^2$ or $Het^3$ are optionally substituted with one or two substituents selected from methyl or methoxy;
$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;

$Het^2$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;
$Het^3$ represents a C-linked 5-or 6-membered monocyclic aromatic ring containing one, two or three heteroatoms each independently selected from O, S, and N;
Y represents $CH_2$;
$X^1$ represents CH or N;
$X^2$ represents CH;
$X^3$ represents CH or N;
$X^4$ represents O or $NR^4$;
or a pharmaceutically acceptable salt, or a solvate thereof.

**2.** The compound according to claim 1, wherein

$R^1$ represents hydrogen, $-CH_2NR^cR^d$, or $-CH_2-C(=O)-NR^cR^d$;
$R^{1a}$ represents $-OR^a$ or $-CH_2OR^b$;
$R^a$ is selected from the group consisting of hydrogen, $C_{1-4}$alkyl, and $C_{1-4}$alkyl substituted with one $Het^1$ substituent;
$R^b$ is selected from the group consisting of hydrogen and $C_{1-4}$alkyl;
$R^c$ and $R^d$ are each independently selected from $C_{1-4}$alkyl substituted with $-O-C_{1-4}$alkyl; or $R^c$ and $R^d$ are taken together to form together with the N-atom to which they are attached a fused bicyclic 6- to 11-membered fully saturated heterocyclyl containing one N-atom and optionally one or two additional heteroatoms each independently selected from O and N;
$R^4$ represents -(C=O)-$Het^3$; wherein said $Het^3$ is optionally substituted with one or two substituents selected from methyl or methoxy;
$Het^1$ represents a 4- to 7-membered monocyclic fully saturated heterocyclyl containing one or two heteroatoms each independently selected from O and N;
$Het^3$ represents a C-linked 5-or 6-membered monocyclic aromatic ring containing one, two or three heteroatoms each independently selected from O and N;
Y represents $CH_2$;
$X^2$ represents CH.

**3.** The compound according to claim 1 or 2, wherein $X^1$ and $X^3$ represent CH.

**4.** The compound according to claim 1, 2 or 3, wherein $X^1$ and $X^3$ represent N.

**5.** The compound according to claim 1, wherein

$R^a$ is selected from the group consisting of hydrogen, $C_{1-4}$alkyl, and $C_{1-4}$alkyl substituted with one substituent selected from the group consisting of $Het^1$ and $-OR^e$;
$R^b$ is selected from the group consisting of hydrogen, $C_{1-4}$alkyl, and $C_{1-4}$alkyl substituted with one substituent selected from the group consisting of $Het^1$ and $-OR^e$.

**6.** A pharmaceutical composition comprising a compound as claimed in any one of claims 1 to 5 and a pharmaceutically acceptable carrier or diluent.

**7.** A process for preparing a pharmaceutical composition as defined in claim 6 comprising mixing a pharmaceutically acceptable carrier with a therapeutically effective amount of a compound according to any one of claims 1 to 5.

**8.** A compound as claimed in any one of claims 1 to 5 or a pharmaceutical composition as claimed in claim 6 for use as a medicament.

**9.** A compound as claimed in any one of claims 1 to 5 or a pharmaceutical composition as claimed in claim 6 for use in the prevention or treatment of cancer.

**10.** The compound or a pharmaceutical composition for use according to claim 9, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

**Patentansprüche**

**1.** Verbindung von Formel (I)

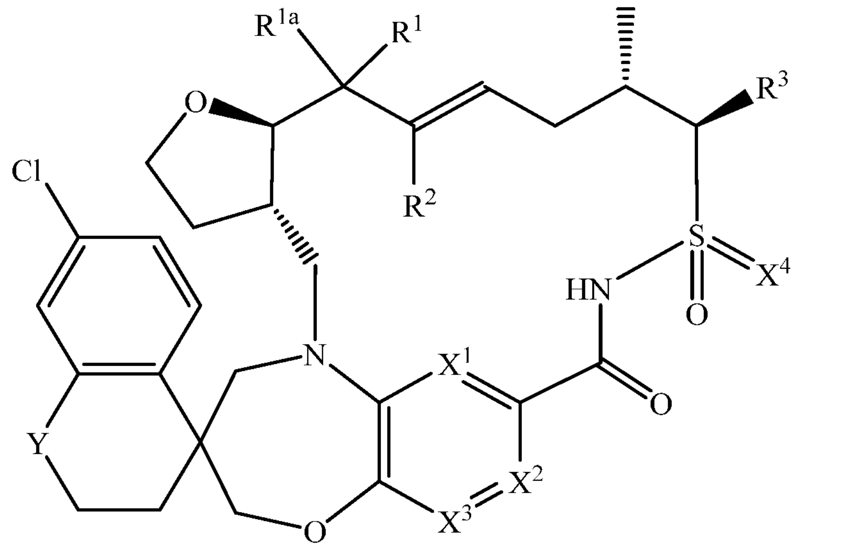

(I)

(I),

wobei

$R^1$ für Wasserstoff, $-OR^a$, $-CH_2OR^b$, $-CH_2NR^cR^d$ oder $-CH_2-C(=O)-NR^cR^d$ steht;
$R^{1a}$ für Wasserstoff, $-OR^a$, $-CH_2OR^b$, $-CH_2NR^cR^d$ oder $-CH_2-C(=O)-NR^cR^d$ steht;
vorausgesetzt, dass $R^1$ und $R^{1a}$ nicht beide $-OR^a$ sein können;
wobei mindestens eines von $R^1$ oder $R^{1a}$ etwas anderes als Wasserstoff ist;
$R^a$ aus der Gruppe ausgewählt ist, bestehend aus Wasserstoff, $C_{1-4}$-Alkyl und $C_{1-4}$-Alkyl, das mit einem Substituenten substituiert ist, der aus der Gruppe ausgewählt ist, bestehend aus $Het^1$, $-OR^e$, und $-NR^fR^g$;
$R^b$ aus der Gruppe ausgewählt ist, bestehend aus Wasserstoff, $C_{1-4}$-Alkyl und $C_{1-4}$-Alkyl, das mit einem Substituenten substituiert ist, der aus der Gruppe ausgewählt ist, bestehend aus $Het^1$, $-OR^e$, und $-NR^fR^g$;
$R^c$ und $R^d$ jeweils aus der Gruppe unabhängig ausgewählt sind, bestehend aus Wasserstoff; $C_{1-4}$-Alkyl; $C_{1-4}$-Alkyl, das mit $-O-C_{1-4}$-Alkyl substituiert ist; und $C_{2-4}$-Alkyl, das mit einem $Het^1$ substituiert ist; oder $R^c$ und $R^d$ zusammengenommen werden, um zusammen mit dem N-Atom, an das sie gebunden sind, ein monocyclisches 4- bis 7-gliedriges vollständig gesättigtes Heterocyclyl auszubilden, das ein N-Atom und optional ein zusätzliches Heteroatom enthält, das aus O, S und N ausgewählt ist, wobei das S-Atom substituiert sein kann, um S(=O) oder $S(=O)_2$ auszubilden; oder ein kondensiertes bicyclisches 6- bis 11-gliedriges vollständig gesättigtes Heterocyclyl, das ein N-Atom und ein optional oder zwei zusätzliche Heteroatome enthält, die jeweils aus O, S und N unabhängig ausgewählt sind; wobei das S-Atom substituiert sein kann, um S(=O) oder $S(=O)_2$ auszubilden;
wobei das monocyclische oder kondensierte bicyclische Heterocyclyl optional auf einem Stickstoff mit einem $C_{1-4}$-Alkyl substituiert ist;
$R^f$ und $R^g$ jeweils aus der Gruppe unabhängig ausgewählt sind, bestehend aus Wasserstoff; $C_{1-4}$-Alkyl; $C_{1-4}$-Alkyl, das mit $-O-C_{1-4}$-Alkyl substituiert ist; und $C_{2-4}$-Alkyl, das mit einem $Het^1$ substituiert ist; oder $R^f$ und $R^g$ zusammengenommen werden, um zusammen mit dem N-Atom, an das sie gebunden sind, ein monocyclisches 4- bis 7-gliedriges vollständig gesättigtes Heterocyclyl auszubilden, das ein N-Atom und optional ein zusätzliches Heteroatom enthält, das aus O, S und N ausgewählt ist, wobei das S-Atom substituiert sein kann, um S(=O) oder $S(=O)_2$ auszubilden; oder ein kondensiertes bicyclisches 6- bis 11-gliedriges vollständig gesättigtes Heterocyclyl, das ein N-Atom und optional ein oder zwei zusätzliche Heteroatome enthält, die jeweils aus O, S und N unabhängig ausgewählt sind; wobei das S-Atom substituiert sein kann, um S(=O) oder $S(=O)_2$ auszubilden;

wobei das monocyclische oder kondensierte bicyclische Heterocyclyl optional auf einem Stickstoff mit einem $C_{1-4}$-Alkyl substituiert ist;
$R^e$ für Wasserstoff oder $C_{1-4}$-Alkyl steht; $-C_{2-4}$-Alkyl-O-$C_{1-4}$-Alkyl oder $-C_{2-4}$-Alkyl-O-$C_{2-4}$-Alkyl-O-$C_{1-4}$-Alkyl-,
$R^2$ für Wasserstoff oder Fluor steht;
$R^3$ für Wasserstoff oder Methyl steht;
$R^4$ für -(C=O)-Phenyl, -(C=O)-$Het^2$ oder -(C=O)-$Het^3$ steht; wobei das Phenyl, $Het^2$ oder $Het^3$ optional mit einem oder zwei Substituenten substituiert sind, die aus Methyl oder Methoxy ausgewählt sind;
$Het^1$ für ein 4- bis 7-gliedriges monocyclisches vollständig gesättigtes Heterocyclyl steht, das ein oder zwei Heteroatome enthält, die jeweils aus O, S und N unabhängig ausgewählt sind; wobei das S-Atom substituiert sein kann, um S(=O) oder $S(=O)_2$ auszubilden;
$Het^2$ für ein C-verknüpftes 4- bis 7-gliedriges monocyclisches vollständig gesättigtes Heterocyclyl steht, das ein oder zwei Heteroatome enthält, die jeweils aus O, S und N unabhängig ausgewählt sind; wobei das S-Atom substituiert sein kann, um S(=O) oder $S(=O)_2$ auszubilden;
$Het^3$ für einen C-verknüpften 5- oder 6-gliedrigen monocyclischen aromatischen Ring steht, der ein, zwei oder drei Heteroatome enthält, die jeweils aus O, S und N unabhängig ausgewählt sind;
Y für $CH_2$ steht;
$X^1$ für CH oder N steht;
$X^2$ für CH steht;
$X^3$ für CH oder N steht;
$Y^4$ für O oder $NR^4$ steht;
oder ein pharmazeutisch unbedenkliches Salz oder ein Solvat davon.

2. Verbindung nach Anspruch 1, wobei

$R^1$ für Wasserstoff, $-CH_2NR^cR^d$ oder $-CH_2-C(=O)-NR^cR^d$ steht;
$R^{1a}$ für $-OR^a$ oder $-CH_2OR^b$ steht;
$R^a$ aus der Gruppe ausgewählt sind, bestehend aus Wasserstoff, $C_{1-4}$-Alkyl und $C_{1-4}$-Alkyl, das mit einem $Het^1$-Substituenten substituiert ist;
$R^b$ aus der Gruppe ausgewählt ist, bestehend aus Wasserstoff und $C_{1-4}$-Alkyl;
$R^c$ und $R^d$ jeweils aus $C_{1-4}$-Alkyl, das mit -O-$C_{1-4}$-Alkyl substituiert ist, unabhängig ausgewählt sind; oder $R^c$ und $R^d$ zusammengenommen werden, um zusammen mit dem N-Atom, an das sie gebunden sind, ein kondensiertes bicyclisches 6- bis 11-gliedriges vollständig gesättigtes Heterocyclyl auszubilden, das ein N-Atom und optional ein oder zwei zusätzliche Heteroatome enthält, die jeweils aus O und N unabhängig ausgewählt sind;
$R^4$ für -(C=O)-$Het^3$ steht; wobei das $Het^3$ optional mit einem oder zwei Substituenten substituiert ist, die aus Methyl oder Methoxy ausgewählt sind;
$Het^1$ für ein 4- bis 7-gliedriges monocyclisches vollständig gesättigtes Heterocyclyl steht, das ein oder zwei Heteroatome enthält, die jeweils aus O und N unabhängig ausgewählt sind;
$Het^3$ für einen C-verknüpften 5- oder 6-gliedrigen monocyclischen aromatischen Ring steht, der ein, zwei oder drei Heteroatome enthält, die jeweils aus O und N unabhängig ausgewählt sind;
Y für $CH_2$ steht;
$X^2$ für CH steht.

3. Verbindung nach Anspruch 1 oder 2, wobei $X^1$ und $X^3$ für CH stehen.

4. Verbindung nach Anspruch 1, 2 oder 3, wobei $X^1$ und $X^3$ für N stehen.

5. Verbindung nach Anspruch 1, wobei

$R^a$ aus der Gruppe ausgewählt ist, bestehend aus Wasserstoff, $C_{1-4}$-Alkyl und $C_{1-4}$-Alkyl, das mit einem Substituenten substituiert ist, der aus der Gruppe ausgewählt ist, bestehend aus $Het^1$ und $-OR^e$;
$R^b$ aus der Gruppe ausgewählt ist, bestehend aus Wasserstoff, $C_{1-4}$-Alkyl und $C_{1-4}$-Alkyl, das mit einem Substituenten substituiert ist, der aus der Gruppe ausgewählt ist, bestehend aus $Het^1$ und $-OR^e$.

6. Pharmazeutische Zusammensetzung, umfassend eine Verbindung nach einem der Ansprüche 1 bis 5 und eine pharmazeutisch unbedenkliche Trägersubstanz oder ein pharmazeutisch unbedenkliches Verdünnungsmittel.

7. Verfahren zum Herstellen einer pharmazeutischen Zusammensetzung wie in Anspruch 6 definiert, umfassend ein Mischen eines pharmazeutisch unbedenklichen Trägers mit einer therapeutisch wirksamen Menge einer Verbindung

nach einem der Ansprüche 1 bis 5.

8. Verbindung nach einem der Ansprüche 1 bis 5 oder pharmazeutische Zusammensetzung nach Anspruch 6 zur Verwendung als ein Arzneimittel.

9. Verbindung nach einem der Ansprüche 1 bis 5 oder eine pharmazeutische Zusammensetzung nach Anspruch 6 zur Verwendung bei einer Vorbeugung oder Behandlung von Krebs.

10. Verbindung oder pharmazeutische Zusammensetzung zur Verwendung nach Anspruch 9, wobei der Krebs aus Prostata, Lunge, Bauchspeicheldrüse, Brust, Eierstock, Gebärmutterhals, Melanom, chronischer lymphatischer Leukämie (CLL) vom B-Zell-Typ, akuter myeloischer Leukämie (AML) und akuter lymphoblastischer Leukämie (ALL) ausgewählt ist.

## Revendications

1. Composé de Formule (I)

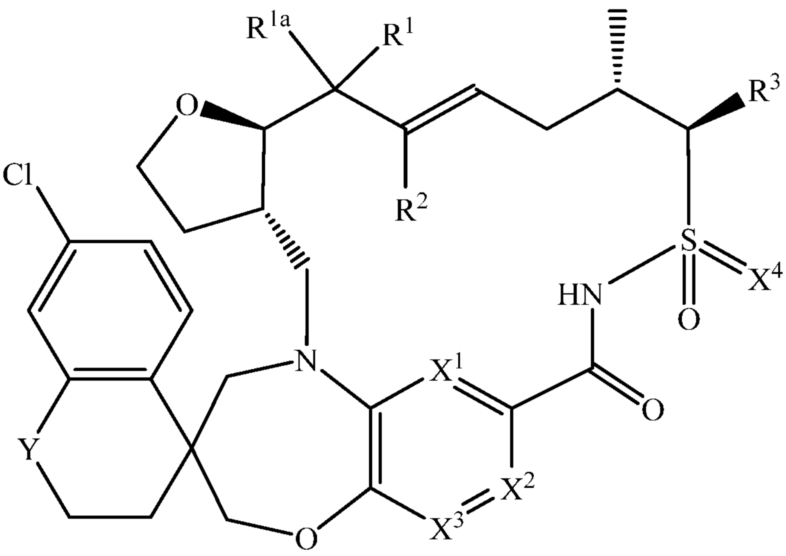

(I)

(I),

dans lequel

$R^1$ représente l'hydrogène, $-OR^a$, $-CH_2OR^b$, $-CH_2NR^cR^d$, ou $-CH_2-C(=O)-NR^cR^d$ ;
$R^{1a}$ représente l'hydrogène, $-OR^a$, $-CH_2OR^b$, $-CH_2NR^cR^d$, ou $-CH_2-C(=O)-NR^cR^d$ ;
à condition que $R^1$ et $R^{1a}$ ne soient pas tous deux $-OR^a$ ;
dans lequel au moins un des éléments $R^1$ ou $R^{1a}$ est autre que l'hydrogène ;
$R^a$ est choisi dans le groupe constitué de l'hydrogène, le $C_{1-4}$alkyle et le $C_{1-4}$alkyle substitué par un substituant choisi dans le groupe constitué de $Het^1$, - $OR^e$, et
$-NR^fR^g$ ;
$R^b$ est choisi dans le groupe constitué de l'hydrogène, le $C_{1-4}$alkyle et le $C_{1-4}$alkyle substitué par un substituant choisi dans le groupe constitué de $Het^1$, - $OR^e$, et
$-NR^fR^g$ ;
$R^c$ et $R^d$ sont chacun choisis indépendamment dans le groupe constitué de l'hydrogène ;
le $C_{1-4}$alkyle ; le $C_{1-4}$alkyle substitué par $-O-C_{1-4}$alkyle ; et le $C_{2-4}$alkyle substitué par un $Het^1$ ;
ou $R^c$ et $R^d$ sont pris ensemble pour former conjointement avec l'atome N auquel ils sont fixés un hétérocyclyle monocyclique complètement saturé de 4 à 7 chaînons contenant un atome N et éventuellement un hétéroatome supplémentaire choisi parmi O, S, et N, dans lequel ledit atome S pourrait être substitué pour former S(=O) ou

$S(=O)_2$ ; ou un hétérocyclyle bicyclique fusionné complètement saturé de 6 à 11 chaînons contenant un atome N et éventuellement un ou deux hétéroatomes supplémentaires choisis chacun indépendamment parmi O, S, et N, dans lequel ledit atome S pourrait être substitué pour former S(=O) ou $S(=O)_2$ ;
dans lequel ledit hétérocyclyle monocyclique ou bicyclique fusionné est éventuellement substitué sur un azote par le $C_{1-4}$alkyle ;
$R^f$ et $R^g$ sont choisis chacun indépendamment dans le groupe constitué de l'hydrogène ;
le $C_{1-4}$alkyle ; le $C_{1-4}$alkyle substitué par -O-$C_{1-4}$alkyle ; et le $C_{2-4}$alkyle substitué par un $Het^1$ ;
ou $R^f$ et $R^g$ sont pris ensemble pour former conjointement avec l'atome N auquel ils sont fixés un hétérocyclyle monocyclique complètement saturé de 4 à 7 chaînons contenant un atome N et éventuellement un hétéroatome supplémentaire choisi parmi O, S, et N, dans lequel ledit atome S pourrait être substitué pour former S(=O) ou $S(=O)_2$ ; ou un hétérocyclyle bicyclique fusionné complètement saturé de 6 à 11 chaînons contenant un atome N et facultativement un ou deux hétéroatomes supplémentaires choisis chacun indépendamment parmi O, S, et N, dans lequel ledit atome S pourrait être substitué pour former S(=O) ou $S(=O)_2$ ;
dans lequel ledit hétérocyclyle monocyclique ou bicyclique fusionné est éventuellement substitué sur un azote par le $C_{1-4}$alkyle ;
$R^e$ représente l'hydrogène ou le $C_{1-4}$alkyle ; -$C_{2-4}$alkyl-O-$C_{1-4}$alkyle, ou -$C_2$-$_4$alkyl-O-$C_{2-4}$alkyl-O-$C_{1-4}$alkyle ;
$R^2$ représente l'hydrogène ou un composé fluoro ;
$R^3$ représente l'hydrogène ou le méthyle ;
$R^4$ représente -(C=O)-phényle, -(C=O)-$Het^2$ or -(C=O)-$Het^3$ ; dans lequel ledit phényle, $Het^2$ ou $Het^3$ est éventuellement substitué par un ou deux substituants choisis parmi le méthyle ou le méthoxy ;
$Het^1$ représente un hétérocyclyle monocyclique complètement saturé de 4 à 7 chaînons contenant un ou deux hétéroatomes choisis chacun indépendamment parmi O, S, et N ; dans lequel ledit atome S pourrait être substitué pour former S(=O) ou $S(=O)_2$ ;
$Het^2$ représente un hétérocyclyle monocyclique complètement saturé de 4 à 7 chaînons lié par C contenant un ou deux hétéroatomes choisis chacun indépendamment parmi O, S, et N ; dans lequel ledit atome S pourrait être substitué pour former S(=O) ou $S(=O)_2$ ;
$Het^3$ représente un anneau aromatique monocyclique de 5 ou 6 chaînons lié par C contenant un, deux ou trois hétéroatomes choisis chacun indépendamment parmi O, S, et N ;
Y représente $CH_2$ ;
$X^1$ représente CH ou N ;
$X^2$ représente CH ;
$X^3$ représente CH ou N ;
$X^4$ représente O ou $NR^4$ ;
ou un sel pharmaceutiquement acceptable, ou un solvate de celui-ci.

**2.** Composé selon la revendication 1, dans lequel

$R^1$ représente l'hydrogène, -$CH_2NR^cR^d$, ou -$CH_2$-C(=O)-$NR^cR^d$ ;
$R^{1a}$ représente $OR^a$ ou -$CH_2OR^b$ ;
$R^a$ est choisi indépendamment dans le groupe constitué de l'hydrogène, le $C_{1-4}$alkyle, et le $C_{1-4}$alkyle substitué par un $Het^1$ ;
$R^b$ est choisi dans le groupe constitué par l'hydrogène et le $C_{1-4}$alkyle ;
$R^c$ et $R^d$ sont choisis chacun indépendamment parmi le $C_{1-4}$alkyle en substitué par -O-$C_{1-4}$alkyl ; ou $R^c$ et $R^d$ sont pris ensemble pour former conjointement avec l'atome N auquel ils sont fixés un hétérocyclyle bicyclique fusionné complètement saturé de 6 à 11 chaînons, contenant un atome N et éventuellement un ou deux hétéroatomes supplémentaires, choisis chacun indépendamment parmi O et N ;
$R^4$ représente -(C=O)-$Het^3$ ; dans lequel ledit $Het^3$ est éventuellement substitué par un ou deux substituants choisis parmi le méthyle ou le méthoxy ;
$Het^1$ représente un hétérocyclyle monocyclique complètement saturé de 4 à 7 chaînons contenant un ou deux hétéroatomes choisis chacun indépendamment parmi O et N ;
$Het^3$ représente un anneau aromatique monocyclique de 5 ou 6 chaînons lié par C contenant un, deux ou trois hétéroatomes choisis chacun indépendamment parmi O et N ;
Y représente $CH_2$ ;
$X^2$ représente CH ;

**3.** Composé selon la revendication 1 ou 2, dans lequel $X^1$ et $X^3$ représentent CH.

**4.** Composé selon la revendication 1, 2 ou 3, dans lequel $X^1$ et $X^3$ représentent N.

**5.** Composé selon la revendication 1, dans lequel

$R^a$ est choisi dans le groupe constitué de l'hydrogène, le $C_{1-4}$alkyle et le $C_{1-4}$alkyle substitué par un substituant choisi dans le groupe constitué de $Het^1$ et - $OR^e$ ;
$R^b$ est choisi dans le groupe constitué de l'hydrogène, le $C_{1-4}$alkyle et le $C_{1-4}$alkyle substitué par un substituant choisi dans le groupe constitué de $Het^1$ et - $OR^e$.

**6.** Composition pharmaceutique comprenant un composé selon l'une quelconque des revendications 1 à 5 et un support ou diluant pharmaceutiquement acceptable.

**7.** Procédé permettant de préparer une composition pharmaceutique telle que définie dans la revendication 6 comprenant le mélange d'un support pharmaceutiquement acceptable avec une quantité thérapeutiquement efficace d'un composé selon l'une quelconque des revendications 1 à 5.

**8.** Composé selon l'une quelconque des revendications 1 à 5 ou composition pharmaceutique selon la revendication 6 destiné à être utilisé comme médicament.

**9.** Composé selon l'une quelconque des revendications 1 à 5 ou composition pharmaceutique selon la revendication 6 destiné à être utilisé dans la prévention ou le traitement d'un cancer.

**10.** Composé ou composition pharmaceutique destiné à être utilisé selon la revendication 9, dans lequel le cancer est choisi parmi les cancers de la prostate, du poumon, du pancréas, du sein, de l'ovaire, du col de l'utérus, le mélanome, la leucémie lymphoïde chronique à lymphocytes B (LLC), la leucémie aiguë myéloïde (LAM), et la leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019046150 A
- WO 2019173181 A
- WO 2016033486 A
- WO 2019036575 A
- WO 2017147410 A
- WO 2018183418 A
- WO 2019222112 A
- WO 2020097577 A


### Non-patent literature cited in the description


- **CHENG et al.** *eLife*, 2016, vol. 5, e17755
- **JULIAN et al.** *Cell Death and Differentiation*, 2017, vol. 24, 1380-1389
- **HANAHAN** ; **WEINBERG**. *Cell*, 2011, 1-44
- **BEROUKHIM et al.** *Nature*, 2010, vol. 463 (7283), 899-905
- **YECIES et al.** *Blood*, 2010, vol. 115 (16), 3304-3313
- **ROBERTS et al.** *NEJM*, 2016, vol. 374, 311-322
- **KOTSCHY et al.** *Nature*, 2016, vol. 538, 477-486
- **MERINO et al.** *Sci. Transl. Med*, 2017 (9)
- **CHEN et al.** *JCI*, 2018, vol. 128 (1), 500-516
- **WANG et al.** *Genes and Dev*, 2013, vol. 27, 1351-1364
- **STEIMER et al.** *Blood*, 2009 (113), 2805-2815
- **CHARRON, CARLIE L. et al.** *Tetrahedron Lett.*, 2016, vol. 57 (37), 4119-4127
- **AUSTIN R. et al.** *Cancer Letters*, 2016
- **T. W. GREENE** ; **P. G. M. WUTS**. Protective Groups in Organic Synthesis. Wiley, 2007
- Part 8 : Pharmaceutical preparations and their Manufacture. **GENNARO et al.** Remington's Pharmaceutical Sciences. Mack Publishing Company, 1990
- *CHEMICAL ABSTRACTS*, 84621-89-6
- *CHEMICAL ABSTRACTS*, 617-86-7
- *CHEMICAL ABSTRACTS*, 109-63-7
- *CHEMICAL ABSTRACTS*, 87413-09-0
- *CHEMICAL ABSTRACTS*, 1122-58-3
- *CHEMICAL ABSTRACTS*, 124-63-0
- *CHEMICAL ABSTRACTS*, 3240-34-4
- *CHEMICAL ABSTRACTS*, 2564-83-2
- *CHEMICAL ABSTRACTS*, 6638-79-5
- *CHEMICAL ABSTRACTS*, 109-02-4
- *CHEMICAL ABSTRACTS*, 25952-53-8
- *CHEMICAL ABSTRACTS*, 1826-67-1
- *CHEMICAL ABSTRACTS*, 1774-47-6
- *CHEMICAL ABSTRACTS*, 52522-40-4
- *CHEMICAL ABSTRACTS*, 12135-22-7
- *CHEMICAL ABSTRACTS*, 79-37-8
- *CHEMICAL ABSTRACTS*, 10049-05-5
- *CHEMICAL ABSTRACTS*, 13462-88-9
- *CHEMICAL ABSTRACTS*, 107-15-3
- *CHEMICAL ABSTRACTS*, 74-88-4
- *CHEMICAL ABSTRACTS*, 76-05-1
- *CHEMICAL ABSTRACTS*, 2526-64-9
- *CHEMICAL ABSTRACTS*, 113100-56-4
- *CHEMICAL ABSTRACTS*, 56553-60-7
- *CHEMICAL ABSTRACTS*, 429-41-4
- *CHEMICAL ABSTRACTS*, 89583-07-3
- *CHEMICAL ABSTRACTS*, 1089280-14-7
- *CHEMICAL ABSTRACTS*, 111-95-5
- *CHEMICAL ABSTRACTS*, 72287-26-4
- *CHEMICAL ABSTRACTS*, 4111-54-0
- *CHEMICAL ABSTRACTS*, 6674-22-2
- *CHEMICAL ABSTRACTS*, 2942-58-7
- *CHEMICAL ABSTRACTS*, 301224-40-8